# EUROPEAN PATENT APPLICATION

(11) **EP 3 964 236 A1**
(43) Date of publication of application: **09.03.2022**
(21) Application number: 20798414.7
(22) Date of filing: 29.04.2020
(51) Int. Cl.: A61K 47/68, A61K 47/18, A61K 47/10, A61K 47/22, A61K 47/26, A61K 45/06, A61P 35/00

(54) **LIGAND-DRUG CONJUGATE INCLUDING LINKER HAVING TRIS STRUCTURE**

(30) Priority: 02.05.2019 KR 20190051618
(71) Applicant: LegoChem Biosciences, Inc., Daejeon 34302 (KR)
(72) Inventor: SONG, Ho Young, Daejeon 34302 (KR); PARK, Yun-Hee, Daejeon 34302 (KR); CHAE, Sang Eun, Daejeon 34302 (KR); BAEK, Ju Yuel, Daejeon 34302 (KR); PARK, Kyung Eun, Daejeon 34302 (KR); LEE, Ju Young, Daejeon 34302 (KR); LEE, Su In, Daejeon 34302 (KR); CHAE, Jeiwook, Daejeon 34302 (KR); LEE, Chang Sun, Daejeon 34302 (KR); KIM, Yong Zu, Daejeon 34302 (KR)
(74) Representative: Vossius & Partner Patentanwälte Rechtsanwälte mbB
(86) International application number: PCT/KR2020/005783
(87) International publication number: WO 2020/222573

(57) **Abstract**

The present invention relates to a ligand-drug conjugate including a ligand; a linker that is connected to the ligand by a covalent bond and has a tris structure represented by a specific structural formula; and an active agent connected to the linker by a covalent bond. In the ligand-drug conjugate, the active agent is bound by the tris structure of the linker, and thus a greater number of active agents can be connected through one linker. Accordingly, a greater number of active agents per antibody binding can be delivered to the target cell, and the drug and/or toxin can stably reach the target cell and effectively exert the drug efficacy.

## Description

### [Technical Field]

The present invention relates to a ligand-drug conjugate including a linker having a tris structure, more particularly to a ligand-drug conjugate including a linker that has a tris structure and has a high active agent delivering efficiency.

### [Background Art]

Cancer refers to a disease caused by an abnormally grown mass by the autonomous overgrowth of body tissues, and is a result of uncontrolled cell growth in various tissues. Early stage tumors may be removed by surgery and radiation therapy, and metastasized tumors are usually treated palliatively by chemotherapy.

Most chemotherapeutic agents administered parenterally may induce serious effects such as unwanted side effects and even toxicity as a result of systemic administration. Therefore, it has been focused on the development of novel chemotherapeutic agents to simultaneously achieve increased drug efficacy and minimized toxicity/side effects through selective application of these chemotherapeutic agents to tumor cells or immediately adjacent tissues.

Antibody-drug conjugates (ADCs) are target-directed new technologies in which an antigen-binding antibody is bound to a toxin or drug and then the toxic substance is released inside the cells to lead the cancer cells and the like to death. Antibody-drug conjugates (ADCs) are a technology that is superior in efficacy to an antibody therapeutic agent itself and can significantly diminish the risk of side effects as compared to existing anti-cancer agents since healthy cells are hardly affected, the drugs are accurately delivered to the target cancer cells, and the toxic substances are released only under certain conditions.

The basic structure of such antibody-drug conjugates is composed of an antibody-linker-low molecular weight drug (toxin). Here, the linker is required not only to serve a functional role of simply connecting the antibody and the drug to each other but also to stably reach the target cell during circulation in the body and then allow the antibody-drug conjugate to enter the cell, the drug to easily fall off by the antibody-drug dissociation phenomenon (for example, as a result of enzymatic hydrolysis), and the drug efficacy to exert on the target cancer cell. In other words, the linker plays a significantly important role in terms of safety such as the efficacy and systemic toxicity of the antibody-drug conjugate depending on the stability of the linker (Discovery Medicine 2010, 10(53): 329-39). 85-8 2018-08-14) .

Linkers of antibody-drug conjugates may be roughly classified as non-cleavable linkers or cleavable linkers. A large number of non-cleavable linkers are attached to antibodies using a thioether containing the cysteine of the antibodies. Such non-cleavable linkers may not normally be separated from the antibodies *in vivo,* and the efficacy thereof may be further diminished when ADC internalization is poor. In the case of the widely used thiol-maleimide method, the antibody-drug conjugate is unstable and may result in dissociation of the drug from the conjugate before or after reaching the target cells.

Instead of chemically unstable linkers exhibiting limited stability in physiological extracellular conditions, such as hydrazone and disulfide-based linkers, linkers that are stable in physiological extracellular conditions are needed. Since the drug is required to be released not outside the cells but only within the cells targeted by the antibody to which the drug is connected, linkers which allow the drug to exhibit high plasma stability are needed in order to enhance therapeutic applicability.

Cleavable linkers may be hydrolyzed, for example, by lysosomal enzymes. Cleavable linkers may have a disulfide bond containing, for example, the cysteine of antibodies. Linkers having a disulfide bond that allows dissociation through a thiol exchange reaction depend in part on the uptake of the antibody-drug conjugate into the target cells and exposure of disulfides to the cytoplasm, a reducing environment. However, the drug may be dissociated from the antibody before reaching the target since different kinds of thiols (for example, albumin and glutathione) are present in the blood.

Korean Patent Application Laid-Open No. 10-2014-0035393 provides a protein-active agent conjugate having an amino acid motif that may be recognized by isoprenoid transferase.

Korean Patent Application Laid-Open No. 2019-0023084 relates to an antibody-drug complex using a bispecific antibody and the use thereof, and discloses that the antibody-drug complex can easily form a complex of an antibody and a drug without a multistep synthesis procedure through the binding of an anti-cotinine single chain variable fragment to a complex of a divalent cotinine-peptide and a drug.

### [Summary of Invention]

### [Technical Problem]

An object of the present invention is to provide a linker that has a tris structure and has a high active agent delivering efficiency, and a ligand-drug conjugate including the linker.

An object of the present invention is to provide a pharmaceutical composition containing a ligand-drug conjugate including a linker having a tris structure, and a treatment method using the pharmaceutical composition.

### [Solution to Problem]

An aspect of the present invention provides a linker for ligand-drug conjugate having a tris structure represented by a specific structure.

Another aspect of the present invention provides a ligand-drug conjugate including a ligand; a linker that is connected to the ligand by a covalent bond and has a tris structure represented by a specific structural formula; and an active agent connected to the linker by a covalent bond.

Still another aspect of the present invention provides a pharmaceutical composition for prevention or treatment of a hyperproliferative, cancer or angiogenic disease, containing a ligand-drug conjugate including a ligand; a linker that is connected to the ligand by a covalent bond and has a tris structure represented by a specific structural formula; and an active agent connected to the linker by a covalent bond or a pharmaceutically acceptable salt or solvate thereof as an active ingredient.

Still another aspect of the present invention provides a method of treating a hyperproliferative, cancer or angiogenic disease in a subject by administering the pharmaceutical composition according to an embodiment of the present invention to the subject.

### [Advantageous Effects of Invention]

The ligand-drug conjugate according to an embodiment of the present invention may include a linker having a tris structure. Since the active agent is bound by the tris structure of the linker, a greater number of active agents can be connected through one linker. In other words, a greater number of active agents per ligand binding can be delivered to the target cell.

The ligand-drug conjugate according to an embodiment of the present invention can effectively, specifically, and selectively deliver a drug.

The linker according to an embodiment of the present invention not only plays a functional role of connecting a ligand and a drug to each other but also can stably reach a target cell during circulation in the body and allow the drug to be easily released after arrival.

The ligand-drug conjugate according to the present invention exhibits excellent stability under physiological extracellular conditions so that the drug can be released not outside the cells but only within the target cells. The drug can be stably bound to the ligand and exhibit the desired cytotoxicity while maintaining *in vivo* stability, and in particular, can be more stable in plasma and exhibit stability during circulation in the body as well.

The linker according to an embodiment of the present invention includes a trigger unit capable of maximizing the drug efficacy by easily releasing the drug within the target cells and thus the drug and/or toxin can stably reach the target cells and effectively exert the drug efficacy.

### [Description of Embodiments]

Hereinafter, embodiments and Examples of the present invention will be described in detail so that those skilled in the art to which the present invention pertains can easily practice the present invention.

However, the present invention may be embodied in several different forms, and thus is not limited to the embodiments and Examples described herein. Throughout the specification of the present invention, when a part "includes" a certain component, it means that other components may be further included rather than excluding other components unless otherwise stated.

As used throughout the specification of the present invention, the terms "about", "substantially" and the like indicating the extent are used in a sense at or close to the numerical value when manufacturing and material tolerances inherent in the stated meaning are presented, and are used to prevent an unconscionable infringer from unduly exploiting the disclosure in which precise or absolute figures are recited to aid understanding of the present invention. As used throughout the specification of the present invention, the term "step (to)" or "step of" indicating the extent does not mean "step for".

Throughout the specification of the present invention, the term "combination of these" included in the Markush grouping means a mixture or combination of one or more selected from the group consisting of the components described in the Markush grouping, and means including one or more selected from the group consisting of the above components. Throughout the specification of the present invention, the description of "A and/or B" means "A or B, or A and B".

The present invention relates to a linker that connects a ligand and a drug to each other, a ligand-drug conjugate including the linker, a pharmaceutical composition for the prevention or treatment of a hyperproliferative, cancer or angiogenic disease containing the ligand-drug conjugate, and a method of treating a hyperproliferative, cancer or angiogenic disease by administering the pharmaceutical composition to a subject.

In an embodiment of the present invention, a ligand is a substance capable of binding to a receptor present inside or outside a cell, and may refer to a molecule capable of forming a complex with a target biomolecule. Examples of the ligand include a molecule that binds to a predetermined position of a target protein and transmits a signal. For example, the ligand may be a protein, substrate, inhibitor, stimulator, neurotransmitter, or radioisotope.

In an embodiment of the present invention the ligand may be an antibody. As will be appreciated by those skilled in the art, the antibody of the antibody-drug conjugate described in an embodiment of the present invention may be replaced with an arbitrary suitable ligand.

References to and discussions of the antibody-drug conjugate according to an embodiment of the present invention may be understood to be equally applicable to a ligand-drug conjugate and a corresponding intermediate thereof (for example, a ligand-linker conjugate) as long as their essence is not contradictory.

A ligand-drug conjugate according to an embodiment of the present invention may be understood to be the same as a ligand-drug complex.

An aspect of the present invention provides a linker having a tris structure. According to an embodiment of the present invention, the linker connecting a ligand and a drug to each other (hereinafter also referred to as "linker for ligand-drug conjugate" or "linker") may have a tris structure, and thus a number of active agents may be conjugated to an antibody through the linker.

According to an embodiment of the present invention, the tris structure may be represented by the following General Formula 1A.

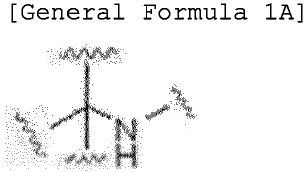

According to an embodiment of the present invention, the linker may include a first linker connected to an active agent and a second linker connected to an antibody, and the first linker and/or the second linker may have a tris structure represented by General Formula 1A.

According to an embodiment of the present invention, the linker having a tris structure may include two or more branched linkers (BL₁, BL₂, and BL₃) through the tris structure.

According to an embodiment of the present invention, the tris structure may be represented by the following General Formula 2A.

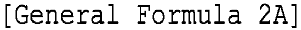

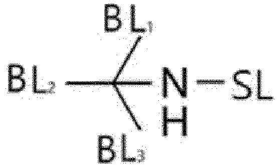

According to an embodiment of the present invention, the linker may include a first linker that is connected to the nitrogen of a tris structure and connected to an active agent and a second linker (SL) connected to an antibody. The first linker may be understood to include two or more branched linkers (BL₁, BL₂, and BL₃) having a tris structure. The second linker (SL) may be bound to an antibody.

According to an embodiment of the present invention, the two or more branched linkers (BL₁, BL₂, and BL₃) and/or the second linker may have a tris structure represented by General Formula 1A.

The branched linkers (BL₁, BL₂, and BL₃) may be connected to an active agent by a covalent bond. Each branched linker may be bound to one or more active agents. In a specific embodiment, any one of the three branched linkers (BL₁, BL₂, and BL₃) may not be connected to an active agent. The active agents may be the same as or different from one another in the same single branched linker, and the active agents on different branched linkers may be the same as or different from one another. On the same antibody as well, the active agents bound to the branched linkers may be the same as or different from one another.

According to an embodiment of the present invention, any one (BL₃) of the branched linkers may be hydrogen, and the linker may be represented by the following General Formula 3A.

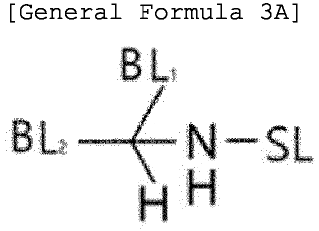

According to an embodiment of the present invention, the tris structure may be represented by the following General Formula 4A.

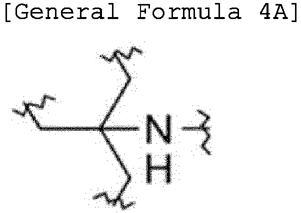

According to an embodiment of the present invention, the tris structure may be represented by the following General Formula 5A.

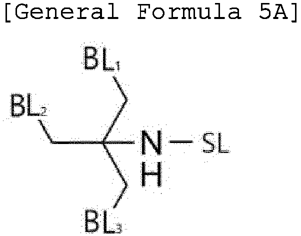

According to an embodiment of the present invention, the linker may include a first linker including the nitrogen of a tris structure and a second linker (SL) connected to an antibody. The first linker may be understood to include two or more branched linkers (BL₁, BL₂, and BL₃) having a tris structure. The second linker (SL) may be bound to an antibody. The branched linkers (BL₁, BL₂, and BL₃) may be connected to an active agent by a covalent bond. Each branched linker may be bound to one or more active agents. In a specific embodiment, any one of the three branched linkers (BL₁, BL₂, and BL₃) may not be connected to an active agent. The active agents may be the same as or different from one another in the same single branched linker, and the active agents on different branched linkers may be the same as or different from one another. On the same antibody as well, the active agents bound to the branched linkers may be the same as or different from one another.

According to an embodiment of the present invention, any one (BL₃) of the branched linkers may be hydrogen, and the linker may be represented by the following General Formula 6A.

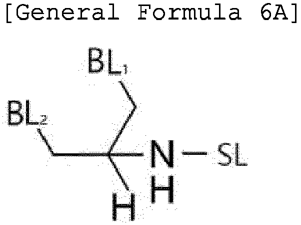

According to an embodiment of the present invention, the first linker (or branched linkers) and/or the second linker may include a polyethylene glycol unit.

In a specific embodiment, the three branched linkers (BL₁, BL₂, and BL₃) and the second linker (SL) may all include a polyethylene glycol unit.

In a specific embodiment, two branched linkers (arbitrary two among BL₁, BL₂, and BL₃) and the second linker (SL) may include a polyethylene glycol unit. Such a combination is not limited thereto, and may be variously modified.

According to an embodiment of the present invention, the first linker (or branched linkers) and/or the second linker may include an atom such as nitrogen. The first linker (or branched linkers) and/or the second linker may include an arbitrary atom or group that forms three bonds, such as an amine, a tertiary amide, or tertiary or quaternary carbon.

In a specific embodiment, the first linker (or branched linkers) and/or the second linker may be an amine or amino acid having a side chain having a group capable of participating in an amide or ester bond.

In an embodiment of the present invention, the amide may be represented by 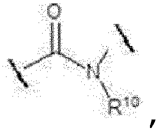 where R¹⁰ may be hydrogen or substituted or unsubstituted alkyl having 1 to 5 carbon atoms.

In a specific embodiment, the first linker (or branched linkers) and/or the second linker may include an amide.

In a specific embodiment, the branched linkers may include an amide.

In a specific embodiment, the first linker, the second linker, and/or the branched linkers may include a naturally-occurring amino acid or an unnatural amino acid. In a specific embodiment, the first linker, the second linker, and/or the branched linkers may include an L-amino acid or a D-amino acid. In a specific embodiment, the first linker, the second linker, and/or the branched linkers may include an α-amino acid or a β-amino acid.

For example, the amino acid may be selected from the group consisting of lysine, 5-hydroxylysine, 4-oxalysine, 4-thialysine, 4-selenalysine, 4-thiahomolysine, 5,5-dimethyllysine, 5,5-difluorolysine, trans-4-dihydrolysine, 2,6-diamino-4-hexinoic acid, cis-4-dehydrolysine, 6-N-methyllysine, diaminopimelic acid, ornithine, 3-methylornithine, α-methylornithine, citrulline, and homocitrulline.

In a specific embodiment, the first linker, the second linker, and/or the branched linkers may include a lysine unit. The lysine unit may also include modifications such as methylation of the ε-amino group, provision of methyl-, dimethyl- and trimethyllysine, and acetylation, sumoylation, and/or ubiquitination.

According to an embodiment of the present invention, the first linker (or branched linkers) and/or the second linker may include a maleimide unit represented by the following Chemical Formula 1G.

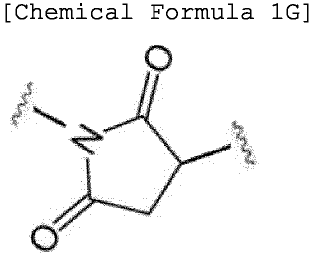

According to an embodiment of the present invention, the first linker (or branched linkers) and/or the second linker may include substituted or unsubstituted alkylene having 1 to 100 carbon atoms, specifically 20 to 80 carbon atoms, and satisfy one or more, specifically, two or more of the following requirements (i) to (iv):
(i) the alkylene has at least one unsaturated bond, specifically 3 or 4 double or triple bonds,
(ii) the alkylene includes at least one heteroarylene;
(iii) at least one carbon atom of the alkylene is substituted with one or more heteroatoms selected from nitrogen (N), oxygen (O) or sulfur (S), specifically at least one nitrogen and at least one oxygen (for example, oxygen in an oxime), and
(iv) the alkylene is substituted with one or more alkyls having 1 to 20 carbon atoms, preferably 2 or 3 methyls.

In an embodiment of the present invention, the linker may include a branching unit, a connection unit, a binding unit, a trigger unit, and an isoprenyl unit. The detailed description thereof will be provided later.

An aspect of the present invention provides a ligand-drug conjugate including a ligand; a linker that is connected to the ligand by a covalent bond and has a tris structure; and an active agent connected to the linker by a covalent bond.

The present invention relates to a linker that connects a ligand and a drug to each other, a ligand-drug conjugate including the linker, a pharmaceutical composition for the prevention or treatment of a hyperproliferative, cancer or angiogenic disease containing the ligand-drug conjugate, and a method of treating a hyperproliferative, cancer or angiogenic disease by administering the pharmaceutical composition to a subject.

In an embodiment of the present invention, the ligand may be an antibody. As will be appreciated by those skilled in the art, it can be understood that the antibody of the antibody-drug conjugate (ADC) described in an embodiment of the present invention may be replaced with an arbitrary suitable ligand and the ligand-drug conjugate described later is equally applicable to the antibody-drug conjugate.

According to an embodiment of the present invention, the linker may have a tris structure represented by General Formula 1A. The description of the linker for ligand-drug conjugate described above may be applied to the ligand-drug conjugate. The description of the linker described below may also be applied to the above-described linker for ligand-drug conjugate.

In an embodiment of the present invention, the linker may be bound to the C-terminus of an antibody (for example, the heavy and light chains of an antibody).

In an embodiment of the present invention, the linker may include a branching unit (BR), a connection unit, or a binding unit.

In a specific embodiment, the connection unit may connect the drug and the branching unit or binding unit to each other. The connection unit may connect the branching unit and the binding unit to each other. The branching unit may be connected to the binding unit without the connection unit, and the binding unit or the connection unit may be connected to an antibody.

In a specific embodiment, the first linker (or branched linkers) and/or the second linker may include a branching unit (BR), a connection unit, or a binding unit.

In a specific embodiment, the first linker and the second linker may include one or more branching units (BRs), connection units, or binding units.

In a specific embodiment, the first linker may include one or more branching units (BRs), connection units (CUs), or binding units (BUs).

In a specific embodiment, the second linker may include one or more connection units.

In an embodiment of the present invention, the branching unit (BR) may be an amino acid.

In a specific embodiment, the branching unit may be a naturally-occurring amino acid or an unnatural amino acid.

In a specific embodiment, the branching unit may be an L-amino acid or a D-amino acid.

In a specific embodiment, the branching unit may be an α-amino acid or a β-amino acid.

For example, the amino acid may be selected from the group consisting of lysine, 5-hydroxylysine, 4-oxalysine, 4-thialysine, 4-selenalysine, 4-thiahomolysine, 5,5-dimethyllysine, 5,5-difluorolysine, trans-4-dihydrolysine, 2,6-diamino-4-hexinoic acid, cis-4-dehydrolysine, 6-N-methyllysine, diaminopimelic acid, ornithine, 3-methylornithine, α-methylornithine, citrulline, and homocitrulline.

In a specific embodiment, the branching unit may be a hydrophilic amino acid. For example, the hydrophilic amino acid may be arginine, aspartate, asparagine, glutamate, glutamine, histidine, lysine, ornithine, proline, serine, or threonine.

In a specific embodiment, the hydrophilic amino acid may be an amino acid including a side chain having a residue having a charge at a neutral pH in an aqueous solution.

In a specific embodiment, the hydrophilic amino acid may be aspartate or glutamate.

In a specific embodiment, the hydrophilic amino acid may be ornithine or lysine.

In a specific embodiment, the hydrophilic amino acid may be arginine.

In a specific embodiment, the branching unit may include a lysine unit. The lysine unit may also include modifications such as methylation of the ε-amino group, provision of methyl-, dimethyl- and trimethyllysine, and acetylation, sumoylation, and/or ubiquitination.

In an embodiment of the present invention, the branching unit (BR) may be hydrogen or alkylene having 1 to 100 carbon atoms, specifically 20 to 80 carbon atoms.

Here, a carbon atom of the alkylene may be substituted with one or more heteroatoms selected from the group consisting of N, O and S, and the alkylene may be further substituted with one or more alkyls having 1 to 20 carbon atoms.

In a specific embodiment, the branching unit may include a nitrogen-containing heteroalkylene having 1 to 50 atoms and two or more atoms of a hydrophilic amino acid, and the nitrogen may form a peptide bond with the carbonyl of the hydrophilic amino acid.

In an embodiment of the present invention, the branching unit (BR) is -C(O)-, -C(O)NR'-, -C(O)O-, - S(O)₂NR'-, -P(O)R"NR'-, -S(O)NR'-, or -PO₂NR'-, where R' and R" each independently include hydrogen, (C₁-C₈)alkyl, (C₃-C₈) cycloalkyl, (C₁-C₈)alkoxy, (C₁-C₈)alkylthio, mono-or di-(C₁-C₈)alkylamino, (C₃-C₂₀)heteroaryl, or (C₆-C₂₀)aryl.

In a specific embodiment, the branching unit may be -C(O)NR'-, where R' may be hydrogen.

In an embodiment of the present invention, the branching unit (BR) may be represented by any one of the following Chemical Formulas 1B to 8B.

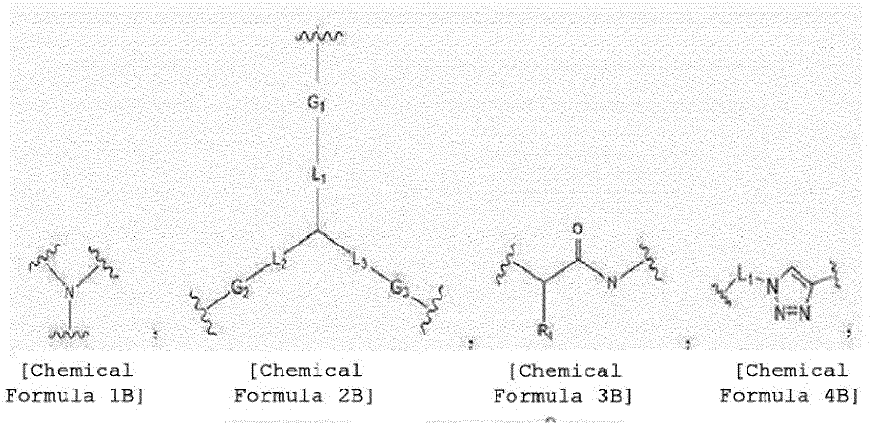

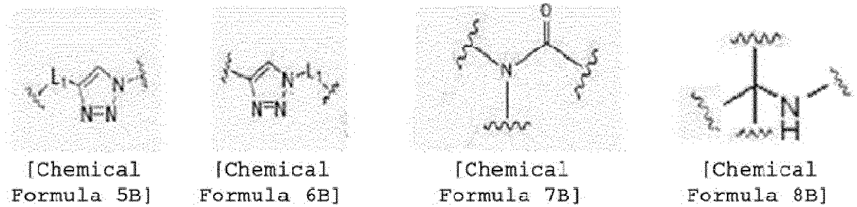

In Chemical Formulas 1B to 8B,
L₁, L₂, and L₃ are each independently a direct bond or -CₙH₂ₙ-, where n is an integer from 1 to 30,
G₁, G2, and G₃ are each independently a direct bond, 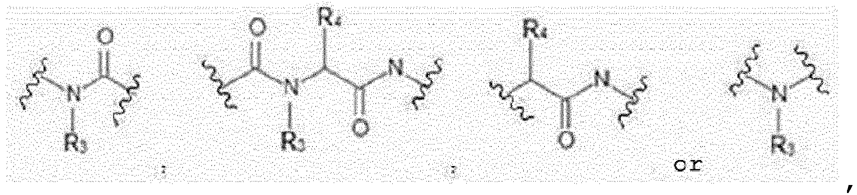 where R₃ is hydrogen or C₁-C₃₀ alkyl, and
R₄ is hydrogen or -L₄-COOR₅, where L₄ is a direct bond or -CₙH₂ₙ-, where n is an integer from 1 to 10, and R₅ is hydrogen or C₁-C₃₀ alkyl.

In an embodiment of the present invention, the branching unit (BR) may be an oxime or an O-substituted oxime.

In an embodiment of the present invention, the branching unit (BR) may be represented by the following Chemical Formula 9B or 10B.

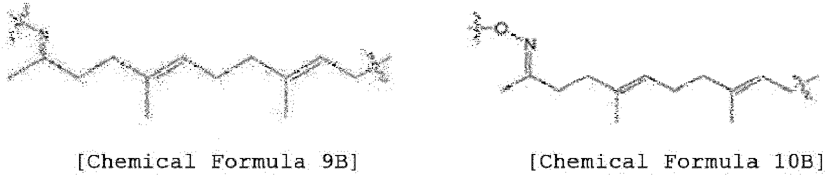

In an embodiment of the present invention, the connection unit may be represented by -(CH₂)ᵣ(V(CH₂)ₚ)_{q}-, - ((CH₂)ₚV)_{q}-, -(CH₂)ᵣ(V(CH₂)ₚ)_{q}Y-, -((CH₂)ₚV)_{q}(CH₂)ᵣ-, - Y((CH₂)ₚV)_{q}-, or -(CH₂)ᵣ(V(CH₂)ₚ)_{q}YCH₂-, where r is an integer from 0 to 10; p is an integer from 1 to 10; q is an integer from 1 to 20; and V and Y are each independently a single bond, -O-, -S-, -NR₂₁-, -C(O)NR₂₂-, -NR₂₃C(O)-, -NR₂₄SO₂-, or -SO₂NR₂₅-, where R₂₁ to R₂₅ are each independently hydrogen, (C₁-C₆)alkyl, (C₁-C₆)alkyl (C₆-C₂₀)aryl, or (C₁-C₆)alkyl (C₃-C₂₀)heteroaryl.

In a specific embodiment, r may be 2.

In a specific embodiment, p may be 2.

In a specific embodiment, q may be an integer from 6 to 20.

In a specific embodiment, q may be 2, 5 or 11.

In a specific embodiment, V and Y may each independently be -O-.

In a specific embodiment, the connection unit may be -(CH₂)ᵣ(V(CH₂)ₚ)_{q}-, where r is an integer from 0 to 10, p is an integer from 0 to 12, q is an integer from 1 to 20, and V is a single bond, -O- or -S-. In a specific embodiment, r may be 2. In a specific embodiment, p may be 2. In a specific embodiment, q may be an integer from 6 to 20.

In a specific embodiment, V may be -O-, r may be 2, p may be 2, and q may be 2, 5 or 11.

In an embodiment of the present invention, the connection unit (CU) may be a polyalkylene glycol unit. More specifically, the connection unit (CU) may be a polyethylene glycol unit or a polypropylene glycol unit.

The polyethylene glycol unit may have a structure of

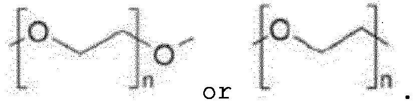

In an embodiment of the present invention, the connection unit may have 1 to 12 -OCH₂CH₂- units, or 5 to 12 -OCH₂CH₂- units, or 6 to 12 -OCH₂CH₂- units.

In an embodiment of the present invention, the connection unit may be -(CH₂CH₂X)w-, where X is a single bond, -O-, (C₁-C₈)alkylene, or -NR₂₁-, where R₂₁ is hydrogen, (C₁-C₆)alkyl, (C₁-C₆)alkyl (C₆-C₂₀)aryl, or (C₁-C₆)alkyl(C₃-C₂₀)heteroaryl, and w is an integer from 1 to 20, specifically 1, 3, 6 or 12.

In a specific embodiment, X may be -O- and w may be an integer from 6 to 20.

In an embodiment of the present invention, the binding unit (BU) may be formed by a 1,3-dipolar cycloaddition reaction, a hetero-Diels-Alder reaction, a nucleophilic substitution reaction, a non-aldol type carbonyl reaction, addition to a carbon-carbon multiple bond, an oxidation reaction, or a click reaction.

In an embodiment of the present invention, the binding unit (BU) may be formed by a reaction of acetylene with azide or a non-aldol type carbonyl reaction, for example, a reaction of an aldehyde or a ketone group with hydrazine or an alkoxyamine.

In an embodiment of the present invention, the binding unit (BU) may be represented by any one of the following Chemical Formulas 1D to 4D.

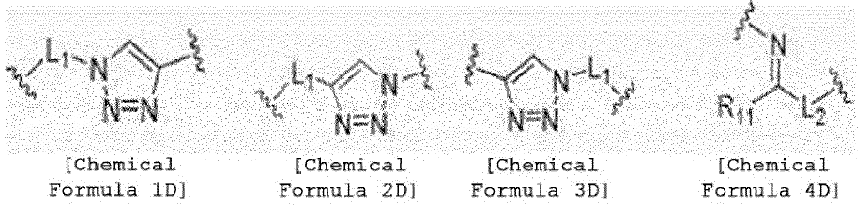

In Chemical Formulas 1D to 4D,
L₁ is a single bond or alkylene having 1 to 30 carbon atoms,
R₁₁ is hydrogen or alkyl having 1 to 10 carbon atoms, specifically methyl, and
L₂ is alkylene having 1 to 30 carbon atoms.

In an embodiment of the present invention, the linker may include any one of units represented by the following Chemical Formulas 4A to 6A.

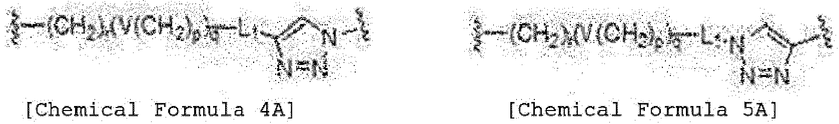

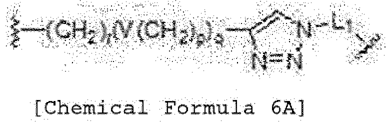

In Chemical Formulas 4A to 6A,
V denotes a single bond, -O-, -S-, -NR₂₁-, -C(O)NR₂₂-, -NR₂₃C(O)-, -NR₂₄SO₂-, or -SO₂NR₂₅-, preferably -O-; where R₂₁ to R₂₅ each independently denote hydrogen, (C₁-C₆)alkyl, (C₁-C₆)alkyl (C₆-C₂₀)aryl, or (C₁-C₆)alkyl (C₃-C₂₀)heteroaryl;
r is an integer from 0 to 10, preferably 2 or 3;
p is an integer from 0 to 10, preferably 1 or 2;
q is an integer from 1 to 20, preferably an integer from 1 to 6; and
L₁ is a single bond.

In an embodiment of the present invention, click chemistry reactions may be conducted under mild conditions in which reactions may be conducted in the presence of an antibody without modification of the antibody. Click chemistry reactions exhibit high reaction specificity. Hence, the click chemistry reactions may be conducted without affecting, for example, the amino acid side chains of the antibody although the antibody has a variety of functional groups (for example, amine, carboxyl, carboxamide, and guanidinium). The click chemistry reactions between the azide group and the acetylene group may take place in the presence of the antibody without modifying the amino acid side chain functional groups of the antibody. In some cases, the reactants are selected to improve the overall reaction efficiency. For example, an azide-acetylene click chemistry reactions may produce a triazole in high yield (for example, see Hia, RK et al., Chem. Rev., 109:5620 (2009); Meldal, M & Tornoe, CW, Chem Rev., 108:2952 (2008); Kolb, HC et al., Angew. Chemie Int. Ed. Engl., 40:2004 (2001), which are each incorporated herein by reference).

Azide and acetylene functional groups are not present in natural proteins. Hence, any of amino acid side chains, N-terminal amines, or C-terminal carboxyls may not be affected by click chemistry reactions using these functional groups.

In an embodiment of the present invention, the binding unit (BU) may be a polyalkylene glycol unit. More specifically, the binding unit (BU) may be a polyethylene glycol unit or a polypropylene glycol unit.

The polyethylene glycol unit may have a structure of

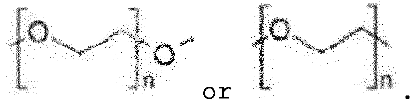

In an embodiment of the present invention, the binding unit may have 1 to 12 -OCH₂CH₂- units, or 5 to 12 -OCH₂CH₂- units, or 6 to 12 -OCH₂CH₂- units.

In an embodiment of the present invention, the binding unit (BU) may be a maleimide unit represented by the following Chemical Formula 1G.

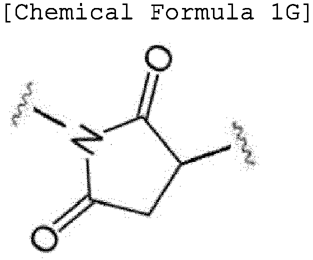

In an embodiment of the present invention, the linker may further include an isoprenyl unit.

The isoprenyl unit may be represented by 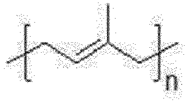 (where n is an integer greater than or equal to 2) .

In an embodiment of the present invention, the isoprenyl unit is a substrate of isoprenoid transferase or a product of isoprenoid transferase.

In an embodiment of the present invention, the isoprenyl unit of the linker is covalently bound to an antibody by a thioether bond, and the thioether bond includes a sulfur atom of cysteine of the antibody.

Cysteine of an antibody, for example, cysteine at the C-terminus of a heavy or light chain of an antibody, forms a thioether bond with a carbon atom of the isoprenyl unit, and the antibody may be thus connected to the linker by a covalent bond.

Hence, in a specific embodiment, the linker may include one isoprenyl unit represented by the following Chemical Formula 1E, specifically, two isoprenyl units, and this may be recognized by isoprenoid transferase, for example, as part of a substrate or product of isoprenoid transferase.

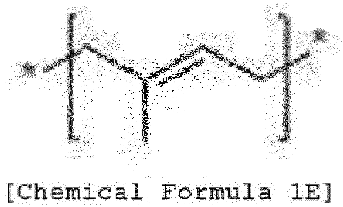

In a specific embodiment, the branching unit may include an oxime, and the isoprenyl unit may connect the oxime and the antibody to each other by a covalent bond.

In an embodiment of the present invention, the linker may be covalently bound to the ligand by a thioether bond, and the thioether bond may include a sulfur atom of cysteine of the ligand.

In an embodiment of the present invention, the ligand may include an amino acid motif that may be recognized by isoprenoid transferase. For example, the C-terminus of at least one antibody may include an amino acid motif that may be recognized by isoprenoid transferase (for example, as a substrate prior to the formation of ligand-drug conjugate or as a product after the formation of ligand-drug conjugate). The ligand may further include a spacer, such as an amino acid or an amino acid stretch, which connects the peptide chain of an antibody to an amino acid motif. The spacer may consist of 1 to 20 consecutive amino acids, specifically 7 or more amino acids. Glycine and proline are preferred amino acids for the spacer, and an arbitrary combination of a series of about 7 glycines may be used.

In a specific embodiment, the C-terminus of the ligand includes the amino acid sequence GGGGGGGCVIM. The ligand may include additions or deletions at the carboxy terminus, for example, with regard to the form of the ligand that is not included in the ligand-drug conjugate.

Examples of isoprenoid transferase include farnesyl protein transferase (FTase) and geranylgeranyl transferase (GGTase), and this may catalyze the transfer of a farnesyl or geranyl-geranyl group to at least one C-terminal cysteine of the target protein. GGTase may be classified as GGTase I or GGTase II. FTase and GGTase I may recognize a CAAX motif and GGTase II may recognize a XXCC, XCXC, or CXX motif, where C denotes cysteine, A denotes an aliphatic amino acid (for example, isoleucine, valine, methionine, or leucine), and each X independently denotes, for example, glutamine, glutamate, serine, cysteine, methionine, alanine, or leucine (see Nature Rev. Cancer, 5(5):405-12 (2005); Nature Chemical Biology 17:498-506 (2010); Lane KT, Bees LS, J. Lipid Research, 47:681-699 (2006); Kasey PJ, Seabra MC, J. Biological Chemistry, 271(10):5289-5292 (1996), which are each incorporated herein by reference in its entirety).

The ligand-drug conjugate according to the present invention may include an amino acid motif, for example, CYYX, XXCC, XCXC, or CXX, preferably CYYX (where C denotes cysteine, each Y independently denotes an aliphatic amino acid, for example, leucine, isoleucine, valine, and/or methionine, and X denotes an amino acid that determines the substrate specificity of isoprenoid transferase, for example, glutamine, glutamate, serine, cysteine, methionine, alanine, and/or leucine).

Isoprenoid transferase from a variety of sources may be used. For example, isoprenoid transferase may be obtained from human, animal, plant, bacterial, viral, or other sources. In several specific embodiments, natural isoprenoid transferase is used. In several specific embodiments, naturally-modified or artificially-modified isoprenoid transferase may be used. For example, isoprenoid transferase may include one or more amino acid substitutions, additions and/or deletions, and/or isoprenoid transferase may be modified by the addition of at least one of histidine-tag, GST, GFP, MBP, CBP, Isopeptag, BCCP, Myc-tag, calmodulin-tag, FLAG-tag, HA-tag, maltose binding protein-tag, Nus-tag, glutathione-S-transferase-tag, green fluorescent protein-tag, thioredoxin-tag, S-tag, Softag 1, Softag 3, Strep-tag, SBP-tag, Ty-tag, or the like.

Isoprenoid transferase recognizes isosubstrates and/or substrates. The term isosubstrate refers to a substrate analog that includes chemical modifications. Isoprenoid transferase may alkylate certain amino acid motifs (for example, CAAX motifs) at the C-terminus of an antibody (for example, see Duckworth, BP et al., ChemBioChem, 8:98 (2007); Uyen TT et al., ChemBioChem, 8:408 (2007); Labadie, GR et al., J. Org. Chem., 72(24):9291 (2007); Wollack, JW et al., ChemBioChem, 10:2934 (2009), which are each incorporated herein by reference in its entirety). Functionalized antibodies may be produced using isoprenoid transferase and an isosubstrate, which are capable of alkylating the C-terminal cysteine.

In a specific embodiment, the isosubstrate may be a compound represented by the following Chemical Formula 2E:

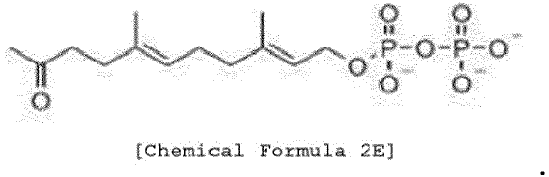

The cysteine of the C-terminal CAAX motif may be bound to the isosubstrate using isoprenoid transferase.

In a specific embodiment, part of a motif, for example, AAX, may be sequentially removed by a protease, leaving, for example, only the cysteine to which the isoprenoid is bound. Cysteine may optionally be methylated at the carboxyl terminus by, for example, enzymes (for example, see Bell, IM, J. Med. Chem., 47(8):1869 (2004), which is incorporated herein by reference in its entirety).

According to an embodiment of the present invention, an active agent may be connected to a linker by a cleavable or non-cleavable bond, and a hydrolytic or nonhydrolytic bond. In an embodiment of the present invention, the active agent may be connected to a first linker, for example, branched linkers (BL₁, BL₂, and BL₃).

In an embodiment of the present invention, the active agent may be connected to the linker through a trigger unit (TU). A trigger unit may be understood as a self-sacrificing group that is cleaved to release the active agent from the ADCs.

In an embodiment of the present invention, the trigger unit may be represented by the following Chemical Formula 1F.

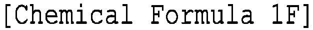

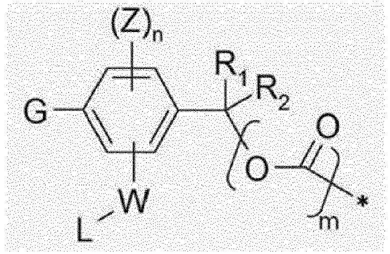

In Chemical Formula 1F,
G is a sugar, sugar acid, or a sugar derivative;
W is -C(O)-, -C(O)NR'-, -C(O)O-, -S(O)₂NR'-, -P(O)R"NR'-, -S(O)NR'-, or -PO₂NR'-, where R' and R" are each independently hydrogen, (C₁-C₈)alkyl, (C₃-C₈)cycloalkyl, (C₁-C₈)alkoxy, (C₁-C₈)alkylthio, mono- or di-(C₁-C₈)alkylamino, (C₃-C₂₀)heteroaryl, or (C₆-C₂₀)aryl when C(O), S, or P is directly connected to a phenyl ring;
each Z is independently hydrogen, (C₁-C₈)alkyl, halogen, cyano, or nitro;
n is an integer from 1 to 3;
m is 0 or 2;
R₁ and R₂ are each independently hydrogen, (C₁-C₈)alkyl or (C₃-C₈)cycloalkyl or form a (C₃-C₈)cycloalkyl ring together with a carbon atom to which R₁ and R₂ are attached;
L means connection with a linker; and
* indicates a site connected to an active agent (drug or toxin).

In a specific embodiment, the trigger unit may be represented by the following Chemical Formula 3F or 4F.

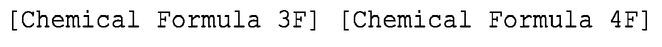

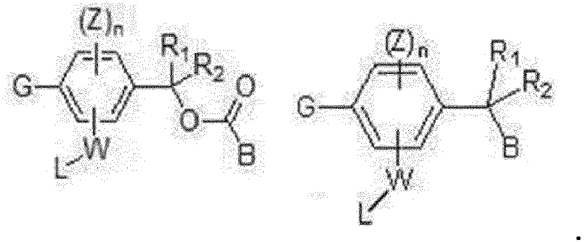

In a specific embodiment, the sugar and sugar acid may be monosaccharides.

In a specific embodiment, G may be represented by the following Chemical Formula 2F.

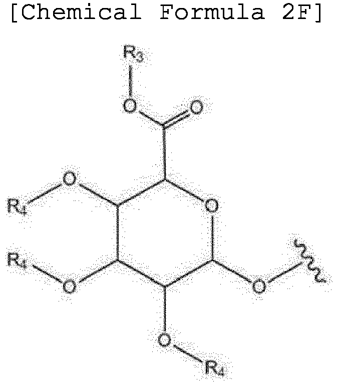

In Chemical Formula 2F,
R₃ is hydrogen or a carboxyl protecting group, and
each R₄ is independently hydrogen or a hydroxyl protecting group.

The carboxyl protecting group may be, for example, an arbitrary suitable protecting group for masking a carboxylic acid in organic synthesis. For example, the carboxyl protecting group may be methyl, methoxymethyl, methylthiomethyl, tetrahydropyranyl, benzyloxymethyl, phenacyl, N-phthalimidomethyl, 2,2,2-trichloroethyl, 2-haloethyl, 2-(p-toluenesulfonyl)ethyl, t-butyl, cinnamyl, benzyl, triphenylmethyl, bis(o-nitrophenyl)methyl, 9-anthrylmethyl, 2-(9,10-dioxo)anthrylmethyl, piperonyl, 2-trimethylsilylethyl, trimethylsilyl, or t-butyldimethylsilyl. In a specific embodiment, the entire moiety R₃-OC(=O)- may be replaced with a carboxyl-masking moiety such as 2-alkyl-1,3-oxazolinyl.

The hydroxyl protecting group may be, for example, an arbitrary suitable protecting group for masking a hydroxyl group in organic synthesis. For example, the hydroxyl protecting group may be acetyl, methyl, ethoxyethyl, benzoyl, benzyl, 4-methoxybenzyl, 3,4-dimethoxybenzyl, tetrahydropyranyl (THP), tetrahydrofuranyl (THF), tert-butyldimethylsilyl (TBDMS), trimethylsilyl (TMS), triethylsilyl (TES), triisopropylsilyl (TIPS), tert-butyldiphenylsilyl (TBDPS), tri-isopropylsilyloxymethyl (TOM), β-methoxyethoxymethyl (MEM), methoxymethyl (MOM), allyl or trityl.

In a specific embodiment, in Chemical Formula 2F, R₃ may be hydrogen and each R₄ may be hydrogen.

In a specific embodiment, W in Chemical Formula 1F may be -C(O)NR'-, where C(O) may be connected to a phenyl ring and NR' may be connected to a linker, for example, the first linker (or branched linkers).

In a specific embodiment, in Chemical Formula 1F, Z may be hydrogen and n may be 3.

In a specific embodiment, R₁ and R₂ in Chemical Formula 1F may each be hydrogen.

In a specific embodiment, the trigger unit may be represented by Chemical Formula 1F, where W may be - C(O)NR'-, where C(O) may be connected to a phenyl ring, NR' may be connected to the first linker (or branched linkers), each Z may be hydrogen, n may be 3, m may be 1, and R₁ and R₂ may each be hydrogen. In this case, G may be a compound represented by Chemical Formula 2F.

In an embodiment of the present invention, the active agent may be a chemotherapeutic agent or a toxin. The active agent may be a drug, a toxin, an affinity ligand, a detection probe, or an arbitrary combination of these.

The active agent may be an immunomodulatory compound, an anti-cancer agent, an anti-viral agent, an anti-bacterial agent, an anti-fungal agent, an anti-parasitic agent, or any combination of these. Active agents selected from among the active agents listed below may be used:
(a) erlotinib, bortezomib, fulvestrant, sutent, letrozole, imatinib mesylate, PTK787/ZK 222584, oxaliplatin, 5-fluorouracil, leucovorin, rapamycin, lapatinib, lonafarnib, sorafenib, gefitinib, AG1478, AG1571, thiotepa, cyclophosphamide, busulfan, improsulfan, piposulfan, benzodopa, carboquone, meturedopa, uredopa, ethylenimine, altretamine, triethylenemelamine, trietylenephosphoramide, triethiylenethiophosphoramide, trimethylolomelamine, bullatacin, bullatacinone, camptothecin, topotecan, bryostatin, callystatin, CC-1065, adozelesin, carzelesin, bizelesin, cryptophycin 1, cryptophycin 8, dolastatin, duocarmycin, KW-2189, CB1-TM1, eleutherobin, pancratistatin, sarcodictyin, spongistatin, chlorambucil, chlornaphazine, cholophosphamide, estramustine, ifosfamide, mechlorethamine, melphalan, novembichin, phenesterine, prednimustine, trofosfamide, uracil mustard, carmustine, chlorozotocin, fotemustine, lomustine, nimustine, ranimnustine, calicheamicin, calicheamicin gamma 1, calicheamicin omega 1, dynemicin, dynemicin A, clodronate, esperamicin, neocarzinostatin chromophore, aclacinomysins, actinomycin, antrmycin, azaserine, bleomycins, cactinomycin, carabicin, carninomycin, carzinophilin, chromomycins, dactinomycin, daunorubicin, detorubucin, 6-diazo-5-oxo-L-norleucine, doxorubicin, morpholino-doxorubicin, cyanomorpholino-doxorubicin, 2-pyrrolino-doxorubucin, liposomal doxorubicin, deoxydoxorubicin, epirubicin, esorubicin, marcellomycin, mitomycin C, mycophenolic acid, nogalamycin, olivomycins, peplomycin, potfiromycin, puromycin, quelamycin, rodorubicin, streptomigrin, streptozocin, tubercidin, ubenimex, zinostatin, zorubicin, 5-fluorouracil, denopterin, methotrexate, pteropterin, trimetrexate, fludarabine, 6-mercaptopurine, thiamiprine, thiguanine, ancitabine, azacytidine, 6-azauridine, carmofur, cytarabine, dideoxyuridine, doxifluridine, enocitabine, floxuridine, calusterone, dromostanolone, propionate, epitiostanol, mepitiostane, testolactone, aminoglutethimide, mitotane, trilostane, folinic acid, aceglatone, aldophosphamide glycoside, aminolevulinic acid, eniluracil, amsacrine, bestrabucil, bisantrene, edatrexate, defofamine, demecolcine, diaziquone, elfornithine, elliptinium acetate, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, polysaccharide-k, razoxane, rhizoxin, sizofiran, spirogermanium, tenuazonic acid, triaziquone, 2,2',2"-trichlorotriethylamine, T-2 toxin, verracurin A, roridin A, anguidine, urethane, vindesine, dacarbazine, mannomustine, mitobronitol, mitolactol, pipobroman, gacytosine, arabinoside, cyclophosphamide, thiotepa, paclitaxel, albumin-engineered nanoparticle formulation of paclitaxel, docetaxel, chlorambucil, gemcitabine, 6-thioguanine, mercaptopurine, cisplatin, carboplatin, vinblastine, platinum, etoposide, ifosfamide, mitoxantrone, vincristine, vinorelbine, novantrone, teniposide, edatrexate, daunomycin, aminopterin, xeloda, ibandronate, CPT-11, topoisomerase inhibitor RFS 2000, difluoromethylornithine, retinoic acid, capecitabine, or any pharmaceutically acceptable salt, solvate or acid thereof;
(b) monokine, lympokine, traditional polypeptide hormone, parathyroid hormone, thyroxine, relaxin, prorelaxin, glycoprotein hormone, follicle stimulating hormone, thyroid stimulating hormone, luteinizing hormone, hepatic growth factor fibroblast growth factor, prolactin, placental lactogen, tumor necrosis factor-α, tumor necrosis factor-β, mullerian-inhibiting substance, mouse gonadotropin-associated peptide, inhibin, activin, vascular endothelial growth factor, thrombopoietin, erythropoietin, osteoinductive factor, interferon, interferon-α, interferon-β, interferon-γ, colony stimulating factor (CSF), macrophage-CSF, granulocytemacrophage-CSF, granulocyte-CSF, interleukin (IL), IL-1, IL-1α, IL-2, IL-3, IL-4, IL-5, IL-6, IL-7, IL-8, IL-9, IL-10, IL-11, IL-12, tumor necrosis factor, TNF-α, TNF-β, polypeptide factor, LIF, kit ligand, or any combination of these;
(c) diphtheria toxin, botulinum toxin, tetanus toxin, dicenteritoxin, cholera toxin, amanitin, α-amanitin, pyrrolobenzodiazepine, pyrrolobenzodiazepine derivatives, tetrodotoxin, brevetoxin, ciguatoxin, ricin, AM toxin, auristatin, tubulysin, geldanamycin, maytansinoid, calicheamicin, daunomycin, doxorubicin, methotrexate, vindesine, SG2285, dolastatin, dolastatin analog, auristatin, cryptophycin, camptothecin, rhizoxin, rhizoxin derivatives, CC-1065, CC-1065 analogs or derivatives, duocarmycin, enediyne antibiotic, esperamicin, epothilone, toxoid, or any combination of these;
(d) an affinity ligand, wherein the affinity ligand is a substrate, an inhibitor, an activator, a neurotransmitter, a radioactive isotope, or any combination of these;
(e) radioactive label, 32P, 35S, fluorescent die, electron density reagent, enzyme, biotin, streptavidin, dioxigenin, hapten, immunogenic protein, nucleic acid molecule with a sequence complementary to a target, or any combination of these;
(f) an immunomodulatory compound, an anti-cancer agent, an anti-viral agent, an anti-bacterial agent, an anti-fungal agent, and an anti-parasitic agent, or any combination of these;
(g) tamoxifen, raloxifene, droloxifene, 4-hydroxytamoxifen, trioxifene, keoxifene, LY117018, onapristone, or toremifene;
(h) 4(5)-imidazole, aminoglutethimide, megestrol acetate, exemestane, letrozole, or anastrozole;
(i) flutamide, nilutamide, bicalutamide, leuprolide, goserelin, or troxacitabine;
(j) an aromatase inhibitor;
(k) a protein kinase inhibitor;
(l) a lipid kinase inhibitor;
(m) shRNA, siRNA, PNA, or anti-sense oligonucleotide;
(n) a ribozyme;
(o) a vaccine;
(p) an anti-angiogenic agent; and
(q) an immuno-oncology therapeutic agent.

The immuno-oncology therapeutic agent may be selected from an antibody, a peptide, a protein, a small molecule, an adjuvant, a cytokine, an oncolytic virus, a vaccine, a bi-specific molecule, a cell therapeutic agent, a checkpoint inhibitor, a STING agonist, an adenosine receptor antagonist, and any combination of these. The checkpoint inhibitor may be an inhibitor of a receptor selected from PD-1, PD-L1 or CTLA-4.

In an embodiment of the present invention, the active agent may be any one among active agents represented by the following Chemical Formulas: 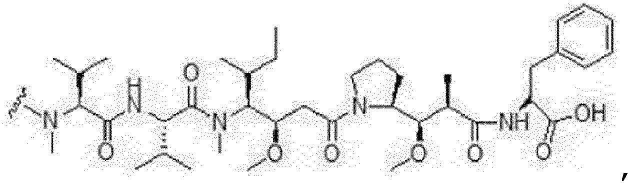 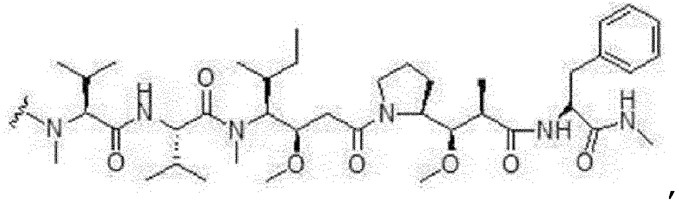 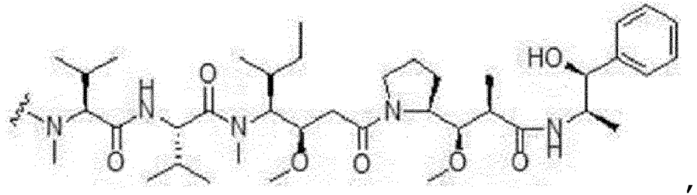 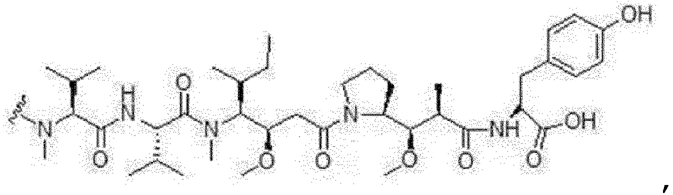 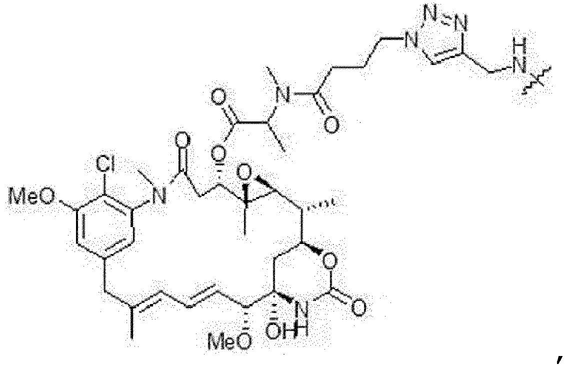 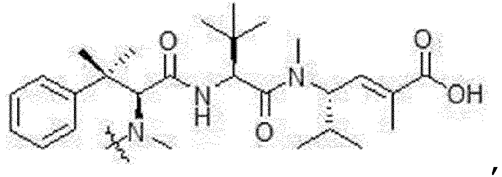 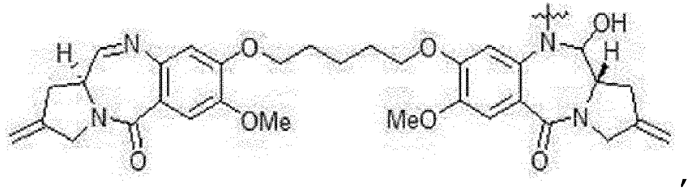 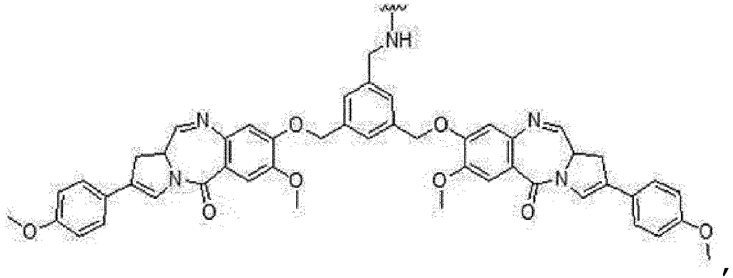 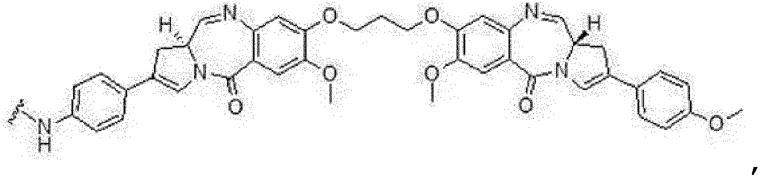 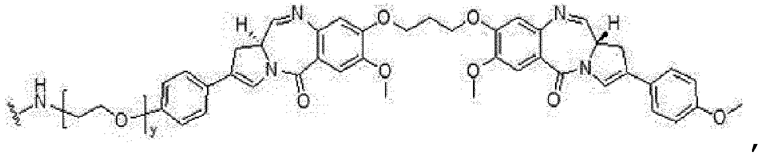 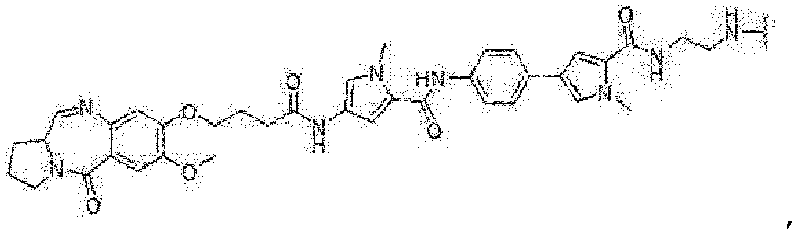 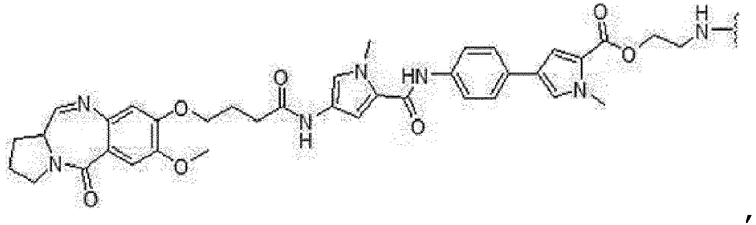 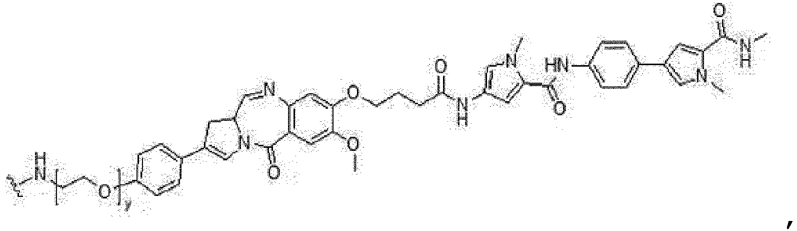 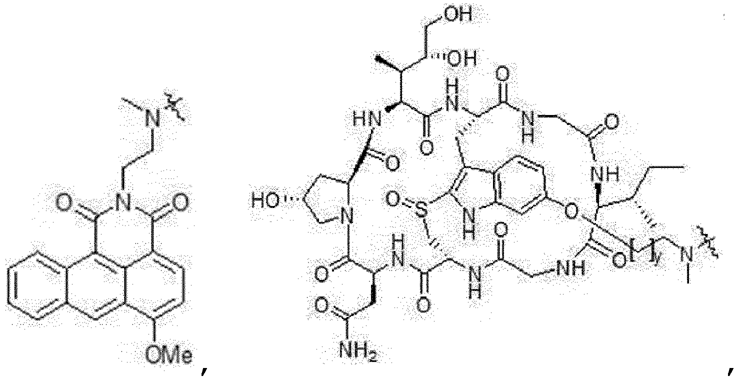 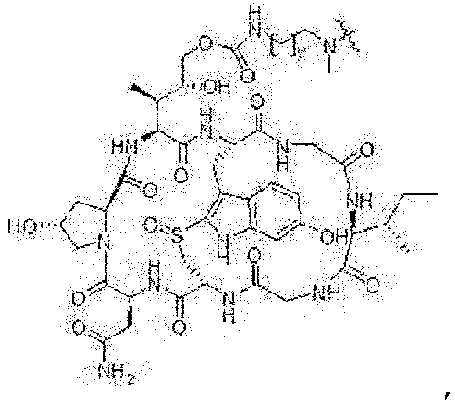 or 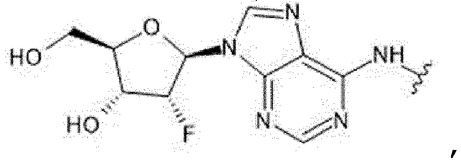 where y is an integer from 1 to 10.

In an embodiment, the linker may be represented by the following structure. * indicates the site connected to the active agent (drug or toxin).

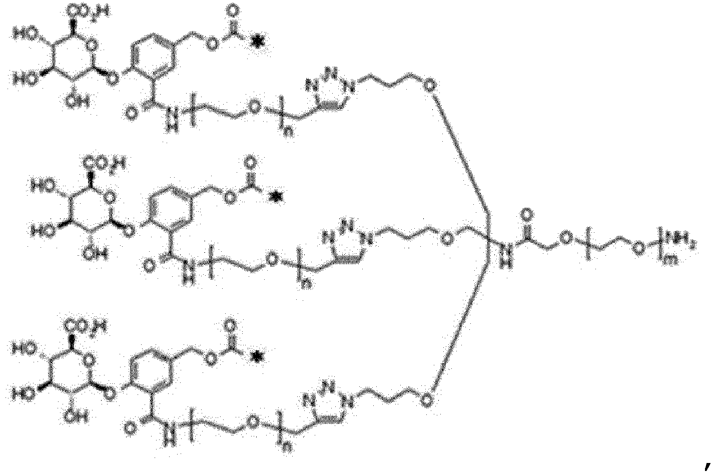

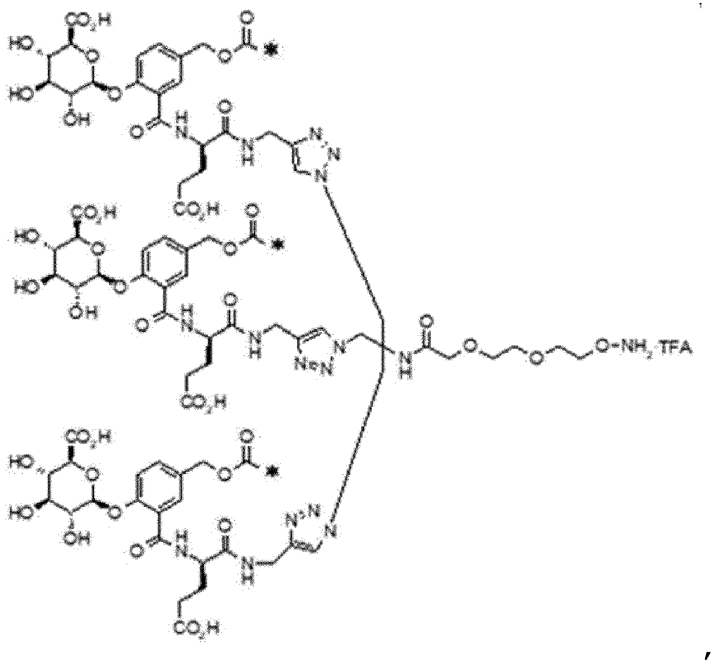

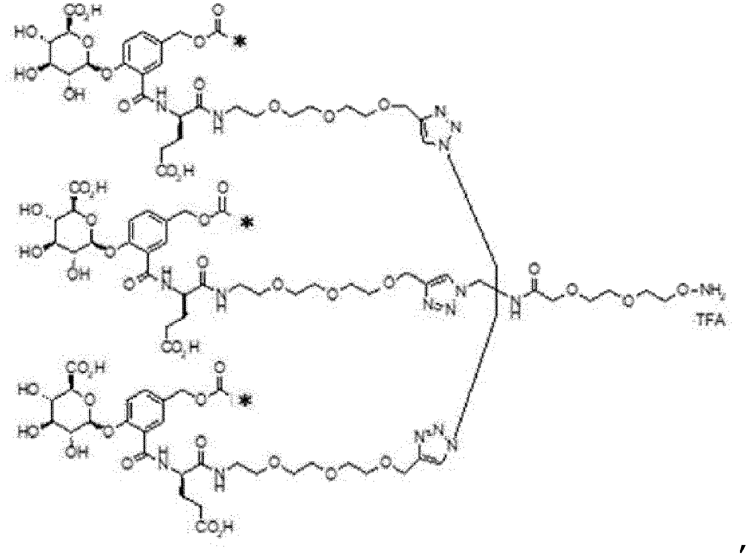

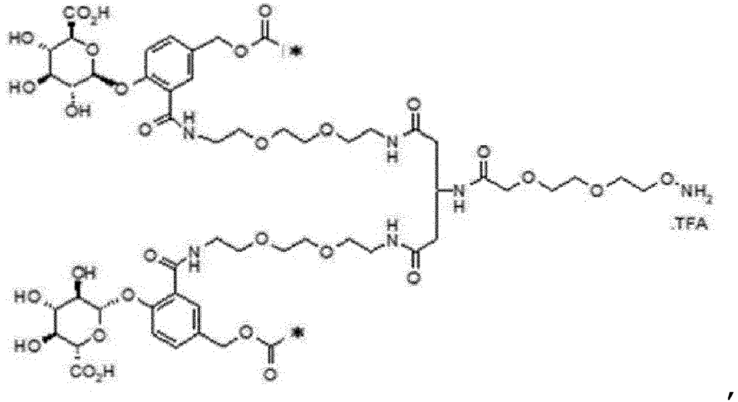

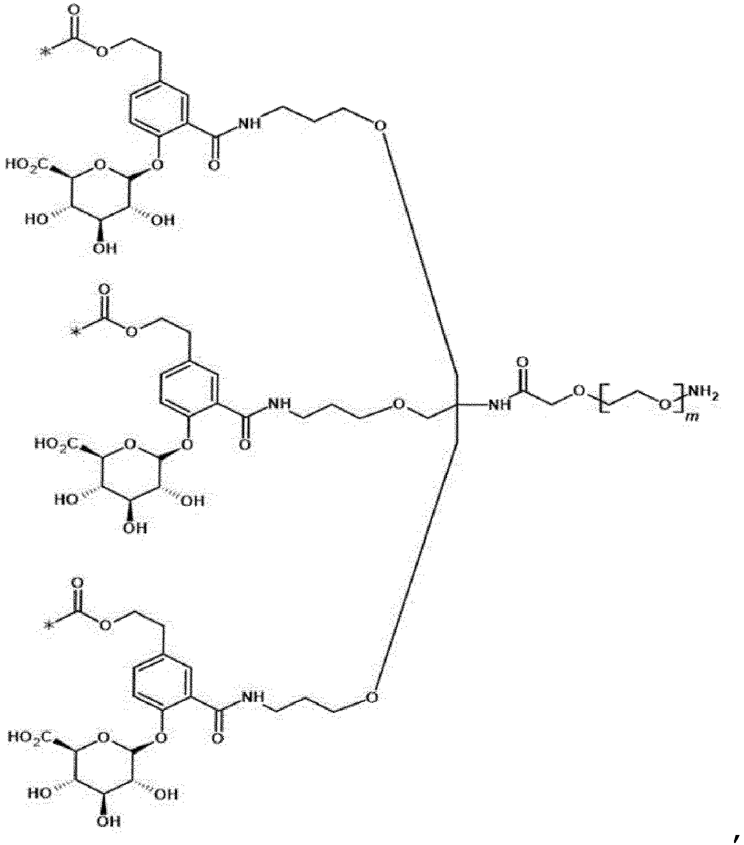

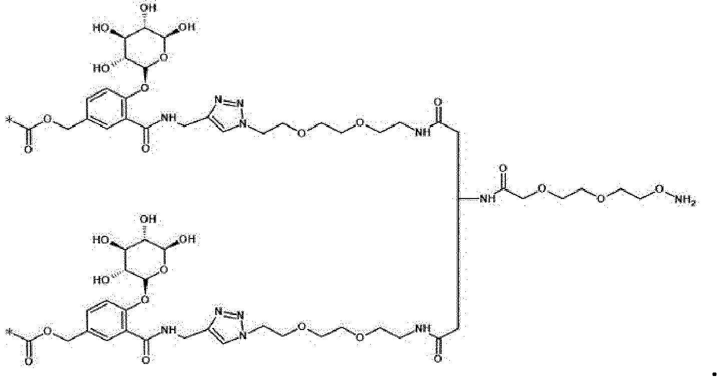

The ligand-drug conjugate according to an embodiment of the present invention may be prepared by methods known in the art, including molecular biology and cell biology methods. For example, transient or stable transfection may be used. An antibody having a specific amino acid motif at the C-terminus may be expressed by inserting a gene sequence encoding a specific amino acid motif that may be recognized by isoprenoid transferase into a known plasmid vector using standard PCR and/or ligation techniques. Hence, an antibody having at least one or more amino acid motifs that may be recognized by isoprenoid transferase may be expressed in an appropriate host, for example, in CHO cells or **E.** coli.

In another aspect of the present invention, there is provided a pharmaceutical composition for the prevention or treatment of a hyperproliferative, cancer or angiogenic disease, containing a ligand-drug conjugate or a pharmaceutically acceptable salt or solvate thereof as an active ingredient.

The cancer may be selected from the group consisting of lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.

The ligand-drug conjugate may be used to deliver an active agent to a target cell of a subject for treatment of the subject.

The composition may be prepared in an injectable form as a liquid solution or a suspension. The composition may also be prepared in a solid form suitable for injection, for example, as an emulsion or together with a ligand-drug conjugate encapsulated in a liposome.

In an embodiment of the present invention, the ligand-drug conjugate may be combined with a pharmaceutically acceptable carrier, including an arbitrary carrier that does not induce production of an antibody in a subject to whom the carrier is administered. Suitable carriers typically include, for example, slowly metabolized macromolecules such as proteins, polysaccharides, polylactic acid, polyglycolic acid, polymeric amino acids, amino acid copolymers, and lipid aggregates.

The composition may contain diluents, for example, water, saline, glycerol and ethanol. As auxiliary substances, for example, wetting or emulsifying agents, pH buffering substances and the like may also be contained. The composition may be administered parenterally by injection, and may be injected subcutaneously or intramuscularly. In some embodiments, the composition may be administered intratumorally. The composition may be inserted (for example, injected) into a tumor. Further formulations are suitable for other dosage forms, for example, by suppository or orally. An oral composition may be administered as a solution, suspension, tablet, pill, capsule, or sustained-release formulation.

The composition may be administered in a manner compatible with the dosage and formulation.

The composition may further contain a chemotherapeutic agent in a therapeutically effective amount. In a specific embodiment, one or more antihyperproliferative, cytostatic or cytotoxic substances may be administered in combination.

The term "therapeutically effective amount" refers to amound of a composition administered on a single dose or multiple dose schedule effective for the treatment or prevention of a disease or disorder. The dosage may vary depending on the subject being treated, the health and physical conditions of the subject, the degree of protection desired, and other relevant factors. The exact amount of active ingredient (for example, antibody-drug conjugate) is at the discretion of the physician. For example, a therapeutically effective amount of an antibody-drug conjugate or a composition containing the antibody-drug conjugate may be administered to a patient suffering from cancer or tumor to treat the cancer or tumor.

The ligand-drug conjugate or a composition containing the antibody-drug conjugate according to the present invention may be administered in the form of a pharmaceutically acceptable salt or solvate thereof.

In a specific embodiment, the ligand-drug conjugate or a composition containing the antibody-drug conjugate according to the present invention may be administered together with a pharmaceutically acceptable carrier, a pharmaceutically acceptable excipient and/or pharmaceutically acceptable additives. Effective amounts and types of pharmaceutically acceptable salts or solvates, excipients and additives may be measured using standard methods (for example, see Remington's Pharmaceutical Sciences, Mack Publishing Co., Easton, PA, 18th Edition, 1990).

The term "therapeutically effective amount" refers to an amount capable of decreasing the number of cancer cells; decreasing the cancer cell size; inhibiting or decreasing invasion of cancer cells into surrounding lineages; inhibiting or decreasing the spread of cancer cells to other lineages; inhibiting the growth of cancer cells; or ameliorating one or more symptoms associated with cancer. In the treatment of cancer, the effectiveness of a drug may be tested by tumor-to-tumor progression (TTP) and/or response (reaction) rate (RR). As used herein, the term "pharmaceutically acceptable salt" includes organic salts and inorganic salts. Examples thereof include, but are not limited to, hydrochloride, hydrobromide, hydroiodide, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantonate, bitartrate, ascorbate, succinate, maleate, gentisinate, fumarate, gluconate, glucoronate, saccharate, formate, benzoate, glutamate, methane sulfonate, ethane sulfonate, benzene sulfonate, p-toluene sulfonate, and pamoate (namely, 1,1'-methylenebis-(2-hydroxy-3-naphthoate)). Pharmaceutically acceptable salts may contain other molecules (for example, acetate ions, succinate ions and other counterions).

Exemplary solvates that can be used as a pharmaceutically acceptable solvate of the ligand-drug conjugate according to the present invention include, but are not limited to, water, isopropanol, ethanol, methanol, DMSO, ethyl acetate, acetic acid, and ethanolamine.

In still another aspect of the present invention, there is provided a method of treating cancer in a subject by administering the pharmaceutical composition for the prevention or treatment of a hyperproliferative, cancer or angiogenic disease, containing the ligand-drug conjugate according to an embodiment of the present invention to the subject.

In a specific embodiment, the subject may be a mammal. For example, the subject may be selected from rodents, lagomorphs, felines, canines, porcines, ovines, bovines, equines, or primates. In a specific embodiment, the subject may be a human.

### [Definition]

The term "antibody" refers to an immunoglobulin molecule that recognizes and specifically binds to another molecule through at least one antigen recognition site within the variable region of the immunoglobulin molecule. As used herein, the term "antibody" includes an intact polyclonal antibody, an intact monoclonal antibody, antibody fragments (for example, Fab, Fab', F(ab')2, Fd, and Fv fragments), single chain Fv (scFv) mutants, a multispecific antibody, such as a bispecific antibody generated from two or more intact antibodies, a chimeric antibody, a humanized antibody, a human antibody, a fusion protein including an epitope of an antibody, and arbitrary another modified immunoglobulin molecule including an antigen recognition site. Antibodies may be any of the five main immunoglobulin classes: IgA, IgD, IgE, IgG and IgM based on the identity of their heavy chain constant domains, referred to as alpha, delta, epsilon, gamma, and mu, respectively, or subclasses (isotypes) thereof (for example, IgG1, IgG2, IgG3, IgG4, IgA1, and IgA2). Different kinds of immunoglobulins have different well-known subunit structures and three-dimensional structures. The term "antibody" does not refer to a molecule that does not share homology with an immunoglobulin sequence. For example, the term "antibody" as used herein does not include "repebodies".

The term "antibody fragment" refers to a portion of an intact antibody, and refers to the epitope variable region of an intact antibody. Examples of antibody fragments include Fab, Fab', F(ab')2, Fd, and Fv fragments, linear antibodies, single chain antibodies, and multispecific antibodies formed from antibody fragments.

The term "monoclonal antibody" refers to a homogeneous population of antibodies that highly specifically recognize and bind to a single antigenic determinant or epitope. This is in contrast to polyclonal antibodies, which typically include other antibodies directed against a variety of different antigenic determinants. The term "monoclonal antibody" includes, but is not limited to, antibody fragments (for example, Fab, Fab', F(ab')2, Fd, and Fv), single chain (scFv) mutants, fusion proteins including antibody portions, and arbitrary other modified immunoglobulin molecules including antigen recognition sites as well as all intact full-length monoclonal antibodies. The term "monoclonal antibody" refers to an antibody prepared by an arbitrary number of methods, including, but not limited to, hybridomas, phage selection, recombinant expression and transgenic animals.

The term "humanized antibody" refers to a form of a non-human (for example, murine) antibody that is a specific immunoglobulin chain, chimeric immunoglobulin, or fragment thereof including minimal non-human (for example, murine) sequence. In general, humanized antibodies are human immunoglobulins substituted with residues from the CDRs of non-human species (for example, murine, rat, rabbit and hamster) in which the complementarity determining regions (CDRs) have the desired specificity, affinity and binding capability (for example, see Jones et al., Nature, 321:522-525 (1986); Riechmann et al., Nature, 332:323-327 (1988); Verhoeyen et al., Science, 239:1534-1536 (1988)). In some examples, Fv framework region (FR) residues of human immunoglobulins are replaced with corresponding residues of an antibody from a non-human species having the desired specificity, affinity, and binding capability. Humanized antibodies may be further modified by substituting additional residues within the Fv framework regions and/or replaced non-human residues to improve and optimize antibody specificity, affinity and/or binding capability. In general, a humanized antibody substantially includes at least one, typically two or three variable domains containing all or substantially all of the CDRs corresponding to a non-human immunoglobulin, whereas a humanized antibody has all or substantially all of the framework region (FR) human immunoglobulin consensus sequence. A humanized antibody may also include at least part of an immunoglobulin constant region or domain (Fc), typically part of a human immunoglobulin. Examples of methods used to produce humanized antibodies are described in US Pat. No. 5,225,539, which is incorporated herein by reference.

The term "human antibody" as used herein refers to an antibody encoded by a human nucleotide sequence or antibody having an amino acid sequence corresponding to an antibody prepared by a human using an arbitrary technique known in the art. This definition of human antibody includes full-length antibodies and/or fragments thereof.

The term "chimeric antibody" refers to an antibody in which the amino acid sequence of an immunoglobulin molecule is derived from two or more species, one of which is preferably human. In general, the variable regions of the light and heavy chains correspond to the variable regions of an antibody derived from one species of mammal (for example, mouse, rat, or rabbit) having the desired specificity, affinity and binding capability, and the constant region is homologous to the sequence of an antibody derived from another species (usually human), for example, to avoid inducing an immune response in that species.

The terms "epitope" and "antigenic determinant" are used interchangeably herein and refer to a portion of an antigen capable of being recognized by and specifically bound to a particular antibody. When the antigen is or includes a polypeptide or a protein, the epitope may be formed from amino acids that are juxtaposed, contiguous and/or non-contiguous, for example, by secondary, tertiary and/or quaternary folding of the protein. Epitopes formed from contiguous amino acids are typically retained upon protein denaturation, whereas epitopes formed by tertiary folding may be lost upon protein denaturation. An epitope typically includes 3 or more, 5 or more, or 8 to 10 or more amino acids in a unique spatial conformation.

The fact that an antibody "specifically binds" to an epitope or antigenic molecule means that an antibody interacts or associates with an epitope or antigenic molecule more frequently, more rapidly, for a longer period of time, with greater affinity, or with some combination of the foregoing than an alternative substance including an unrelated protein. In several specific embodiments, to "specifically bind" means, for example, that the antibody binds to a protein with a KD of about 0.1 mM or less, more generally less than about 1 µM. In a certain specific embodiment, to "specifically bind" means that the antibody binds to a protein with a KD of about 0.1 µM or less, and with a KD of about 0.01 µM or less at other times. Because of sequence homology between homologous proteins in different species, specific binding may involve antibodies that recognize specific proteins in more than one species. It is understood that an antibody or binding moiety that specifically binds to a first target may or may not specifically bind to a second target. As described above, "specific binding" does not necessarily require (although it may include) exclusive binding, namely, binding to a single target. In general, though not necessarily, the term binding as used herein refers to specific binding.

Antibodies including the fragments/derivatives and monoclonal antibodies may be obtained by methods known in the art (see McCafferty et al., Nature 348 : 552-554 (1990); Clackson et al., Nature 352 : 624 Marks et al., J. Mol. Biol., 222 : 581-597 (1991), Marks et al., Bio/Technology 10 : 779-783 (1992), Waterhouse et al., Nucleic Acids Res. 21 : 2265-2266 (1993); Morimoto et al., J Biochemical & Biophysical Methods 24 : 107-117 (1992), Brennan et al., Science 229 : 81 (1985), Carter et al., Bio/Technology 10 : Kilpatrick et al., Hybridoma 16 (4) : 381-389 (1997), Wring et al., J. Pharm. Biomed. Anal., 163-167 (1992), Kohler et al., Nature 256 : 495 (1975) Bynum et al., Hybridoma 18 (5) : 407-411 (1999), Jakobovits et al., Proc. Natl. Acad. Sci. USA, 90 : 2551 (1999)], 19 (5) : 695-707 Barbus et al., Proc. Nat. Acad. Sci. USA, 91 : 517-536), Jakobovits et al., Nature, 362 : 255-258 (1993); Bruggemann et al., Year Immuno. 7:33 (1993) 3809-3813 (1994); Schier et al., Gene 169 : 147-155 (1995), Yelton et al., J. Immunol. 155 : 1994-2004 (1995); Jackson. et al., J. Immunol. 154 (7) : 3310-9 (1995); Hawkins et al., J. Mol. Biol. 226 : 889-896 (1992), U.S. Patent Nos. 4,816,567, 5,514,548, 5,545,806, 5,569,825, 5,591,669, and 5,545,807; PCT Patent Application Publication No. WO97/17852, which are all incorporated herein by reference in their entirety).

The antibody may be muromonab-CD3 abciximab, rituximab, daclizumab, palivizumab, infliximab, trastuzumab, etanercept, basiliximab, gemtuzumab, alemtuzumab, ibritumomab, adalimumab, alefacept, omalizumab, efalizumab, tositumomab, cetuximab, ABT -806, bevacizumab, natalizumab, ranibizumab, panitumumab, eculizumab, rilonacept, certolizumab, romiplostim, AMG-531, golimumab, ustekinumab, ABT-874, belatacept, belimumab, atacicept, anti-CD20 antibody, canakinumab, tocilizumab, atlizumab, mepolizumab, pertuzumab, HuMax CD20, tremelimumab, ticilimumab, ipilimumab, anti-CTLA-4 antibody, IDEC-114, inotuzumab, HuMax EGFR, aflibercept, HuMax-CD4, teplizumab, otelixizumab, catumaxomab, anti-EpCAM antibody IGN101, adecatumomab, oregovomab, dinutuximab, girentuximab, denosumab, bapineuzumab, motavizumab, efumgumab, raxibacumab, anti-CD20 antibody, LY2469298, pembrolizumab, nivolumab, pidilizumab, anti-PD-1 antibody, BMS-936559, durvalumab, avelumab, atezolizumab, MDX-1105, anti-PD-Ll antibody, and veltuzumab.

When an antibody includes at least one light chain and at least one heavy chain, at least one light chain of the antibody, or at least one heavy chain of the antibody, or both of these may include amino acid regions having amino acid motifs that may be recognized by isoprenoid transferase. An antibody may include four polypeptide chains (for example, two heavy chains and two light chains), and thus the antibody may include four amino acid motifs, each of which is used to conjugate an active agent to the antibody via a linker. Hence, the antibody-drug conjugate includes four linkers, each of which is conjugated to at least one active agent. Hence, the antibody-drug conjugate may include at least one linker and at least two active agents. The antibody-drug conjugate may include at least two linkers, and the antibody-drug conjugate may include at least three active agents. The antibody-drug conjugate may include 1, 2, 3 or 4 linkers. The antibody-drug conjugate may include 1, 2, 3 or 4 peptides. The antibody-drug conjugate may include 2 to 100 conjugates, for example, 2 to 50 conjugates, 2 to 20 conjugates, 2 to 16 conjugates, 4 to 16 conjugates, or 4 to 8 conjugates.

The active agent may be a drug, a toxin, an affinity ligand, a detection probe, or an arbitrary combination of these.

The active agent may be erlotinib; bortezomib; fulvestrant; sutent; letrozole; imatinib mesylate; PTK787/ZK 222584; oxaliplatin; 5-fluorouracil; leucovorin; rapamycin; lapatinib; lonafarnib; sorafenib; gefitinib; AG1478; AG1571; alkylating agents (for example, thiotepa and cyclophosphamide); alkyl sulfonates (for example, busulfan, improsulfan, and piposulfan); aziridine (for example, benzodopa, carboquone, meturedopa, and uredopa); ethylenimine, methylmelamine, altretamine, triethylenemelamine, triethylenephosphoramide, triethylenethiophosphoramide, trimethylolomelamine; acetogenins (for example, bullatacin or bullatacinone); camptothecin and camptothecin derivatives and metabolites (SN-38); topotecan; bryostatin; callystatin; CC-1065 (including its adozelesin, carzelesin, or bizelesin synthetic analogs); cryptophycins (for example, cryptophycin 1 or cryptophycin 8); dolastatin; duocarmycin (including synthetic analogs such as KW-2189 and CB1-TM1); eleutherobin; pancratistatin; sarcodictyin; spongestatin; nitrogen mustard (for example, chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, or uracil mustard); nitrousurea (for example, carmustine, chlorozotocin, fotemustine, lomustine, nimustine, or ranimnustine); antibiotics (for example, enediyne antibiotics such as calicheamicin selected from calicheamicin gamma 1I and calicheamicin omega 1I or dynemycin including dynemycin A); bisphosphonate (for example, clodronate; esperamicin, neocarzinostatin chromophore, or related chromoprotein enediyne antibiotic chromophores), aclacinomysins, actinomycin, anthramycin, azaserine, bleomycins, cactinomycin, carabicin, carninomicin, carzinophilin, chromomycins, dactinomycin, daunorubicin, detorubucin, 6-diazo-5-oxo-L-norleucine, doxorubicin (for example, morpholino-doxorubicin, cyanomorpholino-doxorubicin, 2-pyrrolino-doxorubicin, liposomal doxorubicin, or deoxydoxorubicin), epirubicin, esorubicin, marcellomycin, mitomycins (for example, mitomycin C, mycophenolic acid, nogalamycin, olivomycins, peplomycin, potfiromycin, puromycin, quelamycin, rodorubicin, streptomigrin, streptozocin, tubercidin, ubenimex, zinostatin, or zorubicin); anti-metabolites (for example, 5-fluorouracil); folic acid analogs (for example, denopterin, methotrexate, pteropterin, or trimetrexate); purine analogs (for example, fludarabine, 6-mercaptopurine, thiamiprine, or thiguanine); pyrimidine analogs (for example, ancitabine, azacitidine, 6-azauridine, carmofur, cytarabine, dideoxyuridine, doxifluridine, enocitabine, or floxuridine); androgens (for example, calusterone, dromostanolone propionate, epithiostanol, mepitiostane, or testolactone); antiadrenals (for example, aminoglutethimide, mitotane, or trilostane); folic acid replenishers (for example, folinic acid); aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; epothilone; etoglucid; gallium nitrate; hydroxyurea; lentinan; lonidainine; maytansinoids (for example, maytansine or ansamitocins); trichothecenes (especially T-2 toxin, verracurin A, roridin A, or anguidine); mitoguazone; mitoxantrone; mopidanmol; nitraerine; pentostatin; phenamet; pirarubicin; losoxantrone; 2-ethylhydrazide; procarbazine; polysaccharide K complex; razoxane; rhizoxin; sizofiran; spirogermanium; tenuazonic acid; triaziquone; 2,2',2"-trichlorotriethylamine; trichothecenes (especially T-2 toxin, verracurin A, roridin A, and anguidine); urethane; vindesine; dacarbazine; mannomustine; mitobronitol; mitolactol; pipobroman; gacytosine; arabinoside; cyclophosphamide; thiotepa; taxoids (for example, paclitaxel, and ABRAXANE^{™} cremophor-free, albumin-engineered nanoparticle formulation of paclitaxel, docetaxel; chlorambucil; gemcitabine; 6-thioguanine; mercaptopurine; platinum analogs (for example, cisplatin or carboplatin); vinblastine; platinum; etoposide; ifosfamide; mitoxantrone; vincristine; vinorelbine; novantron; teniposide; edatrexate; daunomycin; aminopterin; xeloda; ibandronate; CPT-11; topoisomerase inhibitor (RFS 2000); difluoromethylornithine; retinoid (for example, retinoic acid); capecitabine and pharmaceutically acceptable salts, solvates, acids or derivatives thereof, but is not necessarily limited thereto.

The active agent may be selected from (i) antihormonal agents, which modulate or inhibit hormonal action in tumors, such as anti-estrogen and selective estrogen receptor modulators including, for example, tamoxifen, raloxifene, droloxifene, 4-hydroxytamoxifen, trioxifene, keoxifene, LY117018, onapristone, and toremifene; (ii) aromatase inhibitors, which inhibit aromatase enzymes which modulate estrogen production in the adrenal glands, for example, 4(5)-imidazoles, aminoglutethimide, megestrol acetate, exemestane, letrozole, and anastrozole; (iii) anti-androgens such as flutamide, nilutamide, bicalutamide, leuprolide, and goserelin; as well as troxacitabine (1,3-dioxolane nucleoside cytosine analog); (iv) aromatase inhibitors; (v) protein kinase inhibitors; (vi) lipid kinase inhibitors; (vii) antisense oligonucleotides, especially those that inhibit the expression of genes in signaling pathways involved in adherent cells, for example, PKCalpha, Raf, and H-Ras; (viii) VEGF inhibitors such as ribozymes, for example, ribozymes and HER2 expression inhibitors; (ix) vaccines, for example, gene therapy vaccines; ALLOVECTIN^{®} Vaccine, LEUVECTIN Vaccine, and VAXID Vaccine; PROLEUKIN^{®} rlL-2; LURTOTECAN^{®} topoisomerase 1 inhibitor; and ABARELIX^{®} rmRH; (x) anti-angiogenic agents such as Bevacizumab; or (xi) pharmaceutically acceptable salts, solvating agents, acids or derivatives thereof.

Cytokines may be used as the active agent. Cytokines are small cell signaling protein molecules secreted by numerous cells, and are a category of signaling molecules widely used in intercellular communication. Cytokines include monokines, lymphokines, and typical polypeptide hormones. Examples of cytokines include, but are not limited to, growth hormone (for example, human growth hormone, N-methionyl human growth hormone, or bovine growth hormone); parathyroid hormone; thyroxine; insulin, proinsulin, relaxin; prorelaxin; glycoprotein hormones (for example, follicle stimulating hormone (FSH), thyroid stimulating hormone (TSH), and luteinizing hormone (LH)); hepatic growth factor; fibroblast growth factor; prolactin; placental lactogen; tumor necrosis factor-α, tumor necrosis factor-β; mullerian-inhibiting substance; mouse gonadotropin-associated peptide; inhibin; activin; vascular endothelial growth factor; integrin, thrombopoietin (TPO); nerve growth factor (for example, NGF-β); platelet-growth factor; transforming growth factor (TGF) (for example, TGF-α or TGF-β)); insulin-like growth factor-I, insulin-like growth factor-II; erythropoietin (EPO); osteoinductive factor; interferons (for example, interferon-α, interferon-β, or interferon-γ); colony stimulating factor (CSF) (for example, macrophage-CSF (M-CSF), granulocyte-macrophage-CSF (GM-CSF), or granulocyte-CSF (G-CSF)); interleukin (IL) (for example, IL-1, IL-1α, IL-2, IL-3, IL-4, IL-5, IL-6, IL-7, IL-8, IL-9, IL-10, IL-11, or IL-12); tumor necrosis factor (TNF) (for example, TNF-α or TNF-β); and polypeptide factor (for example, LIF or kit ligands). The term "cytokine" includes cytokines from natural sources or recombinant cell culture and biologically active equivalents of native sequence cytokines.

The term "toxin" refers to a substance that is toxic to living cells or organisms. Toxins may be small molecules, peptides or proteins that can cause cell dysfunction or cell death after contact with or absorption into body tissues, for example, through interaction with one or more biological macromolecules such as enzymes or cell receptors. Toxins include plant toxins and animal toxins. Examples of animal toxins include, but are not limited to, diphtheria toxin, botulinum toxin, tetanus toxin, heterotoxin, cholera toxin, tetrodotoxin, brevetoxin and ciguatoxin. Examples of plant toxins include, but are not limited to, ricin and AM-toxin.

Examples of small molecule toxins include, but are not limited to, auristatin, tubulysin, geldanamycin (Kerr et al., 1997, Bioconjugate Chem. 8(6):781-784), maytansinoid (EP 1391213, ACR 2008, 41, 98-107), calicheamicin (US Patent Publication No. 2009/0105461, Cancer Res. 1993, 53, 3336-3342), daunomycin, doxorubicin, methotrexate, vindesine, SG2285 (Cancer Res. 2010, 70(17), 6849-6858), dolastatin, dolastatin analogs auristatin (US Patent No. 5,635,483), cryptophycin, camptothecin, rhizoxin derivative, CC 1065 analogs or derivatives, duocarmycin, enediyne antibiotic, esperamicin, epothilone, pyrrolobenzodiazepine (PBD) derivatives, α-amanitin, and toxoid. Toxins may exhibit cytotoxicity and cell growth-inhibiting activity by tubulin binding, DNA binding, topoisomerase inhibition, and the like.

A "detectable moiety" or "marker" refers to a composition detectable by spectroscopic, photochemical, biochemical, immunochemical, radioactive or chemical means. For example, useful labels include ³²P, ³⁵S, fluorescent dyes, electron-dense reagents, enzymes (example: those commonly used in ELISAs), biotinstreptavidin, dioxygenin, hapten, and proteins for which antisera or monoclonal antibodies are available, or nucleic acid molecules having sequences complementary to the target. A detectable moiety often generates a measurable signal, for example, a radioactive, chromogenic or fluorescent signal, which may be used to quantify the amount of bound detectable moiety in a sample. Quantification of the signal is accomplished by, for example, scintillation counting, densitometry, flow cytometry, ELISA or direct analysis by mass spectrometry of the original or subsequently digested peptides (one or more peptides may be assayed).

As used herein, the term "probe" refers to a substance capable of (i) providing a detectable signal, (ii) interacting with the first probe or the second probe to alter a detectable signal provided by the first or second probe, such as fluorescence resonance energy transfer (FRET); (iii) stabilizing the interaction with the antigen or ligand or increasing binding affinity; (iv) affecting electrical mobility or cell-invasive action by physical parameters such as charge and hydrophobicity; or (v) modulating ligand affinity, antigen-antibody binding, or ionic complex formation.

The active agent includes an immunomodulatory compound, an anti-cancer agent, an anti-viral agent, an anti-bacterial agent, an anti-fungal agent, an anthelmintic agent, or any combination of these.

The immunomodulatory compound may be selected from aminocaproic acid, azathioprine, bromocriptine, chlorambucil, chloroquine, cyclophosphamide, cyclosporine, cyclosporine A, danazol, dehydroepiandrosterone, dexamethasone, etanercept, hydrocortisone, hydroxychloroquine, infliximab, meloxicam, methotrexate, mycophenylate mofetil, prednisone, sirolimus (sirolimus), or tacrolimus. The anti-cancer agent may be selected from 1-methyl-4-phenylpyridinium ion, 5-ethynyl-1-beta-*D*-ribof uranosylimidazole-4-carboxamide (EICAR), 5-fluorouracil, 9-aminocamptothecin, actinomycin D, asparaginase, bicalutamide, bis-chloroethylnitrosourea (BCNU), bleomycin, bleomycin A2, bleomycin B2, busulfan, camptothecin, carboplatin, carmustine, CB1093, chlorambucil, cisplatin, cristol, cyclophosphamide, cytarabine, cytosine arabinoside, cytoxan, dacarbazine, dactinomycin, daunorubicin, decarbazine, deferoxamine, demethoxy-hypocrellin A, docetaxel, doxifluridine, doxorubicin, EB1089, epirubicin, etoposide, floxuridine, fludarabine, flutamide, gemcitabine, goserelin, hydroxyurea, idarubicin, isopfamide, interferon-α, interferon-γ, irinotecan, KH1060, leuprolide acetate, lomustine, lovastatin, megestrol, melphalan, mercaptopurine, methotrexate, mitomycin, mitomycin C, mitoxantrone, mycophenolic acid, nitrogen mustard, nitrosourea, paclitaxel, peplomycin, photosensitizer Pe4, phthalocyanine, pirarubicin, plicamycin, procarbazine, raloxifene, raltitrexed, revlimid, ribavirin, staurosporine, tamoxifen, teniposide, thalomid, thapsigargin, thioguanine, tiazofurin, topotecan, treosulfan, trimetrexate, tumor necrosis factor, velcade, verapamil, verteporfin, vinblastine, vincristine, vinorelbine, or zorubicin. The anti-viral agent may be selected from pencicyclovir, valacyclovir, gancicyclovir, foscarnet, ribavirin, idoxuridine, vidarabine, trifluridine, acyclovir, famcicyclovir, amantadine, rimantadine, cidofovir, antisense oligonucleotide, immunoglobulin, or interferon. The anti-bacterial agent may be selected from chloramphenicol, vancomycin, metronidazole, trimethoprin, sulfamethazole, quinupristin, dalfopristin, rifampin, spectinomycin, or nitrofurantoin. The anti-fungal agent may be selected from amphotericin B, candicidin, filipin, hamycin, natamycin, nystatin, rimocidin, bifonazole, butoconazole, clotrimazole, econazole, fenticonazole, isoconazole, ketoconazole, luliconazole, miconazole, omoconazole, oxiconazole, sertaconazole, sulconazole, tioconazole, albaconazole, fluconazole, isavuconazole, itraconazole, posaconazole, ravuconazole, terconazole, voriconazole, abafungin, amorolfin, butenafine, naftifine, terbinafine, anidulafungin, caspofungin, micafungin, benzoic acid, ciclopirox, flucytosine, griseofulvin, haloprogin, tolnaftate, undecylenic acid, crystal violet, balsam of peru, ciclopirox olamine, piroctone olamine, zinc pyrithione, or selenium sulfide. The anti-parasitic agent may be selected from mebendazole, pyrantel pamoate, thiabendazole, diethylcarbamazine, ivermectin, niclosamide, praziquantel, albendazole, rifampin, amphotericin B, melarsoprol, eflornithine, metronidazole, tinidazole, or miltefosine.

The antibody may include an amino acid motif selected from Ab-HC-(G)zCVIM, Ab-HC-(G)zCVLL, Ab-LC-(G)zCVIM, or Ab-LC-(G)zCVLL, where Ab denotes antibody, - HC- denotes heavy chain, -LC- denotes light chain, G denotes glycine, C denotes cysteine, V denotes valine, I denotes isoleucine, M denotes methionine, L denotes leucine, and z denotes an integer from 0 to 20.

The term "acyl" is known in the art and denotes a group represented by the general formula hydrocarbyl C(O)-, preferably alkyl C(O)-.

The term "acylamino" is known in the art and refers to an amino group substituted with an acyl group, and may be represented, for example, by the general formula hydrocarbyl C(O)NH-.

The term "acyloxy" is known in the art, refers to a group represented by the general formula hydrocarbyl C(O)O-, and is preferably alkyl C(O)O-.

The term "alkoxy" refers to an alkyl group, preferably a lower alkyl group to which oxygen is attached. Representative alkoxy groups include methoxy, ethoxy, propoxy, tert-butoxy, and the like.

The term "alkoxyalkyl" refers to an alkyl group substituted with an alkoxy group, and may be represented by the general formula alkyl-O-alkyl.

The term "alkenyl" as used herein refers to one or more aliphatic groups and is intended to include both "unsubstituted alkenyl" and "substituted alkenyl", and the latter refers to alkenyl moieties having a substituent replacing hydrogen atoms on one or more carbon atoms of the alkenyl group. Such substituents may be present on one or more carbon atoms that are or are not involved in one or more double bonds. Moreover, the substituents include all contemplated for alkyl groups as discussed below, except where stability is prohibited. For example, alkenyl groups substituted with one or more alkyl, carbocyclyl, aryl, heterocyclyl or heteroaryl groups are contemplated.

An "alkyl" group or "alkane" is a fully saturated straight-chain or branched non-aromatic hydrocarbon. Typically, straight-chain or branched alkyl groups have from 1 to 20 carbon atoms, preferably from 1 to 10 carbon atoms, unless otherwise defined. Examples of straight-chain and branched alkyl groups include methyl, ethyl, n-propyl, iso-propyl, n-butyl, sec-butyl, tert-butyl, pentyl, hexyl, heptyl and octyl. C₁-C₆ straight-chain or branched alkyl groups are also referred to as "lower alkyl" groups.

The term "alkyl" (or "lower alkyl") as used throughout the specification, Examples and claims is intended to include both "unsubstituted alkyl" and "substituted alkyl", and the latter refers to alkyl moieties having substituents in which hydrogen atoms on one or more carbon atoms of a hydrocarbon backbone are replaced. Such substituents, unless otherwise stated, are halogen, hydroxyl, carbonyl (for example, carboxyl, alkoxycarbonyl, formyl, or acyl), thiocarbonyl (for example, thioester, thioacetate, or thioformate), alkoxyl, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, or aromatic or heteroaromatic moieties. It will be understood by those skilled in the art that, where appropriate, a substituted moiety on a hydrocarbon chain may itself be substituted. For example, substituents of substituted alkyl may include substituted and unsubstituted forms of ether, alkylthio, carbonyl (including ketones, aldehydes, carboxylates, and esters), -CF₃, -CN and the like as well as amino, azido, imino, amido, phosphoryl (including phosphonates and phosphinates), sulfonyl (including sulfates, sulfonamido, sulfamoyl, and sulfonates), and silyl groups. Exemplary substituted alkyls are described below. Cycloalkyl may be further substituted with alkyl, alkenyl, alkoxy, alkylthio, aminoalkyl, carbonyl-substituted alkyl, -CF₃, -CN and the like.

For example, the term "C_{x-y}" when used in conjunction with a chemical moiety such as acyl, acyloxy, alkyl, alkenyl, alkynyl, or alkoxy is meant to include a group having x to y carbon atoms in the chain. For example, the term "C_{x-y} alkyl" refers to a substituted or unsubstituted saturated hydrocarbon group including straight-chain and branched chain alkyl groups having x to y carbon atoms in the chain, including haloalkyl groups such as trifluoromethyl and 2,2,2-trifluoroethyl. C₀ alkyl denotes hydrogen at the terminal position of the group, and a bond if internal. The terms "C2-alkenyl" and "C2-alkynyl" refer to substituted or unsubstituted unsaturated aliphatic groups of similar length and capable of substituting for the alkyls described above, but each containing at least one or more double or triple bonds.

As used herein, the term "alkylamino" refers to an amino group substituted with at least one or more alkyl groups.

As used herein, the term "alkylthio" refers to a thiol group substituted with an alkyl group, and may be represented by the general formula alkyl S-.

As used herein, the term "alkynyl" refers to an aliphatic group containing one or more triple bonds and is intended to include both "unsubstituted alkynyl" and "substituted alkynyl", and the latter refers to an alkynyl moiety having a substituent in which hydrogen atoms on one or more carbon atoms of the alkynyl group are replaced. Such substituents may be present on one or more carbon atoms that are or are not involved in one or more triple bonds. Such substituents include all contemplated for alkyl groups, as noted above, except where stability is prohibited. For example, substitution of an alkynyl group with one or more alkyl, carbocyclyl, aryl, heterocyclyl, or heteroaryl groups is contemplated.

The terms "amine" and "amino" are known in the art, refer to both unsubstituted and substituted amines and salts thereof, and represented by, for example, 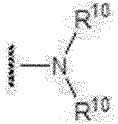 or 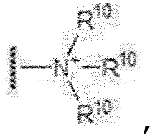 where each R¹⁰ independently denotes hydrogen or a hydrocarbyl group, or two R¹⁰ together with a N atom to which the two R¹⁰ are attached complete a heterocycle having 4 to 8 atoms in the ring structure.

As used herein, the term "aminoalkyl" refers to an alkyl group substituted with an amino group.

As used herein, the term "carboxy" refers to a group represented by the chemical formula -CO₂H.

As used herein, the terms "hetaralkyl" and "heteroaralkyl" refer to an alkyl group substituted with a hetaryl group.

As used herein, the term "heteroalkyl" refers to a saturated or unsaturated chain having carbon atoms and at least one or more heteroatoms, wherein the two heteroatoms are not adjacent.

The terms "heteroaryl" and "hetaryl" include a substituted or unsubstituted aromatic monocyclic structure, preferably a 5- to 7-membered ring, more preferably a 5- to 6-membered ring, and the ring structure thereof contains at least one heteroatom, preferably 1 to 4 heteroatoms, more preferably 1 or 2 heteroatoms. The terms "heteroaryl" and "hetaryl" also include polycyclic ring systems having two or more cyclic rings, wherein two or more carbon atoms are common to two adjacent rings. Here, at least one or more of the rings is heteroaromatic, and for example, the other cyclic rings are cycloalkyl, cycloalkenyl, cycloalkynyl, aryl, heteroaryl, and/or heterocyclyl. Heteroaryl groups include, for example, pyrrole, furan, thiophene, imidazole, oxazole, thiazole, pyrazole, pyridine, pyrazine, pyridazine, and pyrimidine.

As used herein, the term "heteroatom" refers to an atom of an arbitrary element other than carbon or hydrogen. Preferred heteroatoms are nitrogen, oxygen and sulfur.

The terms "heterocyclyl", "heterocycle" and "heterocyclic" refer to substituted or unsubstituted non-aromatic ring structures, preferably 3- to 10-membered rings, more preferably 3- to 7-membered rings, and the ring structures thereof contain at least one heteroatom, preferably 1 to 4 heteroatoms, more preferably 1 or 2 heteroatoms. The terms "heterocyclyl" and "heterocyclic" also include polycyclic ring systems having two or more cyclic rings, wherein two or more carbon atoms are common to two adjacent rings. Here, at least one or more rings are heterocyclic, and for example, the other cyclic rings are cycloalkyl, cycloalkenyl, cycloalkynyl, aryl, heteroaryl, and/or heterocyclyl. Heterocyclyl groups include, for example, piperidine, piperazine, pyrrolidine, morpholine, lactone, and lactam. The heterocyclyl group may also be substituted with an oxo group. For example, "heterocyclyl" includes both pyrrolidine and pyrrolidinone.

As used herein, the term "hydroxyalkyl" refers to an alkyl group substituted with a hydroxy group.

The term "substituted" refers to a moiety having a substituent replacing hydrogen atoms on one or more carbon atoms of the backbone. It will be understood that "substitution" or "substituted with" includes the implied conditions under which such substitution conforms to the acceptable valences of the substituted atom and substituent and the substitution affords a stable compound that does not spontaneously undergo changes, for example, rearrangement, cyclization, and elimination. As used herein, the term "substituted" is intended to include all permissible substituents of organic compounds. In a broad aspect, permissible substituents include acyclic and cyclic, branched and unbranched, carbocyclic and heterocyclic, aromatic and non-aromatic substituents of organic compounds. The permissible substituents may be one or more, the same or different for suitable organic compounds. For the present invention, heteroatoms such as nitrogen may have hydrogen substituents and/or any permissible substituents of the organic compounds described herein, which satisfy the valences of the heteroatoms. Substituents may include any of the substituents described herein, for example, halogen, hydroxyl, carbonyl (example: carboxyl, alkoxycarbonyl, formyl, or acyl), thiocarbonyl (example: thioester, thioacetate, or thioformate), alkoxyl, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, or aromatic or heteroaromatic moieties. Those skilled in the art will understand that a substituent may itself be substituted, if necessary. It is understood that reference to a chemical moiety herein includes substituted modifications, unless specifically stated as "unsubstituted". For example, reference to an "aryl" group or moiety includes implicitly both substituted and unsubstituted modifications.

As used herein, the term "thioalkyl" refers to an alkyl group substituted with a thiol group.

As used herein, the term "thioester" refers to - C(O)SR¹⁰ or -SC(O)R¹⁰, where R¹⁰ denotes hydrocarbyl.

As used herein, the term "thioether" is equivalent to an ether, wherein the oxygen is replaced with sulfur.

A "protecting group" refers to a group of atoms that, upon binding to a reactive functional group in a molecule, masks, diminishes or prevents the reactivity of the functional group. Usually, protecting groups may be selectively removed during synthesis, if necessary. Examples of protecting groups may be found in literatures [see: Greene and Wuts, Protective Groups in Organic Chemistry, 3rd Ed., 1999, John Wiley & Sons, NY and Harrison et al., Compendium of Synthetic Organic Methods, Vols. 1-8, 1971-1996, John Wiley & Sons, NY]. Representative nitrogen protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("TES"), trityl and substituted trityl groups, allyloxycarbonyl, 9-fluorenylmethyloxycarbonyl ("FMOC"), nitro-veratryloxycarbonyl ("NVOC"), and the like. Representative hydroxyl protecting groups include, but are not limited to, acylated (esterified) or alkylated hydroxyl groups such as benzyl and trityl ethers, as well as alkyl ethers, tetrahydropyranyl ethers, trialkylsilyl ethers (example: TMS or TIPS groups), glycol ethers such as ethylene glycol and propylene glycol derivatives, and allyl ethers.

"Connected by a covalent bond/covalently bound" includes both direct and indirect bonding of two chemical species (for example, via an intervening series of atoms). For example, an amino acid may be directly covalently bound to polyethylene glycol. For example, an ester may be formed between the carboxyl of an amino acid and the hydroxyl of a polyethylene glycol, or indirectly, for example, by forming epoxypropyl ether through reaction of polyethylene glycol and epichlorohydrin and reacting the produced epoxide with an amino group of an amino acid covalently bind the amino acid and polyethylene glycol through a 2-hydroxypropyl linker. Various moieties and reactions that directly or indirectly connect the various moieties are well known in the art. In certain preferred embodiments, unless the context dictates otherwise, an indirect bond contains only 1 to 10 intervening atoms (for example, methylene, dibutyl ether, and tripeptide), most preferably 1 to 6 intervening atoms.

As used herein, a therapeutic agent that "prevents" a disorder or condition refers to diminishing the incidence of a disorder or condition in a treated sample as compared to an untreated control sample or diminishing the severity or delaying the onset of one or more symptoms of a disorder or condition as compared to an untreated control sample in statistical samples.

The term "treating" includes prophylactic and/or therapeutic treatments. The term "prophylactic or therapeutic" treatments are known in the art and include administration of one or more of the compositions of the present invention to a host. Treatment is prophylactic (that is, protects the host from developing into an unwanted condition) when administered before clinical symptoms of an unwanted condition (for example, disease or other unwanted condition in the host animal) appear, whereas treatment is therapeutic (that is, intended to diminish, alleviate or stabilize an existing unwanted condition or side effects thereof) when administered after symptoms of the unwanted condition appear.

The term "prodrug" is intended to contain compounds that are converted into a therapeutically active agent of the present invention under physiological conditions. A common method of preparing prodrugs is to contain one or more selected moieties that are hydrolyzed under physiological conditions to reveal the target molecule. In other embodiments, the prodrug is converted by the enzymatic activity of the host animal. For example, esters or carbonates (for example, esters or carbonates of alcohols or carboxylic acids) are preferred prodrugs.

Hereinafter, the configuration of the present invention will be described in detail through Examples, but the following Examples are only for helping the understanding of the present invention and do not limit the scope of the present invention.

### [Examples]

In an aspect of the present invention, the linker-drug compound and the linker-drug-ligand conjugate according to the present invention may be synthesized according to the following procedure.

### [Synthetic pathway of linker-drug]

### 1) Linker synthesis

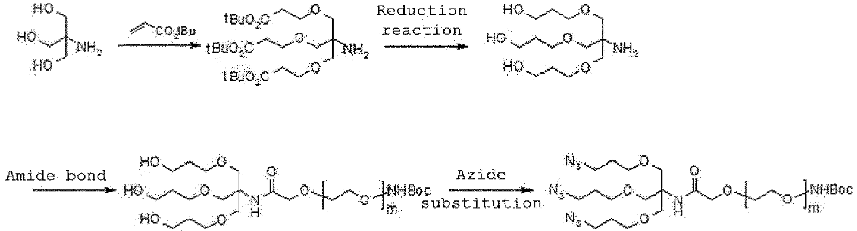

### 2) Linker-Drug Synthesis

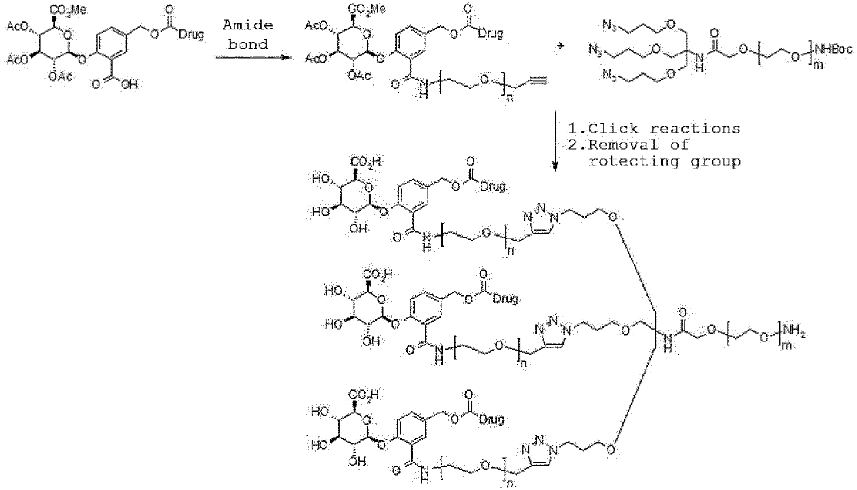

### [Synthetic pathway of linker-drug-ligand conjugate]

Linker-drug-ligand conjugates according to the present invention may be prepared using the techniques provided herein and the knowledge of those skilled in the art.

Linkers, for example, are described in PCT/US2016/063564 and PCT/US2016/063595, which are incorporated herein by reference in their entirety and, if not described therein, are cited herein or can be prepared according to known references by those skilled in the art.

### <Example 1> Preparation of Compound 5

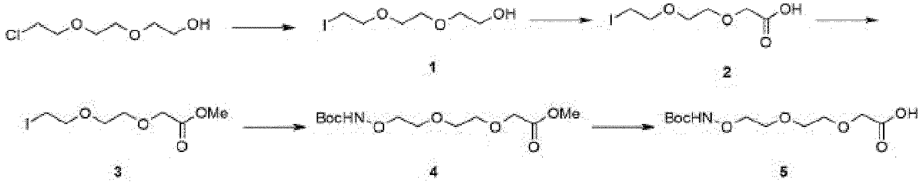

### Preparation of Compound 1

Under a nitrogen atmosphere, 2-(2-(2-chloroethoxy)ethoxy)ethanol (10.0 g, 59.3 mmol) was dissolved in acetone (60 mL), then sodium iodide (26.0 g, 177.9 mmol) was added, and the mixture was refluxed for 12 hours. After completion of the reaction, the reaction mixture was concentrated under reduced pressure and subjected to column chromatography to afford Compound 1 (13.0 g, 85%). ¹H-NMR (400 MHz, CDCl₃) δ 3.79-3.73 (m, 4H), 3.70-3.68 (m, 4H), 3.64-3.62 (m, 2H), 3.29-3.25 (m, 2H) .

### Preparation of Compound 2

Compound 1 (13.0 g, 49.9 mmol) was dissolved in acetone (316 mL) at 0°C under a nitrogen atmosphere, and then Jones reagent (31.6 mL) was added, followed by stirring at room temperature for 15 hours. After completion of the reaction, ethyl acetate (2 × 100 mL) and distilled water (150 mL) were added, and the extracted organic layer was dried over anhydrous sodium sulfate, filtered, and concentrated under reduced pressure. Purification by column chromatography was performed to afford Compound 2 (13.1 g, 96%). ¹H-NMR (400 MHz, CDCl₃) δ 4.22 (s, 2H), 3.80-3.66 (m, 6H), 3.29-3.25 (m, 2H).

### Preparation of Compound 3

Compound 2 (13.1 g, 47.9 mmol) was dissolved in methanol (121 mL) at 0°C under a nitrogen atmosphere, and oxalyl chloride (6.1 mL, 71.9 mmol) was added, followed by stirring at room temperature for 16 hours. After completion of the reaction, the reaction mixture was concentrated under reduced pressure and purified by column chromatography to afford Compound 3 (9.8 g, 71%). ¹H-NMR (400 MHz, CDCl₃) δ 4.19 (s, 2H), 3.78-3.70 (m, 9H), 3.27 (t, *J =* 7.0 Hz, 2H).

### Preparation of Compound 4

Compound 3 (9.75 g, 33.8 mmol) was dissolved in *N,N-*dimethylformamide (143 mL), and then N,N-Di-Boc-hydroxylamine (10.3 g, 44.0 mmol) and sodium hydride (60 % in oil, 1.63 g, 40.6 mmol) were added at 0°C under a nitrogen atmosphere. The reaction mixture was stirred for 15 hours, and distilled water (200 mL) and ethyl acetate (3 × 150 mL) were added, followed by extraction. The obtained organic layer was dried over anhydrous sodium sulfate, filtered, concentrated under reduced pressure, and purified by column chromatography to afford Compound 4 (10.6 g, 80%). ¹H-NMR (400 MHz, CDCl₃) δ 4.17 (s, 2H), 4.09 (t, *J =* 7.0 Hz, 2H), 3.76-3.73 (m, 9H), 1.54 (s, 18H).

### Preparation of Compound 5

Compound 4 (10.6 g, 26.9 mmol) was dissolved in tetrahydrofuran/methanol/distilled water (240 mL/80 mL/80 mL), and sodium hydroxide (2.7 g, 40.4 mmol) was added at 0°C under a nitrogen atmosphere. The reaction temperature was raised to room temperature, and then the mixture was stirred for 3 hours. The pH of the reaction mixture was adjusted to 4 with 1 N aqueous hydrochloric acid solution, and then distilled water (100 mL) and ethyl acetate (2 × 100 mL) were added, followed by extraction. The collected organic layers were dried over anhydrous sodium sulfate, filtered, and concentrated under reduced pressure to afford Compound 5 (6.76 g, 90%), which was used in the next reaction without further purification. ¹H-NMR (400 MHz, CDCl₃) δ 4.15 (s, 2H), 4.05-4.03 (m, 2H), 3.77-3.69 (m, 6H), 1.48 (s, 9H).

### <Example 2> Preparation of Compound 8

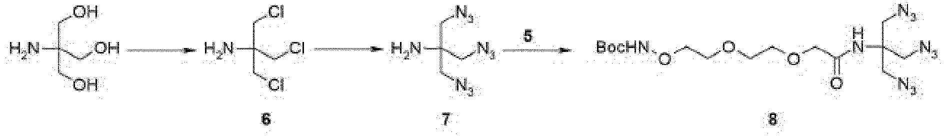

### Preparation of Compound 6

Added were 2-amino-2-(hydroxymethyl)-1,3-propanediol (Tris, 5 g, 41.3 mmol) and thionyl chloride (30.0 mL, 413 mmol) at 0°C under a nitrogen atmosphere, and the mixture was stirred to a suspension state. Pyridine (4.9 mL, 20.6 mmol) was gradually added to the mixture of suspension at -30°C, and the temperature was raised to 120°C when gas started to be generated during the reaction, followed by stirring for 2 hours. The reaction mixture was cooled to 0°C, distilled water (5 mL) was gradually added for 10 minutes, and then, a solution of sulfuric acid (3 mL, 0.04 mmol) diluted with distilled water (5 mL) was added, followed by stirring. Dichloromethane (2 × 300 mL) and 30% aqueous sodium hydroxide solution (100 mL) were added, followed by extraction. The collected organic layers were dried over anhydrous sodium sulfate, filtered, and concentrated under reduced pressure. Diethyl ether (100 mL) and hydrochloric acid (2 M in Et₂O, 23 mL) were added, and the solid thus formed was filtered to afford Compound 6 (4.6 g, 63%). ¹H-NMR (400 MHz, DMSO-d₆) δ 9.06 (s, 2H), 4.00 (s, 6H).

### Preparation of Compound 7

Compound 6 (2.6 g, 14.74 mmol) and sodium azide (4.8 g, 73.5 mmol) were dissolved in distilled water (63 mL), and the solution was stirred at 100°C for 15 hours. The reaction mixture was concentrated under reduced pressure, diluted with distilled water (60 mL), and extracted using diethyl ether (6 × 100 mL). The collected organic layers were dried over anhydrous sodium sulfate, filtered, and concentrated to afford Compound 7 (1.3 g, 45%), which was used in the next reaction without further purification. ¹H-NMR (400 MHz, CDCl₃) δ 3.34 (s, 6H) . ¹H-NMR (400 MHz, DMSO-d₆) 9.06 (s, 2H), 4.00 (s, 6H) .

### Preparation of Compound 8

Compound 5 (1 g, 3.58 mmol) and Compound 7 (773 mg, 3.94 mmol) were dissolved in N,N-dimethylformamide (15 mL), and N,N-diisopropylethylamine (1.3 mL, 7.16 mmol) and *N*,*N*,*N*',*N*'-tetramethyl-*O*-(1*H*-benzotriazol-1-yl)uronium hexafluorophosphate (1.5 g, 3.93 mmol) were added under a nitrogen atmosphere. The reaction mixture was stirred at room temperature for 14 hours, concentrated under reduced pressure, and purified by column chromatography to afford Compound 8 (600 mg, 37%) . ¹H-NMR (400 MHz, CDCl₃) δ 7.39 (s, 1H), 6.87 (s, 1H), 4.07 (t, *J* = 4.6 Hz, 2H), 3.96 (s, 2H), 3.72 (s, 6H), 3.70-3.77 (m, 6H), 1.48 (s, 9H).

### <Example 3> Preparation of Compound 14

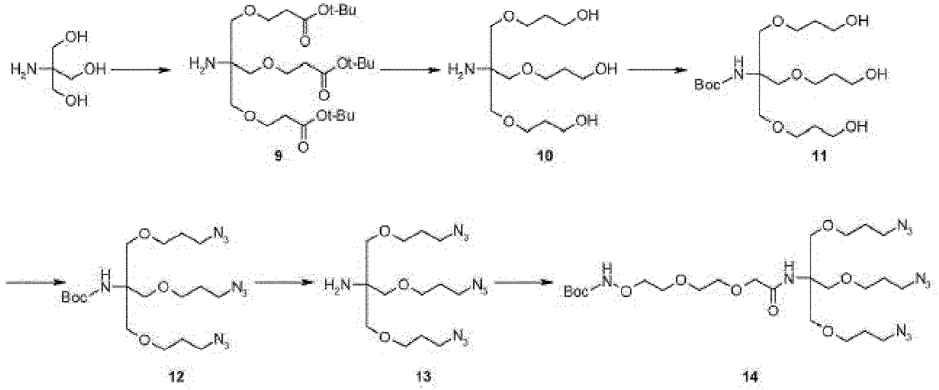

### Preparation of Compound 9

In dimethyl sulfoxide (8 mL), 2-amino-2-(hydroxymethyl)-1,3-propanediol (Tris, 5.0 g, 41.3 mmol) was dissolved, and then 5 N aqueous sodium hydroxide solution (0.8 mL) was added under a nitrogen atmosphere, followed by gradual addition of t-butyl acrylate (21.0 mL, 140 mmol). The reaction mixture was stirred at room temperature for 16 hours, and distilled water (100 mL) was added to the reaction mixture, followed by extraction with ethyl acetate (2 × 100 mL). The collected organic layers were dried over anhydrous sodium sulfate, filtered, concentrated, and purified by column chromatography to afford Compound 9 (9.05 g, 44%). ¹H-NMR (400 MHz, CDCl₃) δ 3.64 (t, *J* = 6.2 Hz, 6H), 3.31 (s, 6H), 2.45 (t, *J* = 6.2 Hz, 6H), 1.45 (s, 27H).

### Preparation of Compound 10

Compound 9 (9.05 g, 17.9 mmol) was dissolved in tetrahydrofuran (170 mL), and lithium aluminum hydride (1 M in THF, 54 mL) was added at 0°C under a nitrogen atmosphere. The reaction mixture was stirred at room temperature for 4 hours, and distilled water (2 mL), 15% w/w aqueous sodium hydroxide solution (2 mL), and distilled water (6 mL) were sequentially added. Drying over anhydrous sodium sulfate, filtration and concentration were performed to afford Compound 10 (4.3 g, 82%), which was used in the next reaction without further purification. ¹H-NMR (400 MHz, CDCl₃) δ 3.75 (t, *J* = 5.4 Hz, 6H), 6.22 (t, *J =* 5.4 Hz, 6H), 3.36 (s, 6H), 1.83-1.78 (m, 6H).

### Preparation of Compound 11

Compound 10 (4.3 g, 14.6 mmol) was dissolved in methanol (50 mL), and then a solution of di-t-butyl dicarbonate (3.3 g, 15.1 mmol) in methanol (25 mL) was gradually added at 0°C under a nitrogen atmosphere. The reaction mixture was stirred at room temperature for 12 hours, concentrated under reduced pressure, and purified by column chromatography to afford Compound 11 (4.05 g, 70%). ¹H-NMR (400 MHz, CDCl₃) δ 3.73 (t, *J* = 5.2 Hz, 6H), 3.67 (s, 6H), 3.61 (t, *J* = 5.2 Hz, 6H), 1.81-1.78 (m, 6H).

### Preparation of Compound 12

Compound 11 (1 g, 2.52 mmol) was dissolved in tetrahydrofuran (8 mL), and then 4-methylmorpholine (1.2 mL, 11.5 mmol) and methanesulfonic anhydride (2 g, 11.5 mmol) were added at 0°C under a nitrogen atmosphere. The reaction mixture was stirred for 2 hours, then concentrated under reduced pressure, and dissolved in N,N-dimethylformamide (9 mL), and sodium azide (850 mg, 12.8 mmol) was added. The reaction mixture was stirred at 100°C for 2 hours and then cooled to room temperature, and saturated aqueous ammonium chloride solution (8 mL) was added, followed by extraction with ethyl acetate (2 × 30 mL). The collected organic layers were washed with brine (2 × 30 mL) and dried over anhydrous sodium sulfate. Filtration, concentration and purification by column chromatography were performed to afford Compound 12 (984 mg, 82%). ¹H-NMR (400 MHz, CDCl₃) δ 3.64 (s, 6H), 3.51 (t, *J* = 5.8 Hz, 6H), 3.36 (t, *J =* 6.6 Hz, 6H), 1.86-1.80 (m, 6H), 1.43 (s, 9H).

### Preparation of Compound 13

Compound 12 (167 mg, 0.34 mmol) was dissolved in dichloromethane (4 mL), and then hydrochloric acid (4 N in 1,4-dioxane, 1 mL, 10 mmol) was added at 0°C under a nitrogen atmosphere. The reaction mixture was stirred at room temperature for 12 hours and then concentrated under reduced pressure to afford Compound 13 (131 mg, 99%). ¹H-NMR (400 MHz, CDCl₃) δ 8.37 (s, 2H), 3.70 (s, 6H), 3.60 (t, *J* = 5.4 Hz, 6H), 3.47 (t, *J* = 6.6 Hz, 6H), 1.90-1.87 (m, 6H) .

### Preparation of Compound 14

Compound 13 (148 mg, 0.4 mmol) and Compound 5 (313 mg, 1.12 mmol) were dissolved in N,N-dimethylformamide (1.6 mL), and then N,N-diisopropylethylamine (0.15 mL, 1.68 mmol), *N*,*N*,*N*',*N*'-tetramethyl-*O*-(1*H*-benzotriazol-1-yl)uronium hexafluorophosphate (410 mg, 1.08 mmol) were sequentially added at 0°C under a nitrogen atmosphere. The reaction mixture was stirred at room temperature for 14 hours, concentrated under reduced pressure, and purified by column chromatography to afford Compound 14 (130 mg, 50%). ¹H-NMR (400 MHz, CDCl₃) δ 7.51 (s, 1H), 6.79 (s, 1H), 4.03 (t, *J* = 4.4 Hz, 2H), 3.92 (s, 2H), 3.72 (s, 6H), 3.70-6.79 (m, 6H), 3.52 (t, *J* = 5.6 Hz, 6H), 3.65 (t, *J* = 6.6 Hz, 6H), 1.86-1.80 (m, 6H), 1.48 (s, 9H).

### <Example 4> Preparation of Compound 18

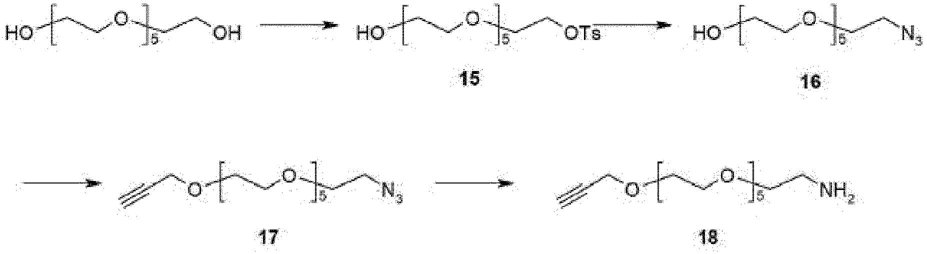

### Preparation of Compound 15

Hexaethylene glycol (50.0 g, 177 mmol), silver oxide (61.6 g, 266 mmol), and potassium iodide (5.85 g, 35.4 mmol) were diluted with dichloromethane (500 mL) and sonicated for 15 minutes. To this solution, a solution of 4-toluenesulfonyl chloride (34.4 g, 181 mmol) in dichloromethane (100 mL) was gradually added at -30°C. The temperature was gradually raised to 0°C, and the reaction mixture was maintained at this temperature for 15 minutes and dried over anhydrous sodium sulfate. Filtration, concentration and purification by column chromatography were performed to afford Compound 15 (61.46 g, 80%). ¹H-NMR (400 MHz, CDCl₃) δ 7.80 (d, *J* = 8.0 Hz, 2H), 7.35 (d, *J* = 8.0 Hz, 2H), 4.16 (m, 2H), 3.71-3.58 (m, 22H), 2.45 (s, 3H).

### Preparation of Compound 16

Compound 15 (5 g, 11.5 mmol) and sodium azide (1.2 g, 17.2 mmol) were dissolved in N,N-dimethylformamide (30 mL), followed by stirring at 100°C for 15 hours. Filtration, concentration and purification by column chromatography were performed to afford Compound 16 (3.16 g, 90%). ¹H-NMR (400 MHz, CDCl₃) δ 3.72-3.66 (m, 20H), 3.62-3.60 (m, 2H), 3.39 (t, *J* = 5.0 Hz, 2H), 2.88 (br s, 1H) .

### Preparation of Compound 17

Compound 16 (3.16 g, 10.3 mmol) was dissolved in N,N-dimethylformamide (21 mL), and sodium hydride (55 % in oil, 494 mg, 12.3 mmol) was added at 0°C under a nitrogen atmosphere. After 30 minutes, propargyl bromide (1.2 mL, 13.4 mmol) was added at 0°C under a nitrogen atmosphere. The reaction mixture was stirred at room temperature for 1 hour, and then distilled water (20 mL) was added to the reaction mixture, followed by extraction with ethyl acetate (2 × 20 mL). The collected organic layers were washed with a saturated aqueous ammonium chloride solution (10 mL) and brine (20 mL), and then dried over anhydrous sodium sulfate. Filtration, concentration and purification by column chromatography were performed to afford Compound 17 (3.05 g, 86%). ¹H-NMR (400 MHz, CDCl₃) δ 4.20 (s, 2H), 3.69-3.66 (m, 22H), 3.39 (t, *J* = 5.0 Hz, 2H), 2.43 (s, 1H).

### Preparation of Compound 18

Compound 17 (3.05 g, 8.83 mmol) was dissolved in tetrahydrofuran (35 mL), and then triphenylphosphine (2.80 g, 10.6 mmol) was added. The reaction mixture was stirred at room temperature for 1 hour, and then distilled water (3.2 mL, 177 mmol) was added. The reaction mixture was heated to reflux and stirred for 18 hours. The reaction mixture was cooled to room temperature, concentrated, and purified by column chromatography to afford Compound 18 (2.60 g, 92%). ¹H-NMR (400 MHz, CDCl₃) δ 4.21 (s, 2H), 3.71-3.64 (m, 20H), 3.51 (t, *J* = 5.0 Hz, 2H), 2.87 (t, *J* = 5.0 Hz, 2H), 2.43 (s, 1H).

### <Example 5> Preparation of Compound 21

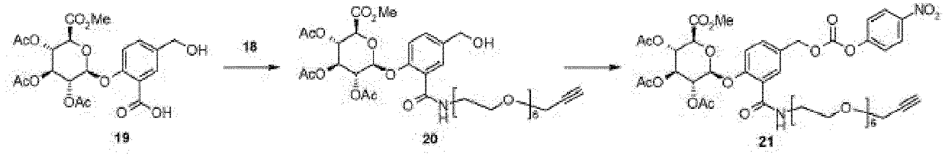

### Preparation of Compound 20

Compound 19 (2.82 g, 5.82 mmol, Compound 19 was prepared by the method described in patent WO 2017089895 A1) was dissolved in N,N-dimethylformamide (15 mL), and then Compound 18 (2.05 g, 6.4 mmol), *N,N-*diisopropylethylamine (2.1 mL 11.6 mmol) and *N,N,N',N'-*tetramethyl-*O*-(1*H*-benzotriazole-1-yl)uronium hexafluorophosphate (2.65 g, 6.98 mmol) were added at 0°C under a nitrogen atmosphere. The reaction mixture was stirred at 0°C for 30 minutes and then stirred at room temperature for 1 hour. Distilled water (50 mL) was added to the reaction mixture, followed by extraction with ethyl acetate (2 × 50 mL). The collected organic layers were dried over anhydrous sodium sulfate, filtered, concentrated under reduced pressure, and purified by column chromatography to afford Compound 20 (3.04 g, 67%). ¹H-NMR (400 MHz, CDCl₃) δ 7.46-7.42 (m, 2H), 7.05 (d, *J* = 8.8 Hz, 1H), 5.39-5.26 (m, 4H), 4.65 (s, 2H), 4.23-4.20 (m, 3H), 3.74 (s, 3H), 3.69-3.62 (m, 24H), 2.45 (s, 1H), 2.06 (s, 9H).

### Preparation of Compound 21

Compound 20 (3.04 g, 3.9 mmol) was dissolved in dichloromethane (40 mL), and then bis(4-nitrophenyl)carbonate (1.42 g, 4.64 mmol) and *N,N-*diisopropylethylamine (1.1 mL, 5.85 mmol) were added at 0°C under a nitrogen atmosphere. The reaction mixture was stirred at 0°C for 30 minutes and then stirred at room temperature for 2 hours. Distilled water (30 mL) was added to the reaction mixture, followed by extraction with dichloromethane (30 mL). The collected organic layers were washed with brine and dried over anhydrous sodium sulfate. Filtration, concentration under reduced pressure, and purification by column chromatography were performed to afford Compound 21 (3.1 g, 84%). ¹H-NMR (400 MHz, CDCl₃) δ 8.28 (d, *J* = 8.4 Hz, 2H), 8.14 (s, 1H), 7.54 (d, *J* = 8.0 Hz 2H), 7.38 (d, *J* = 8.0 Hz, 2H), 7.09 (d, *J* =8.4 Hz, 1H), 5.41-5.31 (m, 4H), 5.27(s, 2H), 4.24-4.22 (m, 3H), 3.74 (s, 3H). 3.69-3.64 (m, 24H), 2.44 (s, 1H), 2.06 (s, 9H).

### <Example 6> Preparation of Compound 27

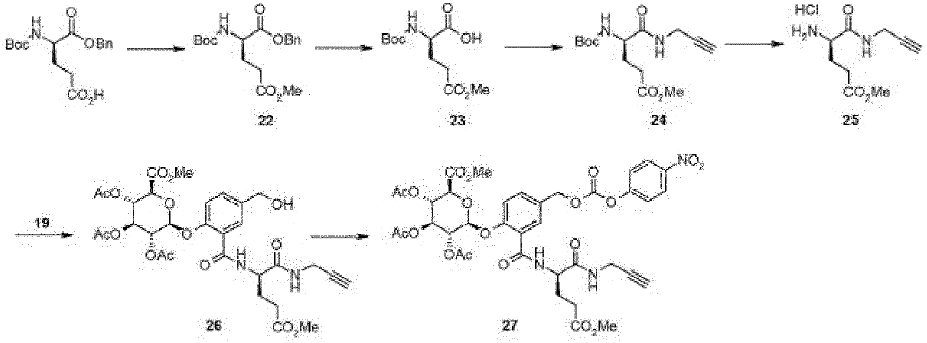

### Preparation of Compound 22

After 1-benzyl N-(t-butoxycarbonyl)-D-glutamate (3.0 g, 8.89 mmol) was dissolved in N,N-dimethylformamide (30 mL), N,N-diisopropylethylamine (1.86 mL, 10.67 mmol) and iodomethane (0.66 mL, 10.67 mmol) were added. The reaction mixture was stirred at room temperature for 2 hours, and distilled water (50 mL) was added, followed by extraction with ethyl acetate (50 mL). The collected organic layers were dried over anhydrous sodium sulfate, filtered, concentrated under reduced pressure, and purified by column chromatography to afford Compound 22 (2.05 g, 66%). ¹H-NMR (400 MHz, CDCl₃) δ 7.35 (s, 5H), 5.20 (s, 1H), 5.13-5.11 (m, 1H), 4.38-4.37 (m, 1H), 3.65 (s, 3H), 2.45-2.30 (m, 2H), 1.98-1.93 (m, 1H), 1.58 (s, 9H) .

### Preparation of Compound 23

Compound 22 (2.05 g, 5.83 mmol) and palladium/charcoal (10% w/w, 200 mg) were dissolved in methanol (30 mL), and then the reaction mixture was stirred at room temperature for 1 hour under a hydrogen atmosphere. The reaction mixture was filtered through celite and concentrated under reduced pressure to afford Compound 23 (1.5 g, 99 %). ¹H-NMR (400 MHz, CDCl₃) δ 5.20-5.18 (m, 1H), 4.34-4.24 (m, 1H), 3.73 (s, 1H), 2.52-2.42 (m, 2H), 2.62-2.23 (m, 1H), 2.04-2.00 (m, 1H), 1.48 (s, 9H).

### Preparation of Compound 24

Compound 23 (730 mg, 2.80 mmol) and propargyl amine (0.22 mL, 3.35 mmol) were dissolved in *N,N-*dimethylformamide (20 mL), and then *N,N-*diisopropylethylamine (0.97 mL, 5.58 mmol), 1-hydroxybenzotriazole (453 mg, 3.35 mmol), and N-(3-dimethylaminopropyl)-N'-ethylcarbodiimide hydrochloride (642 mg, 3.35 mmol) were added. The reaction mixture was stirred at room temperature for 14 hours, and distilled water (30 mL) was added, followed by extraction with ethyl acetate (3 × 30 mL). The collected organic layers were washed sequentially with 0.5 N aqueous hydrochloric acid solution (30 mL), saturated aqueous ammonium chloride solution (30 mL), and brine (30 mL), and then dried over anhydrous sodium sulfate. Filtration, concentration under reduced pressure, and purification by column chromatography were performed to afford Compound 24 (700 mg, 84%). ¹H-NMR (400 MHz, CDCl₃) δ 6.76 (s, 1H), 5.33 (s, 1H), 4.17 (s, 1H), 4.05-4.04 (m, 2H), 3.69 (s, 3H), 2.53-2.23 (m, 2H), 2.26-2.19 (m, 1H), 2.17-2.13 (m, 1H), 1.98-1.89 (m, 1H), 1.44 (s, 9H).

### Preparation of Compound 25

Compound 24 (700 mg, 2.35 mmol) was dissolved in dichloromethane (10 mL), and then hydrochloric acid (4 N in 1,4-dioxane, 5 mL) was added at 0°C under a nitrogen atmosphere. The reaction mixture was stirred at room temperature for 2 hours, and then concentrated under reduced pressure to afford Compound 25 (549 mg, 100%). ¹H-NMR (400 MHz, CDCl₃) δ 10.23 (s, 3H), 4.47-4.09 (m, 1H), 3.99-3.84 (m, 2H), 3.69 (s, 3H), 2.67-2.64 (m, 2H), 2.42-2.28 (m, 3H).

### Preparation of Compound 26

Compound 25 (653 mg, 2.79 mmol) and Compound 19 (1.23 g, 2.54 mmol) were dissolved in *N,N-*dimethylformamide (20 mL), and then *N,N-*diisopropylethylamine (1.33 mL, 7.62 mmol) and *N,N,N',N'-*tetramethyl-*O*-(1*H*-benzotriazol-1-yl)uronium hexafluorophosphate (1.16 g, 3.05 mmol) were added. The reaction mixture was stirred at room temperature for 14 hours, and distilled water (50 mL) was added, followed by extraction with ethyl acetate (3 × 50 mL). The collected organic layers were washed with saturated aqueous sodium hydrogen carbonate solution (30 mL) and brine (30 mL), and then dried over anhydrous sodium sulfate. Filtration, concentration under reduced pressure, and purification by column chromatography were performed to afford Compound 26 (1.36 g, 82%). ¹H-NMR (400 MHz, CDCl₃) δ 7.94 (s, 1H), 7.74 (d, *J* = 8.0 Hz, 1H), 7.52 (dd, *J* = 2.2, 10.4 Hz, 1H), 7.05 (d, *J* = 8.4 Hz, 1H), 6.85 (m, 1H), 5.43-5.33 (m, 4 H), 4.73-4.69 (m, 3H), 4.15-4.09 (m, 3H), 3.69 (d, *J =* 7.6 Hz, 6H), 2.56-2.51 (m, 2 H), 2.40-2.35 (m, 1H), 2.06 (t, *J* = 9.6 Hz), 1.89-1.86 (m, 1H).

### Preparation of Compound 27

Compound 26 (500 mg, 0.75 mmol) was dissolved in N,N-dimethylformamide (20 mL), and then bis(4-nitrophenyl)carbonate (229 mg, 0.75 mmol) and *N,N-*diisopropylethylamine (0.2 mL, 1.13 mmol) were added at 0°C under a nitrogen atmosphere. The reaction mixture was stirred at room temperature for 12 hours, distilled water (50 mL) was added to the reaction mixture, followed by extraction with ethyl acetate (2 × 50 mL), and the collected organic layers were dried over anhydrous sodium sulfate. Filtration, concentration, and purification by column chromatography were performed to afford Compound 27 (518 mg, 83%). ¹H-NMR (400 MHz, CDCl₃) δ 8.27 (d, *J* = 8.8 Hz, 2H), 8.09 (s, 1H), 7.78 (d, *J* = 8.8 Hz, 1H), 7.58 (dd, *J* = 2.2, 10.4 Hz, 1H), 7.38 (d, *J* = 9.2 Hz, 1H), 7.10 (d, *J* = 8.4 Hz, 1H), 6.80 (m, 1H), 5.45-5.39 (m, 4 H), 5.27 (s, 2H), 4.73-4.69 (m, 1H), 4.18-4.09 (m, 3H), 3.69 (d, J = 6.8 Hz, 6H), 2.53-2.51 (m, 2 H), 2.42-2.27 (m, 1H), 2.26-2.23 (m, 1H), 2.21-2.15 (m, 2H), 2.05 (t, *J* = 9.6 Hz).

### <Example 7> Preparation of Compound 29

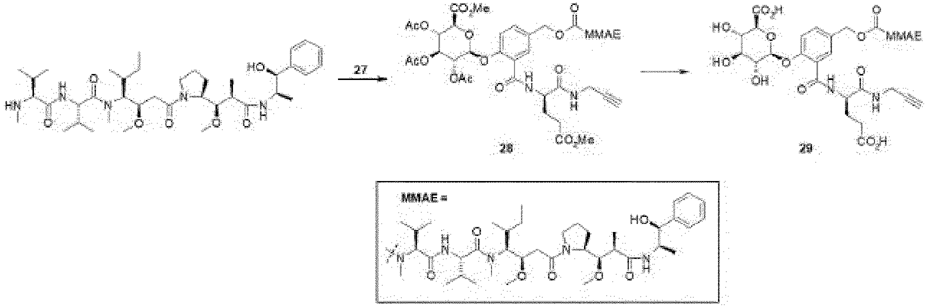

### Preparation of Compound 28

Compound 27 (198 mg, 0.24 mmol) was dissolved in N,N-dimethylformamide (1 mL), and then monomethyl auristatin E (150 mg, 0.22 mmol) was added at room temperature under a nitrogen atmosphere. To the reaction mixture were added 1-hydroxy-7-azabenzotriazole (6 mg, 0.043 mmol), pyridine (0.2 mL), and *N,N-*diisopropylethylamine (0.076 mL, 0.44 mmol). The reaction mixture was stirred at room temperature for 24 hours, then concentrated under reduced pressure, and purified by column chromatography to afford Compound 28 (258 mg, 76%). EI-MS m/z: 1/2[M+H]⁺ 1502.7, [M+H]⁺ 1409.2.

### Preparation of Compound 29

Compound 28 (258 mg, 0.18 mmol) was dissolved in tetrahydrofuran/methanol (2 mL/2 mL), and then a solution of lithium hydroxide (38 mg, 0.92 mmol) in distilled water (2 mL) was gradually added at -40°C. The mixture was stirred for 2 hours while gradually raising the reaction temperature to 0°C. The pH of the reaction mixture was adjusted to 4 to 5 with acetic acid, and then the reaction mixture was concentrated under reduced pressure. The residue was dissolved in acetonitrile/distilled water (1 mL/1 mL), purified by HPLC, and lyophilized to afford Compound 29 as a white solid (159 mg, 69%). EI-MS m/z: [M+H]⁺ 1255.0, [M+Na]⁺ 1277.0.

### <Example 8> Preparation of Compound 34

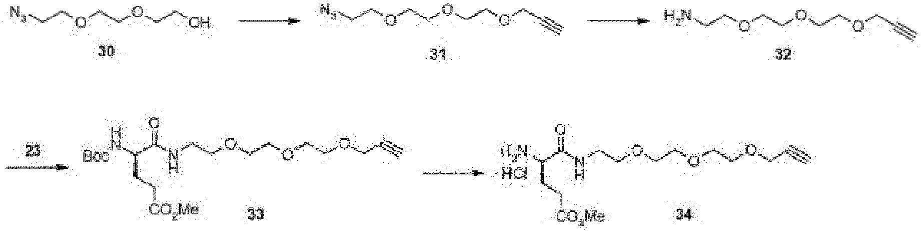

### Preparation of Compound 31

Compound 30 (4.5 g, 25.7 mmol, Compound 30 was prepared by the method described in patent WO 2017089895 A1) was dissolved in N,N-dimethylformamide (50 mL), and then propargyl bromide (80 % in toluene, 4.96 mL, 33.4 mmol) and sodium hydride (60 % in oil, 1.23 g, 30.8 mmol) were added at 0°C under a nitrogen atmosphere. The reaction mixture was stirred at room temperature for 3 hours, and distilled water (50 mL) was added, followed by extraction with ethyl acetate (50 mL). The collected organic layers were dried over anhydrous sodium sulfate, filtered, concentrated under reduced pressure, and then purified by column chromatography to afford Compound 31 (4.35 g, 79%). ¹H-NMR (400 MHz, CDCl₃) δ 4.21 (d, *J* = 2.4 Hz, 2H), 3.70-3.67 (m, 10H), 3.39 (d, *J* = 5.2 Hz, 2H), 2.43 (t, *J* = 2.4 Hz, 1H).

### Preparation of Compound 32

Compound 31 (2.7 g, 7.23 mmol) was dissolved in tetrahydrofuran/distilled water (30 mL/1.5 mL), and then triphenylphosphine (2.09 g, 7.97 mmol) was added. The reaction mixture was stirred at room temperature for 24 hours, concentrated under reduced pressure, and purified by column chromatography to afford Compound 32 (1.32 g, 99%). ¹H-NMR (400 MHz, CDCl₃) δ 4.21 (s, 2H), 3.69-3.64 (m, 8H), 3.53-3.50 (m, 2H), 2.88-2.85(m, 2H), 2.43 (s, 1H). EI-MS m/z: [M+H]⁺ 188.2, [M+Na]⁺ 210.3.

### Preparation of Compound 33

Compound 23 (888 mg, 3.40 mmol) and Compound 32 (700 mg, 3.74 mmol) were dissolved in N,N-dimethylformamide (30 mL), and then N,N-diisopropylethylamine (1.18 mL, 6.80 mmol), 1-hydroxybenzotriazole (551 mg, 4.08 mmol), and N-(3-dimethylaminopropyl)-N'-ethylcarbodiimide hydrochloride (782 mg, 4.08 mmol) were added at 0°C under a nitrogen atmosphere. The reaction mixture was stirred at room temperature for 14 hours, and distilled water (30 mL) was added, followed by extraction with ethyl acetate (3 × 30 mL). The collected organic layers were washed sequentially with 0.5 N aqueous hydrochloric acid solution (30 mL), saturated aqueous sodium hydrogen carbonate solution (30 mL), and brine (30 mL), and then dried over anhydrous sodium sulfate. Filtration, concentration, and purification by column chromatography were performed to afford Compound 33 (1.23 g, 84%). ¹H-NMR (400 MHz, CDCl₃) δ 4.22 (s, 2H), 4.21 (br, 1H), 3.72-3.58 (m, 10H), 3.57-3.55 (m, 2H), 3.48-3.44 (m, 2H), 2.48-2.39 (m, 3H), 2.17-2.11 (m, 2H), 1.96-1.88 (m, 2H), 1.43 (s, 9H).

### Preparation of Compound 34

Compound 33 (770 mg, 1.79 mmol) was dissolved in dichloromethane (10 mL), and then hydrochloric acid (4 N in 1,4-dioxane, 5 mL) was added at 0°C under a nitrogen atmosphere. The reaction mixture was stirred at room temperature for 1 hour and then concentrated under reduced pressure to afford Compound 34 (656 mg, 100%). EI-MS m/z: [M+H]⁺ 331.3, [M+Na]⁺ 353.3.

### <Example 9> Preparation of Compound 37

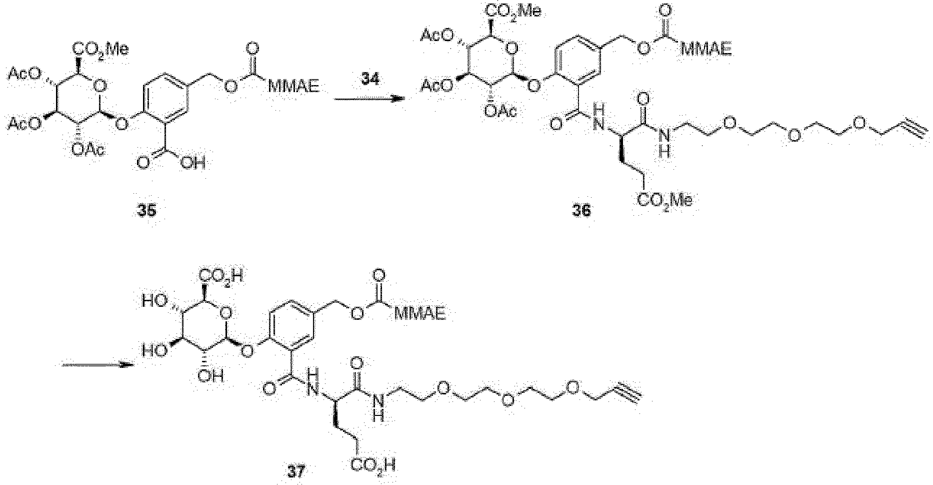

### Preparation of Compound 36

Compound 35 (150 mg, 0.122 mmol, Compound 35 was prepared by the method described in patent WO 2017089895 A1) and Compound 34 (47 mg, 0.128 mmol) were dissolved in N,N-dimethylformamide (2 mL), and then *N,N-*diisopropylethylamine (0.053 mL, 0.305 mmol) and *N*,*N*,*N*',*N*'-tetramethyl-*O*-(1*H*-benzotriazol-1-yl)uronium hexafluorophosphate (56 mg, 0.146 mmol) were added at 0°C under a nitrogen atmosphere. The reaction mixture was stirred at room temperature for 22 hours, concentrated under reduced pressure, and purified by column chromatography to afford Compound 36 (150 mg, 80%). EI-MS m/z: 1/2[M+H]⁺ 771.6, [M+H]⁺ 1541.4, [M+Na]⁺ 1563.4.

### Preparation of Compound 37

Compound 36 (150 mg, 0.097 mmol) was dissolved in tetrahydrofuran/methanol (2 mL/2 mL), and then a solution of lithium hydroxide (38 mg, 0.584 mmol) in distilled water (2 mL) was gradually added at -40°C. The mixture was stirred for 2 hours while gradually raising the reaction temperature to 0°C. The pH of the reaction mixture was adjusted to 4 to 5 with acetic acid, and then the reaction mixture was diluted with acetonitrile/distilled water (1 mL/1 mL), purified by HPLC, and lyophilized to afford Compound 37 as a white solid (100 mg, 74%). EI-MS m/z: 1/2[M+H]⁺ 694.4, [M+H]⁺ 1387.2, [M+Na]⁺ 1409.2.

### <Example 10> Preparation of Compound 39

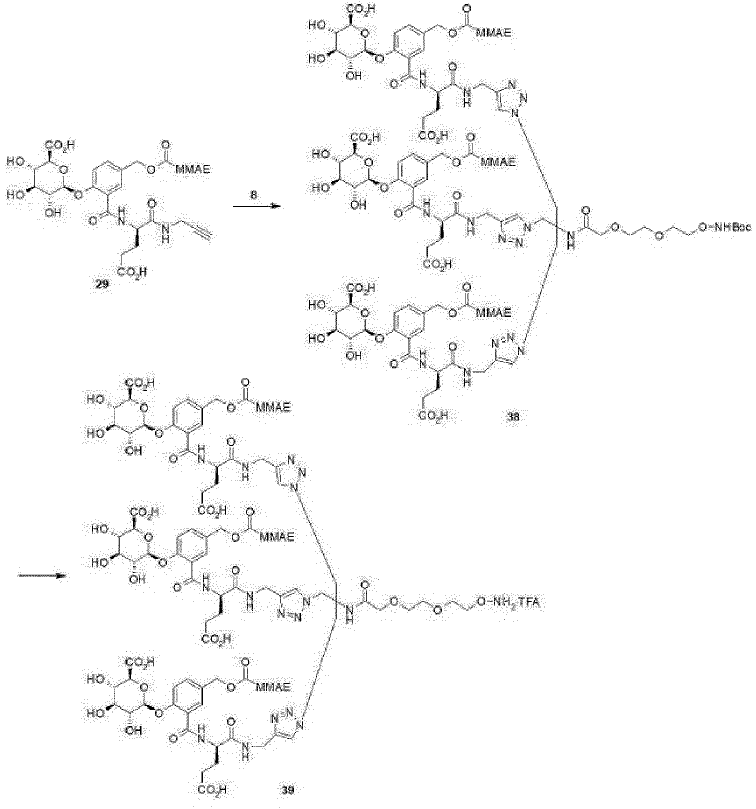

### Preparation of Compound 38

Compound 29 (61 mg, 0.05 mmol) and Compound 8 (5 mg, 0.014 mmol) were dissolved in ethanol/dichloromethane (1 mL) and distilled water (1 mL), and then copper sulfate (1.7 mg, 0.007 mmol) and sodium ascorbate (1.7 mg, 0.007 mmol) were added at 0°C under a nitrogen atmosphere. The reaction mixture was stirred at room temperature for 1 hour, then the reaction solvent was evaporated with nitrogen gas, the residue was diluted with dimethyl sulfoxide (1 mL), purified by HPLC, and lyophilized to afford Compound 38 (40.0 mg, 70%) as a white solid. EI-MS m/z: 1/2[M+H]⁺ 2111.9, 1/3[M+H]⁺ 1408.3, 1/4[M+H]⁺ 1056.5.

### Preparation of Compound 39

Compound 38 (28 mg, 6.0 µmol) was dissolved in dichloromethane (1 mL), trifluoroacetic acid (0.4 mL) was added at 0°C, and the mixture was stirred for 1 hour. The reaction mixture was concentrated under reduced pressure, purified by HPLC, and lyophilized to afford Compound 39 as a white solid (10 mg, 40%). EI-MS m/z : 1/2[M+H]⁺ 2061.3, 1/3[M+H]⁺ 1374.9, 1/4[M+H]⁺ 1031.4.

### <Example 11> Preparation of Compound 41

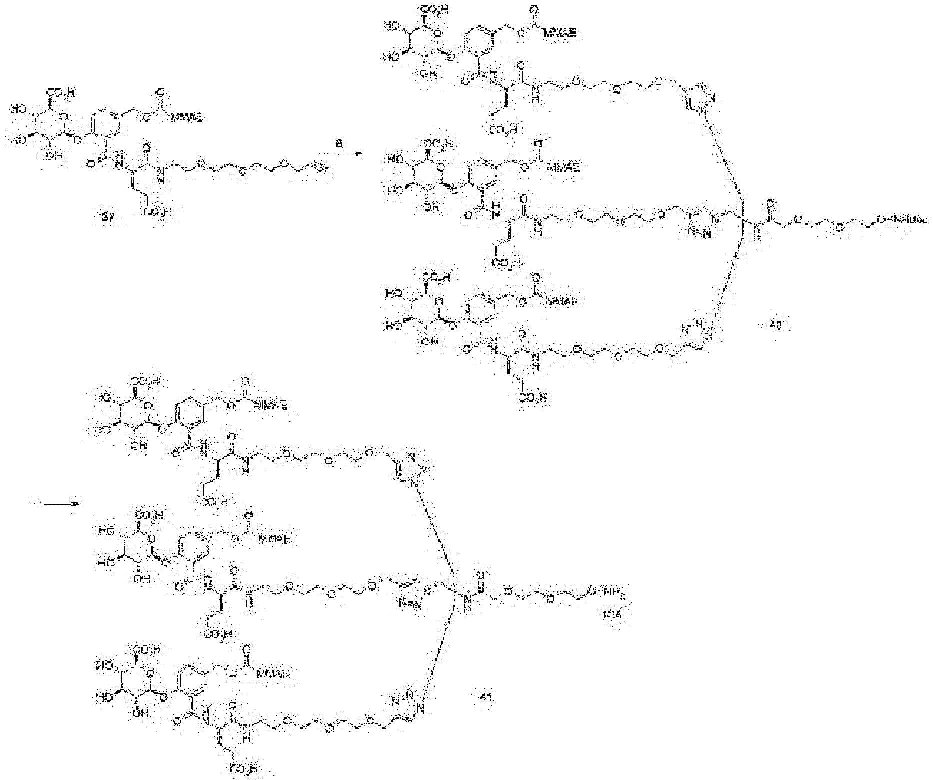

### Preparation of Compound 40

Compound 37 (68 mg, 0.049 mmol) and Compound 8 (5 mg, 0.014 mmol) were dissolved in ethanol/dichloromethane (1 mL) and distilled water (1 mL), and then copper sulfate (1.7 mg, 7 µmol) and sodium ascorbate (3.0 mg, 0.014 mmol) were added at 0°C under a nitrogen atmosphere. The reaction mixture was stirred at room temperature for 1 hour, then the reaction solvent was evaporated with nitrogen gas, the residue was diluted with dimethyl sulfoxide (1 mL), purified by HPLC, and lyophilized to afford Compound 40 as a white solid (28 mg, 37%). EI-MS m/z: 1/2[M+H]⁺ 2309.8, 1/3[M+H]⁺ 1540.4, 1/4[M+H]⁺ 1155.6.

### Preparation of Compound 41

Compound 40 (28 mg, 6.0 µmol) was dissolved in dichloromethane (1 mL), trifluoroacetic acid (0.4 mL) was added at 0°C, and the mixture was stirred for 3 hours. The reaction mixture was concentrated under reduced pressure, purified by HPLC, and lyophilized to afford Compound 41 as a white solid (5 mg, 20%). EI-MS m/z: 1/2[M+H]⁺ 2260.4, 1/3[M+H]⁺ 1507.6, 1/4[M+H]⁺ 1130.6.

### <Example 12> Preparation of Compound 47

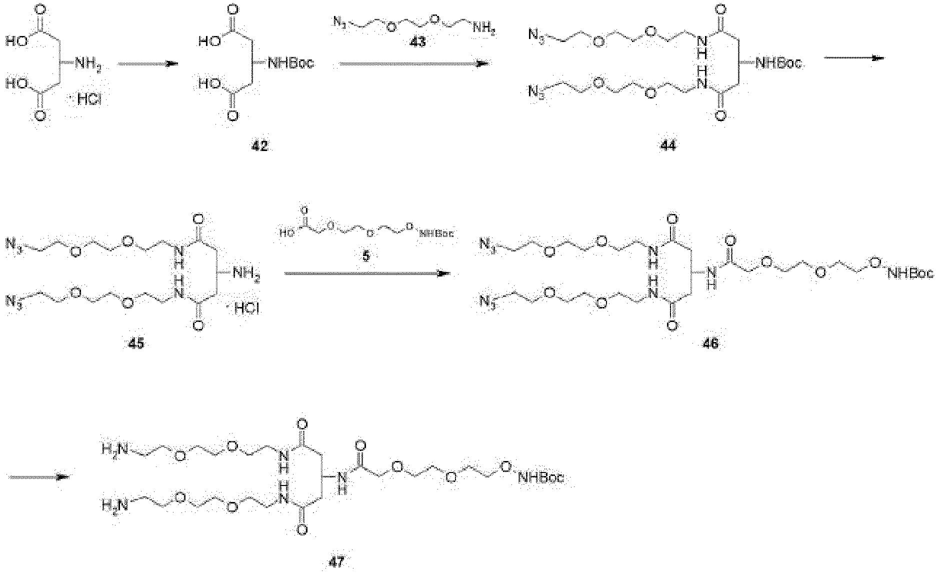

### Preparation of Compound 42

In distilled water/1,4-dioxane (6 mL/44 mL), 3-aminopentanedioic acid hydrochloride (5.0 g, 27.2 mmol) was dissolved, and then aqueous sodium hydroxide solution (4 M, 20.4 mL, 81.6 mmol) and di-t-butyl dicarbonate (6.87 mL, 29.9 mmol) were added. The reaction mixture was stirred at room temperature for 15 hours, and the pH was adjusted to 2 with 5% aqueous potassium hydrogen sulfate solution, followed by extraction with ethyl acetate (3 × 50 mL). The collected organic layers were dried over anhydrous sodium sulfate, filtered, and concentrated to afford Compound 42 (4.0 g, 60%). ¹H-NMR (400MHz, CD₃OD) δ 4.25 (t, *J* = 6.2 Hz, 1H), 2.56 (d, *J* = 5.6 Hz, 4H), 1.43 (s, 9H). EI-MS m/z : [M+Na]⁺ 270.3.

### Preparation of Compound 44

Compound 42 (3.04 g, 12.3 mmol) and Compound 43 (4.28 g, 24.6 mmol, Compound 43 was prepared by the method described in J. Am. Chem. Soc. 2016, 138, 3382-3394) were dissolved in N,N-dimethylformamide (25 mL), and then *N*,*N*,*N*',*N*'-tetramethyl-*O*-(1*H*-benzotriazol-1-yl)uronium hexafluorophosphate (7.02 g, 18.45 mmol), and N,N-diisopropylethylamine (4.28 mL, 24.6 mmol) were added at 0°C under a nitrogen atmosphere. The reaction mixture was stirred at room temperature for 6 hours, and then a saturated aqueous ammonium chloride solution (50 mL) was added to the reaction mixture, followed by extraction with ethyl acetate (2 × 50 mL). The collected organic layers were washed with brine (30 mL) and dried over anhydrous sodium sulfate. Filtration, concentration, and purification by column chromatography were performed to afford Compound 44 (3.93 g, 57%) . ¹H-NMR (400 MHz, CDCl₃) δ 6.84 (s, 2H), 6.06 (s, 1H), 4.17 (m, 1H), 3.66-3.61 (m, 16H), 3.45-3.39 (m, 8H), 2.60-2.56 (m, 2H), 2.37-2.32 (m, 2H), 1.43 (s, 9H).

### Preparation of Compound 45

Compound 44 (3.93 g, 7.02 mmol) was dissolved in dichloromethane (12 mL), and then hydrochloric acid (4 M in 1,4-dioxane, 12.3 mL, 49.1 mmol) was added at 0°C under a nitrogen atmosphere. The reaction mixture was stirred at room temperature for 1 hour, and then concentrated under reduced pressure to afford Compound 45 (crude 3.5 g). ¹H-NMR (400 MHz, CDCl₃) δ 8.45 (s, 2H), 7.41 (s, 1H), 3.97 (m, 1H), 3.66-3.43 (m, 24H), 3.29-2.93 (m, 2H), 2.76-2.73 (m, 2H). EI-MS m/z : [M+H]⁺ 460.5.

### Preparation of Compound 46

Compound 45 (crude 3.5 g) and Compound 5 (2.24 g, 8.0 mmol) were dissolved in N,N-dimethylformamide (18 mL), and then *N*,*N*,*N*',*N*'-tetramethyl-*O*-(1*H*-benzotriazol-1-yl)uronium hexafluorophosphate (3.21 g, 8.44 mmol) and N,N-diisopropylethylamine (2.44 mL, 14.04 mmol) were added at 0°C under a nitrogen atmosphere. The reaction mixture was stirred at room temperature for 15 hours, and saturated aqueous ammonium chloride solution (50 mL) was added to the reaction mixture, followed by extraction with ethyl acetate (2 × 50 mL). The collected organic layers were washed with brine (30 mL) and dried over anhydrous sodium sulfate. Filtration, concentration, and purification by column chromatography were performed to afford Compound 46 (3.97 g, 2 steps 79%). ¹H-NMR (400 MHz, CDCl₃) δ 8.45 (s, 1H), 7.88 (d, *J* = 7.2 Hz, 1H), 6.79 (s, 2H), 4.56 (m, 1H), 4.07-3.97 (m, 4H), 3.75-3.58 (m, 22H), 3.44-3.39 (m, 8H), 2.63-2.60 (m, 2H), 2.44-2.40 (m, 2H), 1.47 (s, 9H). EI-MS m/z : [M+H]⁺ 721.7.

### Preparation of Compound 47

Compound 46 (370 mg, 0.51 mmol) was dissolved in tetrahydrofuran (5 mL), and then triphenylphosphine (296 mg, 1.13 mmol) and distilled water (0.18 mL) were added. The reaction mixture was heated to reflux, stirred for 4 hours, and purified by column chromatography to afford Compound 47 (316 mg, 92%). ¹H-NMR (400 MHz, CDCl₃) δ 4.57 (m, 1H), 4.07-3.97 (m, 4H), 3.75-3.42 (m, 30H), 3.01 (m, 2H), 2.57 (m, 2H), 1.47 (s, 9H). EI-MS m/z : [M+H]⁺ 669.8.

### <Example 13> Preparation of Compound 50

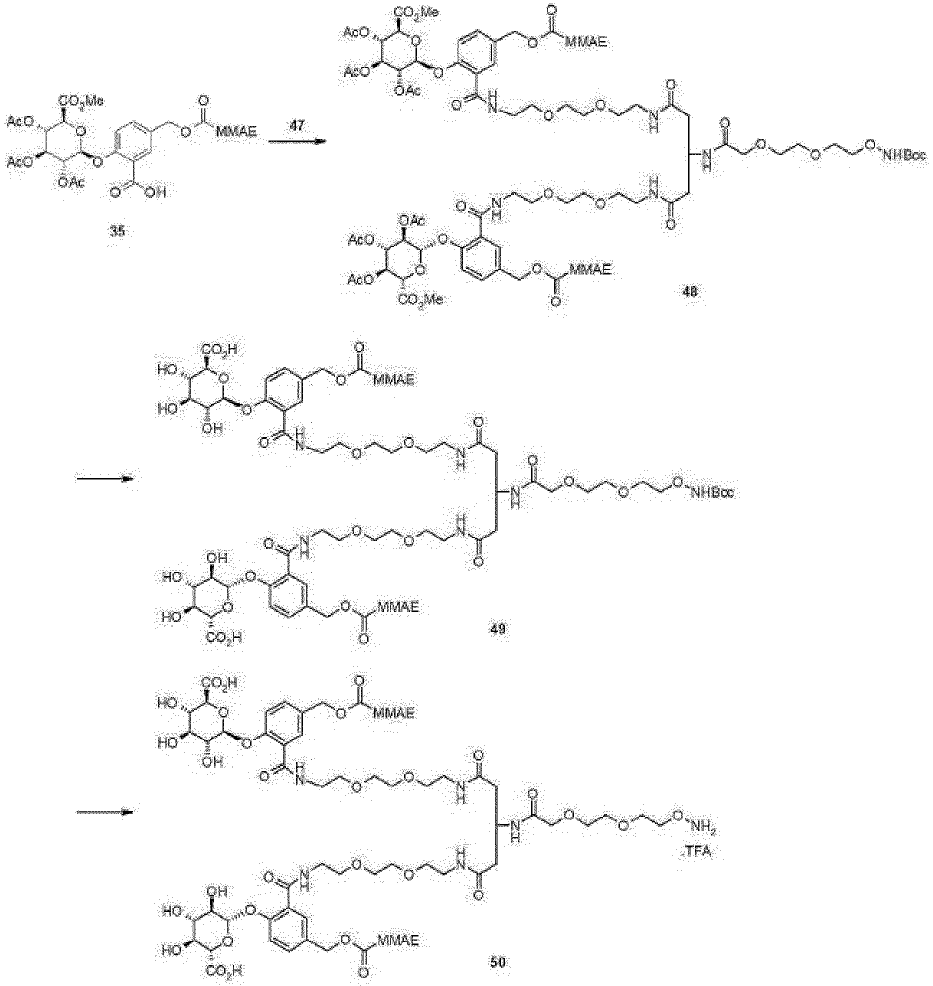

### Preparation of Compound 48

Compound 47 (37 mg, 0.055 mmol) and Compound 35 (143 mg, 0.116 mmol) were dissolved in N,N-dimethylformamide (2 mL), and then *N*,*N*,*N*',*N*'-tetramethyl-*O*-(1*H-*benzotriazol-1-yl)uronium hexafluorophosphate (43.9 mg, 0.111 mmol) and N,N-diisopropylethylamine (0.04 mL, 0.221 mmol) were added at 0°C under a nitrogen atmosphere. The reaction mixture was stirred at room temperature for 72 hours, and then a saturated aqueous ammonium chloride solution (20 mL) was added to the reaction mixture, followed by extraction with ethyl acetate (2 × 20 mL). The collected organic layers were washed with brine (10 mL) and dried over anhydrous sodium sulfate. Filtration, concentration, and purification by column chromatography were performed to afford Compound 48 (95 mg, 56%). EI-MS m/z : 1/2[M+H]⁺ 1546.1, 1/3[M+H]⁺ 1031.1.

### Preparation of Compound 49

Compound 48 (95 mg, 0.031 mmol) was dissolved in methanol/tetrahydrofuran (0.8 mL/1.6 mL), and then a solution of lithium hydroxide (12.9 mg, 0.31 mmol) in distilled water (1.6 mL) was gradually added at -40°C. The mixture was stirred for 1 hour while gradually raising the reaction temperature to 0°C. The reaction mixture was neutralized with acetic acid, then concentrated under reduced pressure, and lyophilized to afford Compound 49 (crude 105.6 mg). EI-MS m/z : 1/2[M+H]⁺ 1406.0, 1/3[M+H]⁺ 937.6.

### Preparation of Compound 50

Compound 49 (crude 105.6 mg) was dissolved in dichloromethane (3.5 mL), trifluoroacetic acid (0.7 mL) was added at 0°C, and the mixture was stirred for 3 hours. The reaction mixture was concentrated under reduced pressure, purified by HPLC, and lyophilized to afford Compound 50 as a white solid (27 mg, 2 steps 31%). EI-MS m/z : 1/2[M+H]⁺ 1355.9, 1/3[M+H]⁺ 904.3.

### <Example 14> Preparation of Compound 51

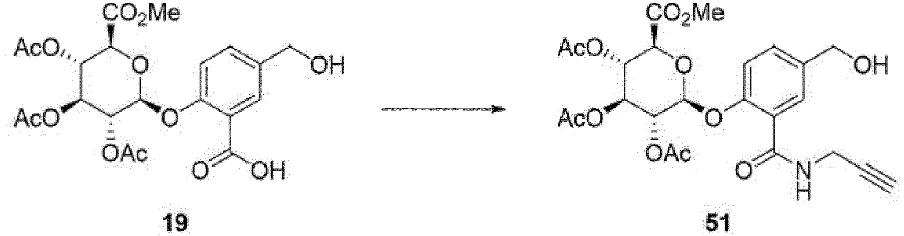

Compound 19 (5 g, 10.32 mmol) was dissolved in *N,N-*dimethylformamide (50 mL), and then propargylamine (0.8 mL, 12.38 mmol), N,N-diisopropylethylamine (3.6 mL 20.64 mmol), and *N*,*N*,*N*',*N*'-tetramethyl-*O*-(1*H*-benzotriazol-1-yl)uronium hexafluorophosphate (4.69 g, 12.38 mmol) were added at 0°C under a nitrogen atmosphere. The reaction mixture was stirred at 0°C for 30 minutes and then stirred at room temperature for 1 hour. Distilled water (50 mL) was added to the reaction mixture, followed by extraction with ethyl acetate (2 × 50 mL). The collected organic layers were dried over anhydrous sodium sulfate, filtered, concentrated under reduced pressure, and purified by column chromatography to afford Compound 51 (4.4 g, 82%). ¹H-NMR (400 MHz, CDCl₃) δ 8.07 (s, 1H), 7.58 (br, 1H), 7.05 (d, *J* = 8.8 Hz, 1H), 7.03 (d, *J* = 8.8 Hz, 1H), 5.42-5.31 (m, 4H), 4.67 (d, *J* = 4.8 Hz, 2H), 4.29-4.13 (m, 3H), 3.75 (s, 3H), 2.24 (s, 1H), 2.06 (s, 9H) .

### <Example 15> Preparation of Compound 54

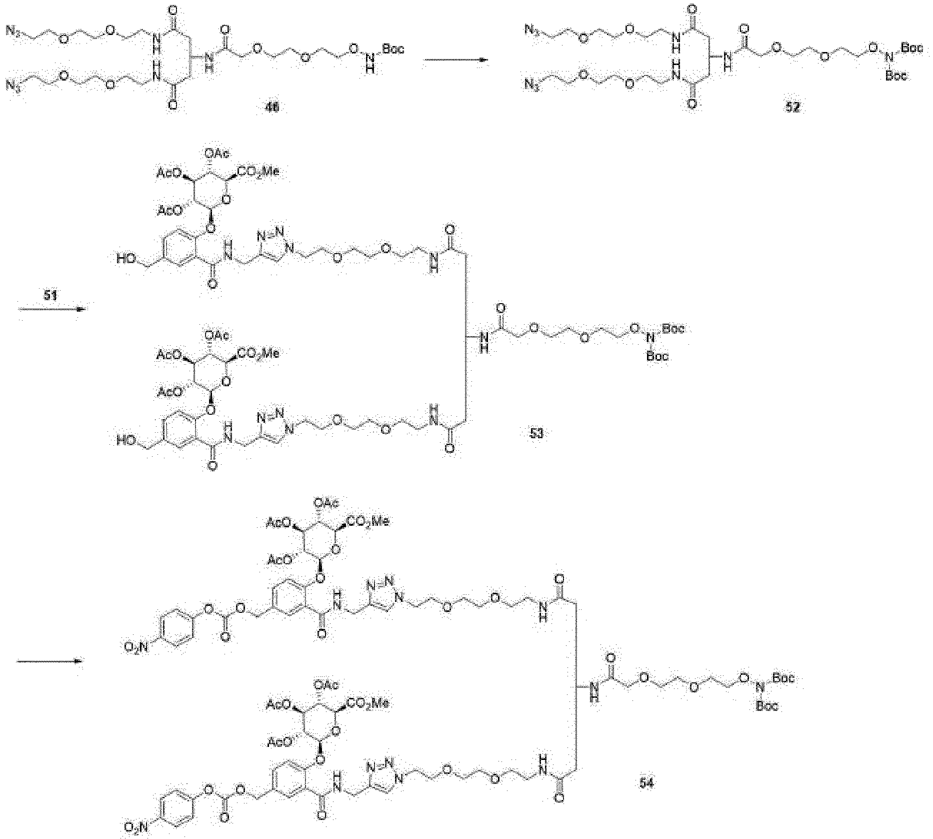

### Preparation of Compound 52

Compound 46 (1.73 g, 2.4 mmol) was dissolved in dichloromethane (10 mL), and then the solution was cooled to 0°C. Then di-t-butyl dicarbonate (0.628 g, 2.88 mmol), 4-dimethylaminopyridine (0.058 g, 0.48 mmol), and triethylamine (0.485 g, 4.8 mmol) were sequentially added under a nitrogen atmosphere. The temperature was raised to room temperature, and the mixture was stirred for about 1 hour. After completion of the reaction, the reaction mixture was diluted with water (10 mL) and extracted with dichloromethane (2 × 50 mL), and the organic layer was dried over anhydrous sodium sulfate, filtered, and concentrated under reduced pressure. Purification by column chromatography was performed to afford Compound 52 (1.33 g, 66%). EI-MS m/z: [M+H]⁺ 821.9, ¹H-NMR (400 MHz, CDCl₃) δ 7.98 (d, *J* = 8.4 Hz, 1H), 6.86 (t, *J* = 5.6 Hz, 2H), 4.60-4.50 (m, 1H), 4.10 (t, *J* = 4.4 Hz, 2H), 3.99 (s, 2H), 3.77 (t, *J* = 4.8 Hz, 4H), 3.74-3.64 (m, 14H), 3.60 (t, *J* = 4.8 Hz, 4H), 3.46-3.77 (m, 8H), 2.62-2.58 (m, 2H), 2.40-2.35 (m, 2H), 1.54 (s, 18H).

### Preparation of Compound 53

Compound 52 (500 mg, 0.61 mmol) and Compound 51 (700 mg, 1.34 mmol) were dissolved in t-butanol (10 mL), and then copper sulfate (30.4 mg, 0.12 mmol) and sodium ascorbate (121 mg, 0.61 mmol) dissolved in distilled water (10 mL) were added at 0°C. To the reaction mixture was added tris(3-hydroxypropyltriazolemethyl)amine (THPTA) (106 mg, 0.24 mmol), and the mixture was stirred at room temperature for 2 hours under a nitrogen atmosphere. The reaction mixture was diluted with ethyl acetate (100 mL) and washed with distilled water (70 mL) and saturated sodium chloride solution (70 mL). The organic layer was dried over anhydrous sodium sulfate, filtered, concentrated under reduced pressure, and then purified by column chromatography to afford Compound 53 (1.12 g, 97%). EI-MS m/z: [M+H]⁺ 1864.7, 1/2[M+H]⁺ 932.97, ¹H-NMR (400 MHz, CDCl₃) δ 8.06-7.95 (m, 5H), 7.85 (d, *J =* 2.4 Hz, 2H), 7.48-7.38 (m,4H), 6.99 (d, *J* = 8.4 Hz, 2H), 5.44-5.25 (m, 8H), 4.77-4.72 (m, 2H), 4.67-4.60 (m, 6H0, 4.56-4.50 (m, 4H), 4.42-4.32 (m, 1H), 4.30-4.21 (m, 4H), 4.08 (t, *J* = 4.8 Hz, 2H), 3.92 (s, 2H), 3.83 (t, *J* = 4.8 Hz, 4H), 3.27 (t, *J* = 5.6 Hz, 4H), 2.51-2.47 (m, 2H), 2.36-2.28 (m, 2H), 2.06 (s, 9H), 2.05 (s, 9H), 1.53 (s, 18H) .

### Preparation of Compound 54

Compound 53 (1.12 g, 0.60 mmol) and bis(4-nitrophenyl)carbonate (0.64 g, 2.10 mmol) were dissolved in N,N-dimethylformamide (10 mL), *N,N-*diisopropylethylamine (0.42 mL 2.40 mmol) was added at 0°C, and the mixture was stirred at room temperature for 4 hours. The reaction mixture was diluted with ethyl acetate (70 mL) and washed with distilled water (3 × 60 mL) and saturated sodium chloride solution (70 mL). The organic layer was dried over anhydrous sodium sulfate, filtered, concentrated under reduced pressure, and then purified by column chromatography to afford Compound 54 (1.06 g, 81%). EI-MS m/z: [M+H]⁺ 2195.3, 1/2[M+H]⁺ 1098.1, ¹H-NMR (400 MHz, CDCl₃) δ 8.27 (d, *J* = 9.28 Hz, 4H), 8.15 (d, *J* = 2 Hz, 2H), 7.95-7.89 (m, 3H), 7.79 (s, 2H), 7.54-7.51 (m, 2H), 7.38 (d, *J* = 8.8 Hz, 4H), 7.10-7.02 (m, 4H), 5.44-5.25 (m, 11H), 4.80-4.75 (m, 2H), 4.68-4.63 (m, 2H), 4.58-4.44 (m, 5H), 4.23 (d, *J* = 10 Hz, 2H), 4.09 (t, *J* = 4.4 Hz, 2H), 3.95 (s, 2H), 3.88 (t, *J* = 5.2 Hz, 4H), 3.79-3.62 (m, 12H), 3.60-3.52 (m, 8H), 3.52-3.44 (m, 5H), 3.44-3.33 (m, 4H), 2.62-2.57 (m, 2H), 2.42-2.36 (m, 2H), 2.07 (s, 9H), 2.06 (s, 9H), 1.53 (s, 18H).

### <Example 16> Preparation of Compound 59

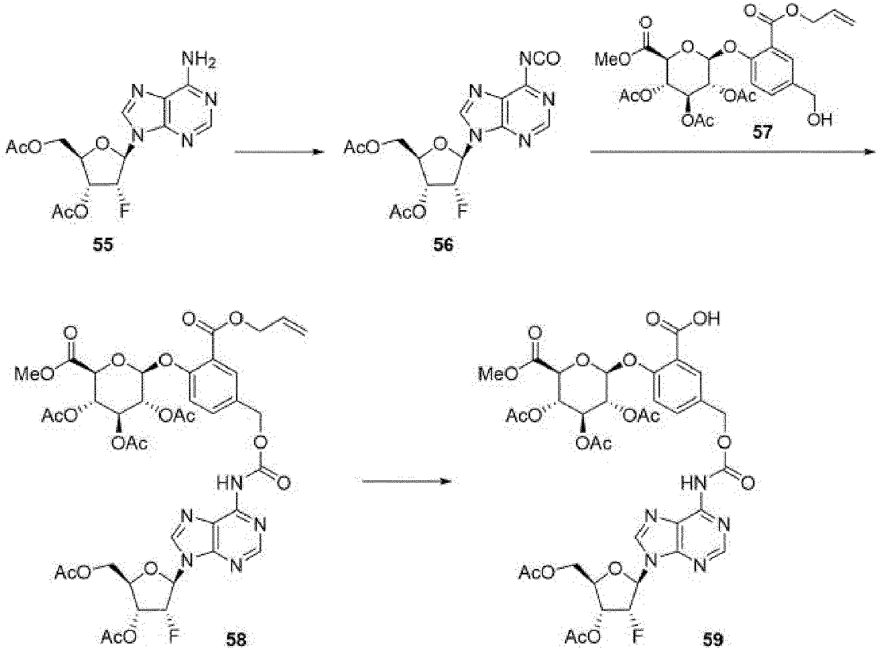

### Preparation of Compound 58

Compound 55 (300 mg, 0.85 mmol) was dissolved in dichloromethane (3 mL), and then 1-methylimidazole (0.067 mL, 0.85 mmol) and triphosgene (252 mg, 0.85 mmol) were sequentially added at 0°C under a nitrogen atmosphere. The reaction mixture was stirred for 3 hours, then 1-methylimidazole (0.2 mL, 2.54 mmol) and triphosgene (90.7 mg, 0.03 mmol) were further added, the reaction temperature was raised to room temperature, and the mixture was stirred for 3 hours. The reaction mixture was concentrated under reduced pressure and purified by column chromatography to afford Compound 56 (241 mg, 75%).

Compound 56 obtained above was dissolved in tetrahydrofuran (7 mL), then Compound 57 (456 mg, 0.87 mmol, Compound 57 was prepared by the method described in Korean Patent Publication No. 10-2018-0078329) and 1-methylimidazole (0.69 mL, 0.87 mmol) were added, and the mixture was heated to reflux for 12 hours. The reaction mixture was concentrated under reduced pressure and purified by column chromatography to afford Compound 58 (273 mg, 45%). EI-MS m/z : [M+H]⁺ 904.5.

### Preparation of Compound 59

Compound 58 (270 mg, 0.298 mmol) was dissolved in dichloromethane (3 mL), then tetrakis(triphenylphosphine)palladium(0) (17.2 mg, 0.015 mmol) and pyrrolidine (0.03 mL, 0.388 mmol) were added at 0°C under a nitrogen atmosphere, and the mixture was stirred at room temperature for 1 hour. The reaction mixture was diluted with 0.5 N aqueous hydrochloric acid (10 mL), followed by extraction with dichloromethane (20 mL) and drying over anhydrous sodium sulfate. Filtration, concentration under reduced pressure, and purification by column chromatography were performed to afford Compound 59 (250 mg, 97%) . EI-MS m/z : [M+H]⁺ 864.4.

### <Example 17> Preparation of Compound 61

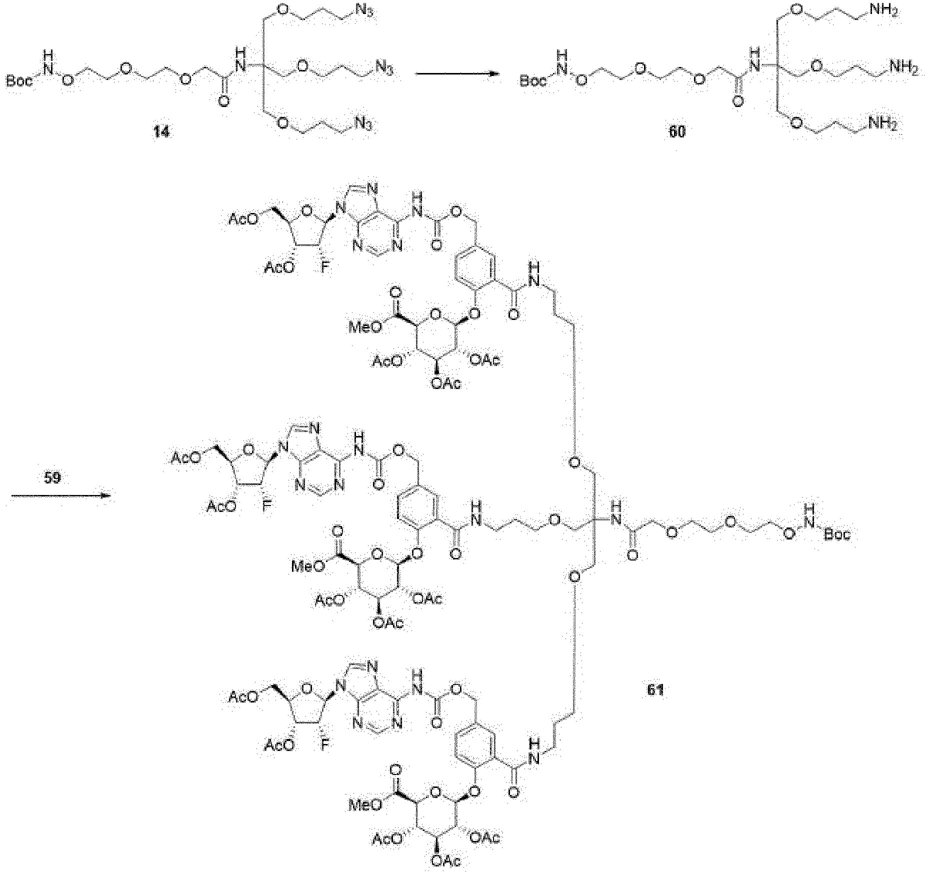

### Preparation of Compound 60

Compound 14 (300 mg, 0.47 mmol) was dissolved in tetrahydrofuran (5 mL), and then triphenylphosphine (447 mg, 1.70 mmol) and distilled water (0.6 mL) were added. The reaction mixture was heated to reflux and stirred for 4 hours. The reaction mixture was concentrated under reduced pressure and purified by column chromatography to afford Compound 60 (208 mg, 76%). EI-MS m/z : [M+H]⁺ 554.4.

### Preparation of Compound 61

Compound 59 (259 mg, 0.30 mmol) and Compound 60 (50.3 mg, 0.09 mmol) were dissolved in *N,N-*dimethylformamide (2 mL), and then *N*,*N*,*N*',*N*'-tetramethyl-*O*-(1*H*-benzotriazol-1-yl)uronium hexafluorophosphate (204 mg, 0.54 mmol) and N,N-diisopropylethylamine (0.11 mL, 0.63 mmol) were added at 0°C under a nitrogen atmosphere. The reaction mixture was stirred at room temperature for 72 hours, diluted with a saturated aqueous ammonium chloride solution (20 mL), and extracted with ethyl acetate (2 × 20 mL). The collected organic layers were washed with brine (10 mL) and dried over anhydrous sodium sulfate. Filtration, concentration, and purification by column chromatography were performed to afford Compound 61 (100 mg, 36%). EI-MS m/z : 1/2[M+H]⁺ 1546.4.

### <Example 18> Preparation of Compound 62

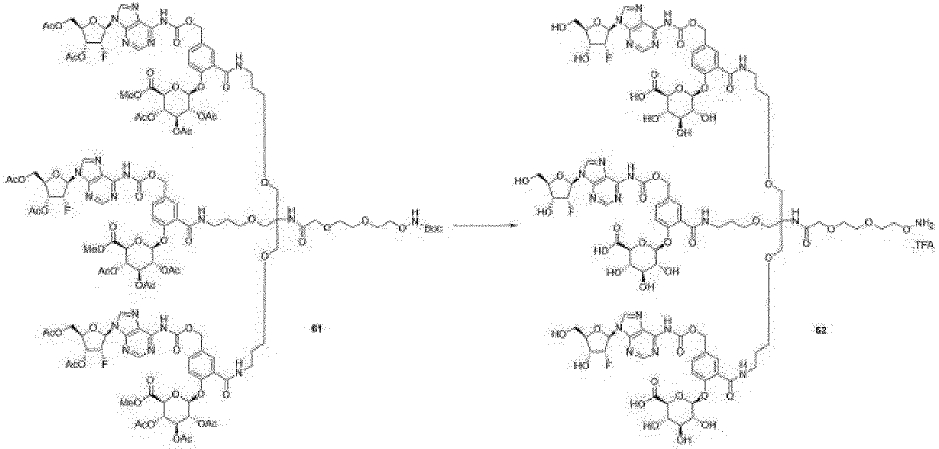

Compound 61 (100 mg, 0.032 mmol) was dissolved in methanol/tetrahydrofuran (0.8 mL/1.6 mL), and then a solution of lithium hydroxide (13.0 mg, 0.32 mmol) in distilled water (1.6 mL) was gradually added at -40°C. The mixture was stirred for 1 hour while gradually raising the reaction temperature to 0°C. The reaction mixture was neutralized with acetic acid, then concentrated under reduced pressure, and lyophilized to afford a hydrolyzed compound before purification (crude, EI-MS m/z : 1/2[M+H]⁺ 1210.0).

The compound (crude) obtained above was dissolved in dichloromethane (3.5 mL), then trifluoroacetic acid (0.7 mL) was added at 0°C, and the mixture was stirred for 3 hours. The reaction mixture was concentrated under reduced pressure, purified by HPLC, and lyophilized to afford Compound 62 as a white solid (21 mg, 2 steps 27%). EI-MS m/z : 1/2[M+H]⁺ 1160.04, [M+H]⁺ 2319.4.

### <Example 19> Preparation of Compound 63

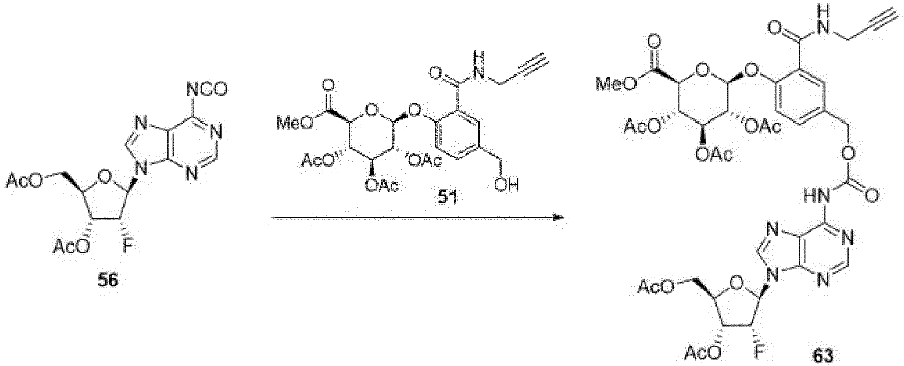

Compound 56 was dissolved in tetrahydrofuran (7 mL), then Compound 51 (488 mg, 0.935 mmol) and 1-methylimidazole (0.69 mL, 0.87 mmol) were added, and the mixture was heated to reflux for 12 hours. The reaction mixture was concentrated under reduced pressure and purified by column chromatography to afford Compound 63 (224 mg, 28%). EI-MS m/z : [M+H]⁺ 901.6.

### <Example 20> Preparation of Compound 65

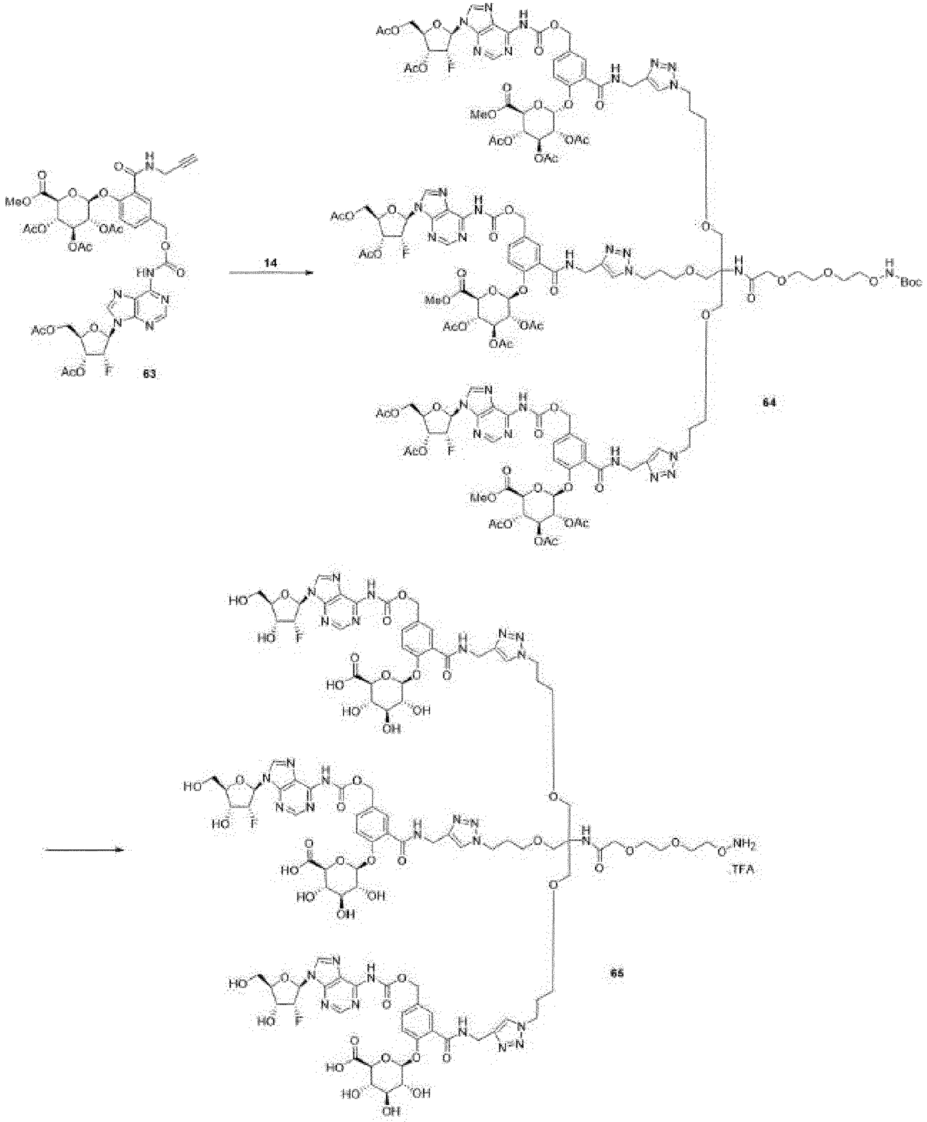

### Preparation of Compound 64

Compound 63 (138 mg, 0.153 mmol) and Compound 14 (29.3 mg, 0.046 mmol) were dissolved in t-butanol (1 mL), and then copper sulfate (2.3 mg, 0.009 mmol) and sodium ascorbate (9.27 mg, 0.046 mmol) dissolved in distilled water (1 mL), and tris-[1-(3-hydroxypropyl)-1*H*-[1,2,3]-triazol-4-yl]methyl)amine (THPTA, 8.3 mg, 0.0189 mmol) were added at 0°C under a nitrogen atmosphere. The reaction mixture was stirred at room temperature for 1 hour, and tetrahydrofuran (0.5 mL) was added, followed by further stirring at room temperature for 5 hours. Anhydrous magnesium sulfate was added to the reaction mixture, and the mixture was filtered, concentrated under reduced pressure, and purified by column chromatography to afford Compound 64 (140 mg, 27%). EI-MS m/z: 1/2[M+H]⁺ 1668.1.

### Preparation of Compound 65

Compound 64 (140 mg, 0.04 mmol) was dissolved in methanol/tetrahydrofuran (2 mL/1 mL), and a solution of lithium hydroxide (26.0 mg, 0.629 mmol) in distilled water (3 mL) was gradually added at -40°C. The mixture was stirred for 5 hours while gradually raising the reaction temperature to 0°C. The reaction mixture was neutralized with acetic acid, then concentrated under reduced pressure, and lyophilized to afford a hydrolyzed compound before purification (crude, EI-MS m/z : 1/2[M+H]⁺ 1331.7).

The compound (crude) obtained above was dissolved in dichloromethane (4 mL), trifluoroacetic acid (1 mL) was added at 0°C, and the mixture was stirred for 4 hours. The reaction mixture was concentrated under reduced pressure, purified by HPLC, and lyophilized to afford Compound 65 as a white solid (15.2 mg, 2 steps 14%). EI-MS m/z : 1/2[M+H]⁺ 1281.7.

### [Preparation of ADC]

ADCs were prepared through the following two steps, and commonly used LCB14-0511, LCB14-0512 and LCB14-0606 were prepared by the method described in Korean Patent Application Laid-Open No. 10-2014-0035393. The structural formulas of LCB14-0606, LCB14-0511 and LCB14-0512 are as follows:

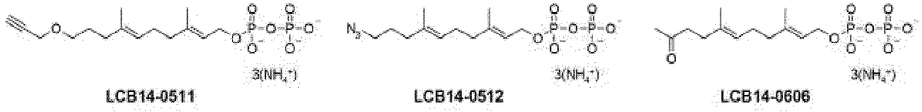

### Step 1: Preparation of prenylated antibody

A reaction mixture for prenylation of antibody was prepared and reacted at 30°C for 16 hours. The reaction mixture was composed of 24 µM antibody, 200 nM FTase (Calbiochem #344145), and a buffer (50 mM Tris-HCl (pH 7.4), 5 mM MgCl₂, 10 µM ZnCl₂, 0.5 mM DTT) containing 0.144 mM LCB14-0511 or LCB14-0512 or LCB14-0606. After completion of the reaction, the prenylated antibody was decontaminated with G25 Sepharose column (AKTA purifier, GE healthcare) equilibrated with PBS buffer.

### Step 2: Drug-conjugation method

### <Conjugation by oxime bond formation>

A mixture for oxime bond formation reaction between the prenylated antibody and a linker-drug was prepared by mixing 100 mM Na-acetate buffer pH 5.2, 10% DMSO, 24 µM antibody, and 240 µM linker-drug (in house, the compounds in Table 1 as the final products of Examples 10, 11, and 13) together, and gently stirred at 30°C. After 24 hours of reaction, excess low molecular weight compounds were removed through FPLC (AKTA purifier, GE healthcare) process, and protein fractions were collected and concentrated.

### <Conjugation by click reaction>

A mixture for click reactions between the prenylated antibody and the linker-drug was prepared by mixing 10% DMSO, 24 µM antibody and 240 µM linker-drug (in house), 1 mM copper(II) sulfate pentahydrate, 2 mM (BimC4A)3 (Sigma-Aldrich 696854), 10 mM sodium ascorbate, and 10 mM aminoguanidine hydrochloride, reacted at 25°C for 3 hours, then treated with 2.0 mM EDTA, and reacted for 30 minutes. After completion of the reaction, excess low molecular weight compounds were removed through FPLC (AKTA purifier, GE healthcare) process, and the protein fractions were collected and concentrated.

**[Table 1]**

| Prepared ADC list | |
|---|---|
| ADCs | Linker-Toxin |
| ADC1 | Compound 39 (Example 10) |
| ADC2 | Compound 41 (Example 11) |
| ADC3 | Compound 50 (Example 13) |

### <Experimental Example 1> In vitro cytotoxicity evaluation

The cell proliferation inhibitory activity of the drug and ADCs listed in Table 2 below on cancer cell lines was measured. As the cancer cell lines, commercially available human breast cancer cell lines MCF-7 (HER2 negative to normal), SK-BR3 (HER2 positive), and JIMT-1 (HER2 positive) were used. MMAE was used as the drug, and the ADCs in Table 1 were used as the ADC. Each cancer cell line was seeded in a 96-well plate at 2,500 to 5,000 cells per well for 144-hour treatment group and 1,500 to 3,000 cells per well for 168-hour treatment group, cultured for 24 hours, and then treated with the ADCs and drug at concentrations of 0.00015 to 10.0 nM (4-fold serial dilution) or 0.0015 to 10 nM (3-fold serial dilution). After 144/168 hours, the number of viable cells was quantified using SRB (sulforhodamine B) dye.

**[Table 2]**

| Comparison of cytotoxicity among ADC samples | | | |
|---|---|---|---|
| Test samples | CC50 (nM) | | |
| | SK-BR3 | JIMT-1 | MCF7 |
| MMAE | 0.12 | 0.09 | 0.31 |
| ADC1 | 0.006 | 0.08 | >10 |
| ADC2 | 0.005 | 0.05 | >10 |
| ADC3* | 0.01 | 0.12 | >10 |

| | | | |
|---|---|---|---|
| * 144 h incubation. | | | |

It has been confirmed that ADC1, ADC2, and ADC3 exhibit greatly excellent cytotoxicity to breast cancer cell lines.

Those skilled in the art will recognize, or be able to ascertain using no more than routine experimentation, many equivalents to the specific embodiments of the present invention described herein. Such equivalents are intended to be covered by the following claims. The above description of the present application is for illustration, and those skilled in the art to which the present application pertains will understand that it can be easily modified into other specific forms without changing the technical spirit or essential features of the present application. Therefore, it should be understood that the embodiments described above are illustrative in all respects and not restrictive. For example, each component described as a single type may be implemented in a dispersed form, and likewise components described as distributed may be implemented in a combined form. All changes or modifications derived from the meaning and scope of the claims to be described below, and equivalents thereof, may be construed as being included in the scope of the present invention.

### [Industrial Applicability]

The ligand-drug conjugate according to an embodiment of the present invention can include a linker having a tris structure, and in the ligand-drug conjugate, the active agent is bound by the tris structure of the linker and thus a greater number of active agents can be connected through one linker. In other words, a greater number of active agents per ligand binding can be delivered to the target cell. Consequently, the ligand-drug conjugate of the present invention can effectively, specifically, and selectively deliver a drug, stably reach a target cell during circulation in the body, and easily release the drug after arrival. The linker according to an embodiment of the present invention can be used in the art since it includes a trigger unit capable of maximizing drug efficacy by easily releasing the drug within the target cell and thus the drug and/or toxin can stably reach the target cell and effectively exert the drug efficacy.

## Claims

1. A linker for ligand-drug conjugate comprising a tris structure represented by the following General Formula 1A:

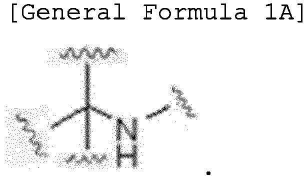

2. The linker for ligand-drug conjugate according to claim 1, wherein the linker includes a first linker connected to an active agent and a second linker connected to an antibody, wherein the first linker or the second linker has a tris structure represented by General Formula 1A.

3. The linker for ligand-drug conjugate according to claim 1, wherein the linker includes first linkers (BL₁, BL₂, and BL₃) that are connected to nitrogen of a tris structure and bound to an active agent and a second linker (SL) connected to an antibody, and is represented by the following General Formula 2A:

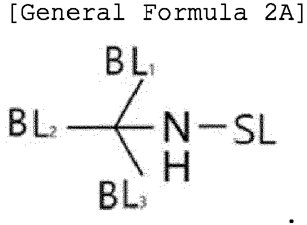

4. The linker for ligand-drug conjugate according to claim 3, wherein the first linkers include branched linkers (BL₁, BL₂, and BL₃).

5. The linker for ligand-drug conjugate according to claim 4, wherein any one of the branched linkers is hydrogen and the linker is represented by the following General Formula 3A:

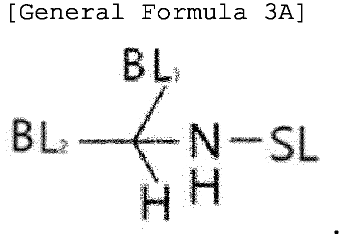

6. The linker for ligand-drug conjugate according to claim 1, wherein the tris structure is represented by the following General Formula 4A:

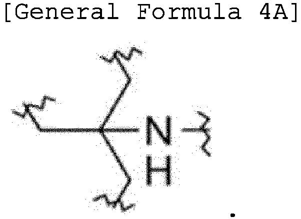

7. The linker for ligand-drug conjugate according to claim 1, wherein the linker includes a first linker that is connected to nitrogen of a tris structure, is bound to an active agent, and includes branched linkers (BL₁, BL₂, and BL₃) and a second linker (SL) connected to an antibody, and is represented by the following General Formula 5A:

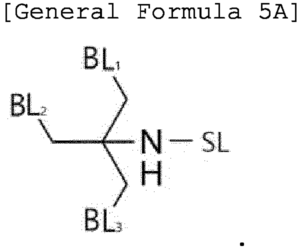

8. The linker for ligand-drug conjugate according to claim 7, wherein any one of the branched linkers is hydrogen and the linker is represented by the following General Formula 6A:

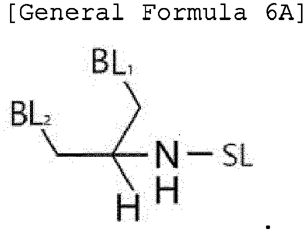

9. The linker for ligand-drug conjugate according to claim 1, wherein the linker includes at least one or more polyalkylene glycol units.

10. The linker for ligand-drug conjugate according to claim 1, wherein the linker includes an arbitrary atom or group that forms three bonds, such as an amine, a tertiary amide, or tertiary or quaternary carbon.

11. The linker for ligand-drug conjugate according to claim 1, wherein the linker includes at least one or more amino acids.

12. The linker for ligand-drug conjugate according to claim 1, wherein the linker includes at least one or more maleimide units represented by the following Chemical Formula 1G:

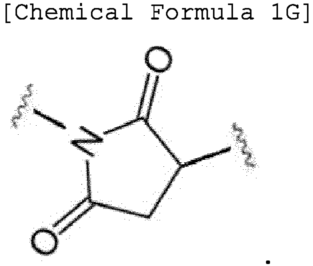

13. The linker for ligand-drug conjugate according to claim 1, wherein the linker includes substituted or unsubstituted alkylene having 1 to 100 carbon atoms, and satisfies one or more of the following requirements (i) to (iv):
(i) the alkylene has at least one unsaturated bond, specifically 3 or 4 double or triple bonds,
(ii) the alkylene includes at least one heteroarylene;
(iii) at least one carbon atom of the alkylene is substituted with one or more heteroatoms selected from nitrogen (N), oxygen (O) or sulfur (S), specifically at least one nitrogen and at least one oxygen (for example, oxygen in an oxime), and
(iv) the alkylene is substituted with one or more alkyls having 1 to 20 carbon atoms, preferably 2 or 3 methyls.

14. A ligand-drug conjugate comprising:
a ligand;
a linker that is connected to the ligand by a covalent bond and has a tris structure represented by the following General Formula 1A; and
an active agent connected to the linker by a covalent bond:

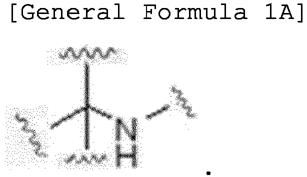

15. The ligand-drug conjugate according to claim 14, wherein the linker includes first linkers (BL₁, BL₂, and BL₃) that are connected to nitrogen of a tris structure and bound to a drug and a second linker (SL) connected to an antibody; and is represented by the following General Formula 2A:

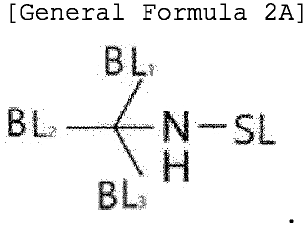

16. The ligand-drug conjugate according to claim 14, wherein the linker includes a branching unit (BR), a connection unit, or a binding unit, wherein the connection unit connects a drug and the branching unit or binding unit to each other and the binding unit or connection unit is connected to an antibody.

17. The ligand-drug conjugate according to claim 16, wherein the branching unit includes at least one or more amino acids.

18. The ligand-drug conjugate according to claim 16, wherein the branching unit includes at least one or more L-amino acids or D-amino acids.

19. The ligand-drug conjugate according to claim 16, wherein the branching unit includes at least one or more α-amino acids or β-amino acids.

20. The ligand-drug conjugate according to claim 16, wherein the branching unit includes at least one or more amino acids selected from the group consisting of lysine, 5-hydroxylysine, 4-oxalysine, 4-thialysine, 4-selenalysine, 4-thiahomolysine, 5,5-dimethyllysine, 5,5-difluorolysine, *trans*-4-dihydrolysine, 2,6-diamino-4-hexinoic acid, cis-4-dehydrolysine, 6-N-methyllysine, diaminopimelic acid, ornithine, 3-methylornithine, α-methylornithine, citrulline, and homocitrulline.

21. The ligand-drug conjugate according to claim 16, wherein the branching unit includes at least one or more hydrophilic amino acids.

22. The ligand-drug conjugate according to claim 16, wherein the branching unit includes at least one or more hydrophilic amino acids including a side chain having a residue having a charge at a neutral pH in an aqueous solution.

23. The ligand-drug conjugate according to claim 16, wherein the branching unit includes at least one or more of arginine, aspartate, asparagine, glutamate, glutamine, histidine, lysine, ornithine, proline, serine, or threonine.

24. The ligand-drug conjugate according to claim 16, wherein the branching unit includes at least one or more hydrogen atoms or alkylenes having 1 to 100 carbon atoms.

25. The ligand-drug conjugate according to claim 16, wherein the branching unit includes at least one or more alkylenes having 1 to 100 carbon atoms;
at least one or more carbon atoms of the alkylenes are substituted with one or more heteroatoms selected from nitrogen (N), oxygen (O) or sulfur (S); and
the alkylenes are further substituted with at least one or more alkyls having 1 to 20 carbon atoms.

26. The ligand-drug conjugate according to claim 16, wherein the branching unit (BR) is -C(O)-, -C(O)NR'-, - C(O)O-, -S(O)₂NR'-, -P(O)R"NR'-, -S(O)NR'-, or -PO₂NR'-, where R' and R" each independently include hydrogen, (C₁-C₈)alkyl, (C₃-C₈)cycloalkyl, (C₁-C₈)alkoxy, (C₁-C₈)alkylthio, mono- or di- (C₁-C₈)alkylamino, (C₃-C₂₀)heteroaryl, or (C₆-C₂₀)aryl.

27. The ligand-drug conjugate according to claim 16, wherein the branching unit has a structure represented by any one of the following Chemical Formulas 1B to 8B: 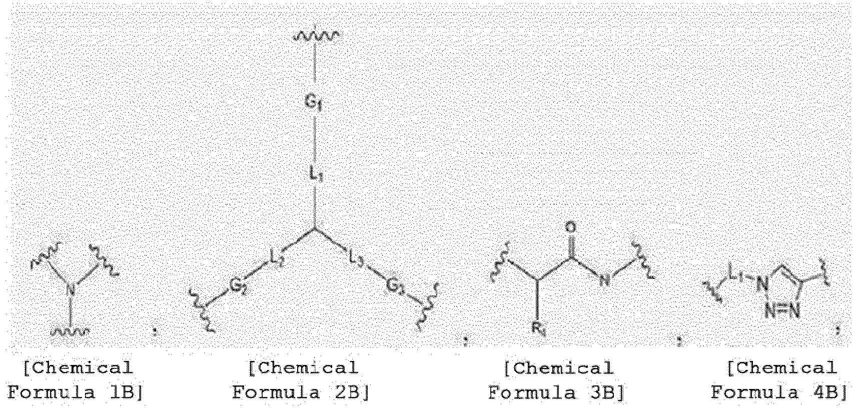 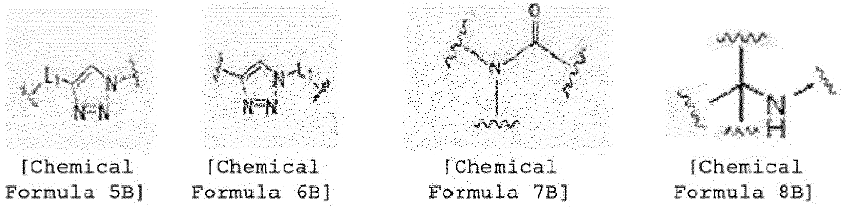 in Chemical Formulas 1B to 8B,
L₁, L₂, and L₃ are each independently a direct bond or -CₙH₂ₙ-, where n is an integer from 1 to 30,
G₁, G2, and G₃ are each independently a direct bond, 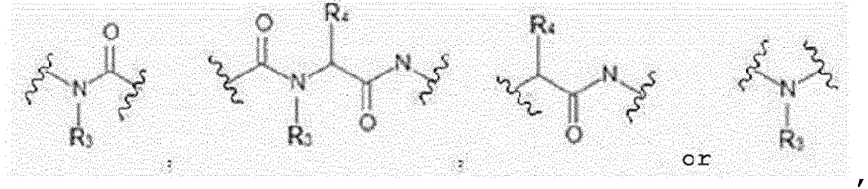 where R₃ is hydrogen or C₁-C₃₀ alkyl, and
R₄ is hydrogen or -L₄-COOR₅, where L₄ is a direct bond or -CₙH₂ₙ-, where n is an integer from 1 to 10, and R₅ is hydrogen or C₁-C₃₀ alkyl.

28. The ligand-drug conjugate according to claim 16, wherein the branching unit includes an oxime or an O-substituted oxime.

29. The ligand-drug conjugate according to claim 16, wherein the branching unit has a structure represented by the following Chemical Formula 9B or 10B:

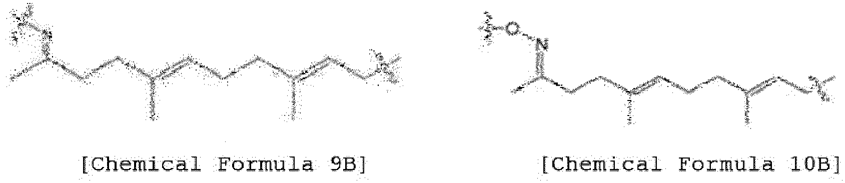

30. The ligand-drug conjugate according to claim 16, wherein the connection unit is represented by - (CH₂)ᵣ(V(CH₂)ₚ)_{q}-, -((CH₂)ₚV)_{q}-, -(CH₂)ᵣ(V(CH₂)ₚ)_{q}Y-, - ((CH₂)ₚV)_{q}(CH₂)ᵣ-, -Y((CH₂)ₚV)_{q}-, or -(CH₂)ᵣ(V(CH₂)ₚ)_{q}YCH₂-, where r is an integer from 0 to 10; p is an integer from 1 to 10; q is an integer from 1 to 20; and V and Y are each independently a single bond, -O-, -S-, -NR₂₁-, - C(O)NR₂₂-, -NR₂₃C(O)-, -NR₂₄SO₂-, or -SO₂NR₂₅-, where R₂₁ to R₂₅ are each independently hydrogen, (C₁-C₆)alkyl, (C₁-C₆)alkyl (C₆-C₂₀)aryl, or (C₁-C₆)alkyl (C₃-C₂₀)heteroaryl.

31. The ligand-drug conjugate according to claim 16, wherein the connection unit includes -(CH₂)ᵣ(V(CH₂)ₚ)_{q}-, where r is an integer from 0 to 10, p is an integer from 0 to 12, q is an integer from 1 to 20, and V is a single bond, -O-, or -S-.

32. The ligand-drug conjugate according to claim 16, wherein the connection unit includes a polyethylene glycol unit having a structure of

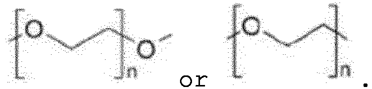

33. The ligand-drug conjugate according to claim 16, wherein the connection unit includes 1 to 12 -OCH₂CH₂-units.

34. The ligand-drug conjugate according to claim 16, wherein the connection unit includes -(CH₂CH₂X)_{W}-, where X is a single bond, -O-, (C₁-C₈)alkylene, or -NR₂₁-, where R₂₁ is hydrogen, (C₁-C₆)alkyl, (C₁-C₆)alkyl (C₆-C₂₀)aryl, or (C₁-C₆)alkyl (C₃-C₂₀)heteroaryl, and w is an integer from 1 to 20.

35. The ligand-drug conjugate according to claim 16, wherein the binding unit is formed by a 1,3-dipolar cycloaddition reaction, a hetero-Diels-Alder reaction, a nucleophilic substitution reaction, a non-aldol type carbonyl reaction, addition to a carbon-carbon multiple bond, an oxidation reaction, or a click reaction.

36. The ligand-drug conjugate according to claim 16, wherein the binding unit is formed by a reaction of acetylene with azide or a reaction of an aldehyde or a ketone group with hydrazine or an alkoxyamine.

37. The ligand-drug conjugate according to claim 16, wherein the binding unit has a structure represented by any one of the following Chemical Formulas 1D to 4D: 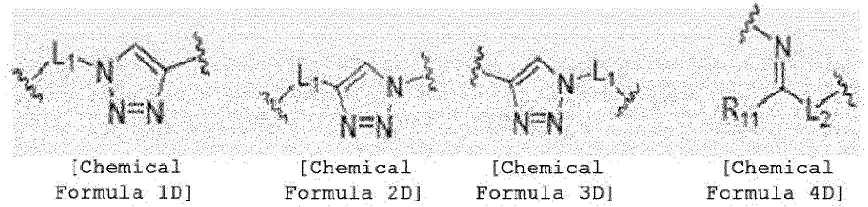 in Chemical Formulas 1D to 4D,
L₁ is a single bond or alkylene having 1 to 30 carbon atoms,
R₁₁ is hydrogen or alkyl having 1 to 10 carbon atoms, and
L₂ is alkylene having 1 to 30 carbon atoms.

38. The ligand-drug conjugate according to claim 16, wherein the binding unit includes a polyethylene glycol unit having a structure of

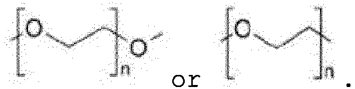

39. The ligand-drug conjugate according to claim 16, wherein the binding unit includes a maleimide unit represented by the following Chemical Formula 1G:

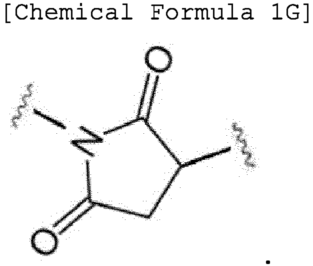

40. The ligand-drug conjugate according to claim 16, wherein the linker includes an isoprenyl unit represented by 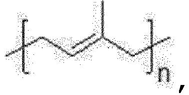 where n is an integer greater than or equal to 2.

41. The ligand-drug conjugate according to claim 14, wherein the linker includes any one of units represented by the following Chemical Formulas 4A to 6A: 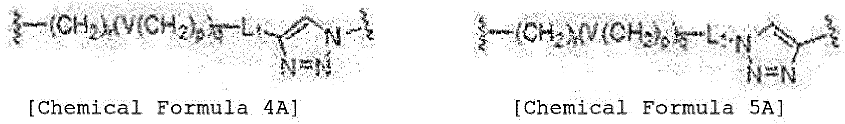 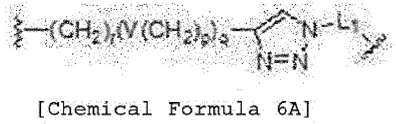 in Chemical Formulas 4A to 6A,
V denotes a single bond, -O-, -S-, -NR₂₁-, -C(O)NR₂₂-, -NR₂₃C(O)-, -NR₂₄SO₂-, or -SO₂NR₂₅-, preferably -O-; where R₂₁ to R₂₅ each independently denote hydrogen, (C₁-C₆)alkyl, (C₁-C₆)alkyl (C₆-C₂₀)aryl, or (C₁-C₆)alkyl (C₃-C₂₀)heteroaryl;
r is an integer from 0 to 10, preferably 2 or 3;
p is an integer from 0 to 10, preferably 1 or 2;
q is an integer from 1 to 20, preferably an integer from 1 to 6; and
L₁ is a single bond.

42. The ligand-drug conjugate according to claim 14, wherein the linker is a linker for ligand-drug conjugate that includes at least one or more substituted or unsubstituted alkylenes having 1 to 100 carbon atoms and satisfies one or more of the following requirements (i) to (iv):
(i) the alkylenes have at least one unsaturated bond, specifically 3 or 4 double or triple bonds,
(ii) the alkylenes include at least one heteroarylene;
(iii) at least one carbon atom of the alkylenes is substituted with one or more heteroatoms selected from nitrogen (N), oxygen (O) or sulfur (S), specifically at least one nitrogen and at least one oxygen (for example, oxygen in an oxime), and
(iv) the alkylenes are substituted with one or more alkyls having 1 to 20 carbon atoms, preferably 2 or 3 methyls.

43. The ligand-drug conjugate according to claim 14, wherein the linker is covalently bound to a ligand by a thioether bond, wherein the thioether bond includes a sulfur atom of cysteine of the ligand.

44. The ligand-drug conjugate according to claim 14, wherein the ligand includes a C-terminal amino acid motif recognized by isoprenoid transferase.

45. The ligand-drug conjugate according to claim 44, wherein the amino acid motif is a CYYX sequence; where
C denotes cysteine;
each Y independently denotes an aliphatic amino acid; and
each X independently denotes glutamine, glutamate, serine, cysteine, methionine, alanine, or leucine.

46. The ligand-drug conjugate according to claim 44, wherein the amino acid motif is a CVIM or CVLL sequence.

47. The ligand-drug conjugate according to claim 14, wherein the active agent is connected to a linker by a cleavable or non-cleavable bond, and a hydrolytic or non-hydrolytic bond.

48. The ligand-drug conjugate according to claim 14, wherein the active agent is connected to a linker through a trigger unit represented by the following Chemical Formula 1F: 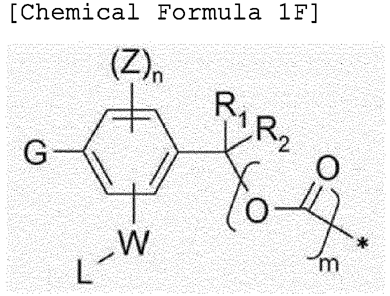 in Chemical Formula 1F,
G is a sugar, sugar acid, or a sugar derivative;
W is -C(O)-, -C(O)NR'-, -C(O)O-, -S(O)₂NR'-, - P(O)R"NR'-, -S(O)NR'-, or -PO₂NR'-, where R' and R" are each independently hydrogen, (C₁-C₈)alkyl, (C₃-C₈)cycloalkyl, (C₁-C₈)alkoxy, (C₁-C₈)alkylthio, mono- or di- (C₁-C₈)alkylamino, (C₃-C₂₀)heteroaryl, or (C₆-C₂₀)aryl when C(O), S, or P is directly connected to a phenyl ring;
each Z is independently hydrogen, (C₁-C₈)alkyl, halogen, cyano, or nitro;
n is an integer from 1 to 3,
m is 0 or 2;
R₁ and R₂ are each independently hydrogen, (C₁-C₈)alkyl or (C₃-C₈)cycloalkyl or form a (C₃-C₈)cycloalkyl ring together with a carbon atom to which R₁ and R₂ are attached;
L means connection with a linker; and
* indicates a site connected to an active agent (or drug or toxin).

49. The ligand-drug conjugate according to claim 48, wherein the trigger unit is represented by the following Chemical Formula 3F or 4F:

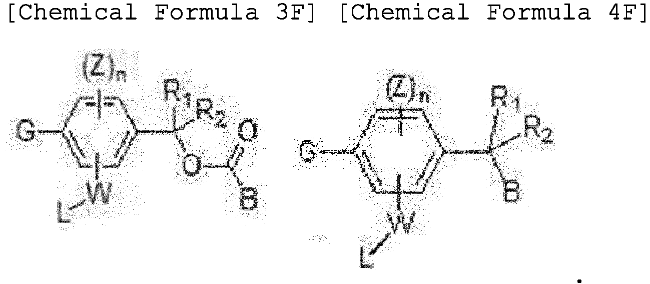

50. The ligand-drug conjugate according to claim 48, wherein the sugar and sugar acid are monosaccharides.

51. The ligand-drug conjugate according to claim 48, wherein G is represented by the following Chemical Formula 2F: 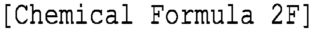 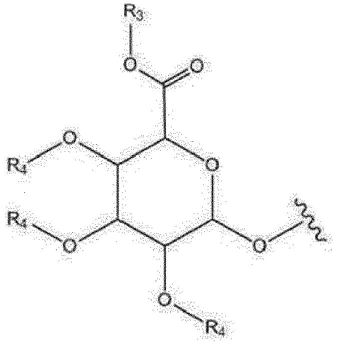 in Chemical Formula 2F,
R₃ is hydrogen or a carboxyl protecting group, and each R₄ is independently hydrogen or a hydroxyl protecting group.

52. The ligand-drug conjugate according to claim 48, wherein W is -C(O)NR'-, where C(O) is connected to a phenyl ring and NR' is connected to a linker, and G is represented by the following Chemical Formula 2F: 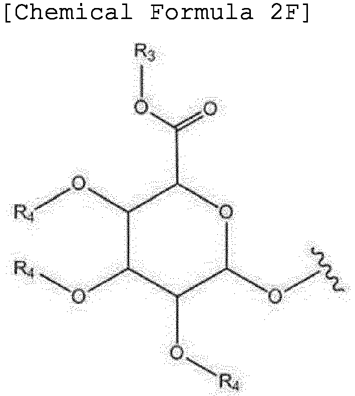 in Chemical Formula 2F,
R₃ is hydrogen or a carboxyl protecting group, and each R₄ is independently hydrogen or a hydroxyl protecting group.

53. The ligand-drug conjugate according to claim 14, wherein the active agent is a drug, a toxin, an affinity ligand, or a detection probe.

54. The ligand-drug conjugate according to claim 14, wherein the active agent is an immunomodulatory compound, an anti-cancer agent, an anti-viral agent, an anti-bacterial agent, an anti-fungal agent, or an anti-parasitic agent.

55. The ligand-drug conjugate according to claim 14, wherein the active agent is selected from active agents listed below:
(a) erlotinib, bortezomib, fulvestrant, sutent, letrozole, imatinib mesylate, PTK787/ZK 222584, oxaliplatin, 5-fluorouracil, leucovorin, rapamycin, lapatinib, lonafarnib, sorafenib, gefitinib, AG1478, AG1571, thiotepa, cyclophosphamide, busulfan, improsulfan, piposulfan, benzodopa, carboquone, meturedopa, uredopa, ethylenimine, altretamine, triethylenemelamine, trietylenephosphoramide, triethiylenethiophosphoramide, trimethylolomelamine, bullatacin, bullatacinone, camptothecin, topotecan, bryostatin, callystatin, CC-1065, adozelesin, carzelesin, bizelesin, cryptophycin 1, cryptophycin 8, dolastatin, duocarmycin, KW-2189, CB1-TM1, eleutherobin, pancratistatin, sarcodictyin, spongistatin, chlorambucil, chlornaphazine, cholophosphamide, estramustine, ifosfamide, mechlorethamine, melphalan, novembichin, phenesterine, prednimustine, trofosfamide, uracil mustard, carmustine, chlorozotocin, fotemustine, lomustine, nimustine, ranimnustine, calicheamicin, calicheamicin gamma 1, calicheamicin omega 1, dynemicin, dynemicin A, clodronate, esperamicin, neocarzinostatin chromophore, aclacinomysins, actinomycin, antrmycin, azaserine, bleomycins, cactinomycin, carabicin, carninomycin, carzinophilin, chromomycins, dactinomycin, daunorubicin, detorubucin, 6-diazo-5-oxo-L-norleucine, doxorubicin, morpholino-doxorubicin, cyanomorpholino-doxorubicin, 2-pyrrolino-doxorubucin, liposomal doxorubicin, deoxydoxorubicin, epirubicin, esorubicin, marcellomycin, mitomycin C, mycophenolic acid, nogalamycin, olivomycins, peplomycin, potfiromycin, puromycin, quelamycin, rodorubicin, streptomigrin, streptozocin, tubercidin, ubenimex, zinostatin, zorubicin, 5-fluorouracil, denopterin, methotrexate, pteropterin, trimetrexate, fludarabine, 6-mercaptopurine, thiamiprine, thiguanine, ancitabine, azacytidine, 6-azauridine, carmofur, cytarabine, dideoxyuridine, doxifluridine, enocitabine, floxuridine, calusterone, dromostanolone, propionate, epitiostanol, mepitiostane, testolactone, aminoglutethimide, mitotane, trilostane, folinic acid, aceglatone, aldophosphamide glycoside, aminolevulinic acid, eniluracil, amsacrine, bestrabucil, bisantrene, edatrexate, defofamine, demecolcine, diaziquone, elfornithine, elliptinium acetate, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, polysaccharide-k, razoxane, rhizoxin, sizofiran, spirogermanium, tenuazonic acid, triaziquone, 2,2',2''-trichlorotriethylamine, T-2 toxin, verracurin A, roridin A, anguidine, urethane, vindesine, dacarbazine, mannomustine, mitobronitol, mitolactol, pipobroman, gacytosine, arabinoside, cyclophosphamide, thiotepa, paclitaxel, albumin-engineered nanoparticle formulation of paclitaxel, docetaxel, chlorambucil, gemcitabine, 6-thioguanine, mercaptopurine, cisplatin, carboplatin, vinblastine, platinum, etoposide, ifosfamide, mitoxantrone, vincristine, vinorelbine, novantrone, teniposide, edatrexate, daunomycin, aminopterin, xeloda, ibandronate, CPT-11, topoisomerase inhibitor RFS 2000, difluoromethylornithine, retinoic acid, capecitabine, or any pharmaceutically acceptable salt, solvate or acid thereof;
(b) monokine, lympokine, traditional polypeptide hormone, parathyroid hormone, thyroxine, relaxin, prorelaxin, glycoprotein hormone, follicle stimulating hormone, thyroid stimulating hormone, luteinizing hormone, hepatic growth factor fibroblast growth factor, prolactin, placental lactogen, tumor necrosis factor-α, tumor necrosis factor-β, mullerian-inhibiting substance, mouse gonadotropin-associated peptide, inhibin, activin, vascular endothelial growth factor, thrombopoietin, erythropoietin, osteoinductive factor, interferon, interferon-α, interferon-β, interferon-γ, colony stimulating factor (CSF), macrophage-CSF, granulocyte-macrophage-CSF, granulocyte-CSF, interleukin (IL), IL-1, IL-1α, IL-2, IL-3, IL-4, IL-5, IL-6, IL-7, IL-8, IL-9, IL-10, IL-11, IL-12, tumor necrosis factor, TNF-α, TNF-β, polypeptide factor, LIF, kit ligand, or any combination of these;
(c) diphtheria toxin, botulinum toxin, tetanus toxin, dicenteritoxin, cholera toxin, amanitin, α-amanitin, pyrrolobenzodiazepine, pyrrolobenzodiazepine derivatives, tetrodotoxin, brevetoxin, ciguatoxin, ricin, AM toxin, auristatin, tubulysin, geldanamycin, maytansinoid, calicheamicin, daunomycin, doxorubicin, methotrexate, vindesine, SG2285, dolastatin, dolastatin analog, auristatin, cryptophycin, camptothecin, rhizoxin, rhizoxin derivatives, CC-1065, CC-1065 analogs or derivatives, duocarmycin, enediyne antibiotic, esperamicin, epothilone, toxoid, or any combination of these;
(d) an affinity ligand, wherein the affinity ligand is a substrate, an inhibitor, an activator, a neurotransmitter, a radioactive isotope, or any combination of these;
(e) radioactive label, 32P, 35S, fluorescent die, electron density reagent, enzyme, biotin, streptavidin, dioxigenin, hapten, immunogenic protein, nucleic acid molecule with a sequence complementary to a target, or any combination of these;
(f) an immunomodulatory compound, an anti-cancer agent, an anti-viral agent, an anti-bacterial agent, an anti-fungal agent, and an anti-parasitic agent, or any combination of these;
(g) tamoxifen, raloxifene, droloxifene, 4-hydroxytamoxifen, trioxifene, keoxifene, LY117018, onapristone, or toremifene;
(h) 4(5)-imidazole, aminoglutethimide, megestrol acetate, exemestane, letrozole, or anastrozole;
(i) flutamide, nilutamide, bicalutamide, leuprolide, goserelin, or troxacitabine;
(j) an aromatase inhibitor;
(k) a protein kinase inhibitor;
(l) a lipid kinase inhibitor;
(m) shRNA, siRNA, PNA, or anti-sense oligonucleotide;
(n) a ribozyme;
(o) a vaccine;
(p) an anti-angiogenic agent; and
(q) an immuno-oncology therapeutic agent.

56. The ligand-drug conjugate according to claim 14, wherein the linker has any one of the following structures: 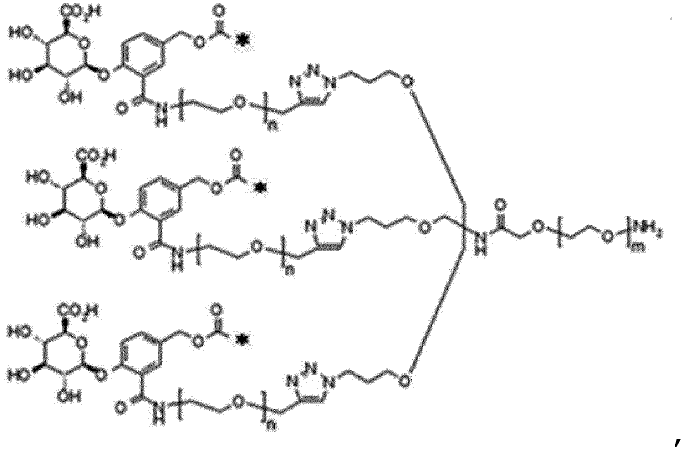 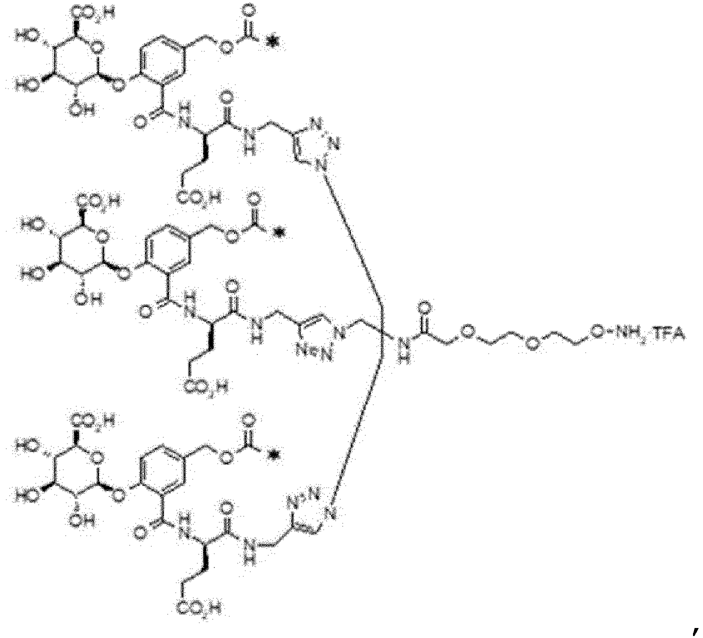 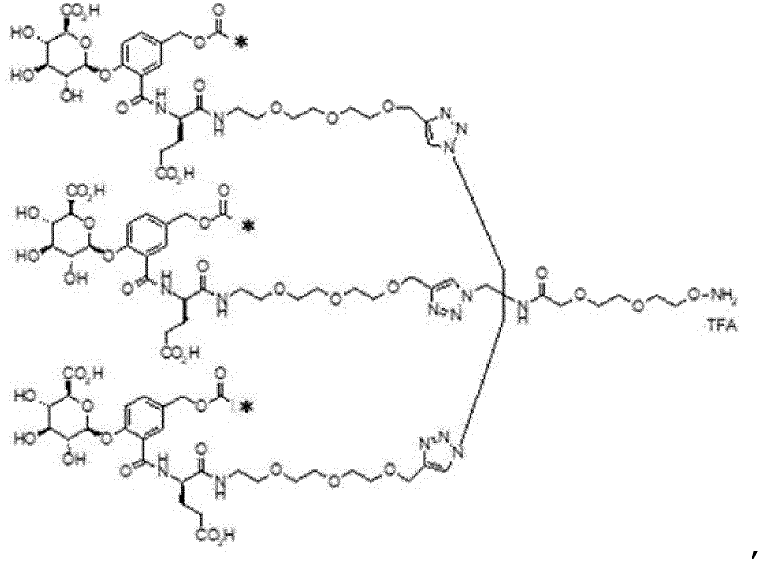 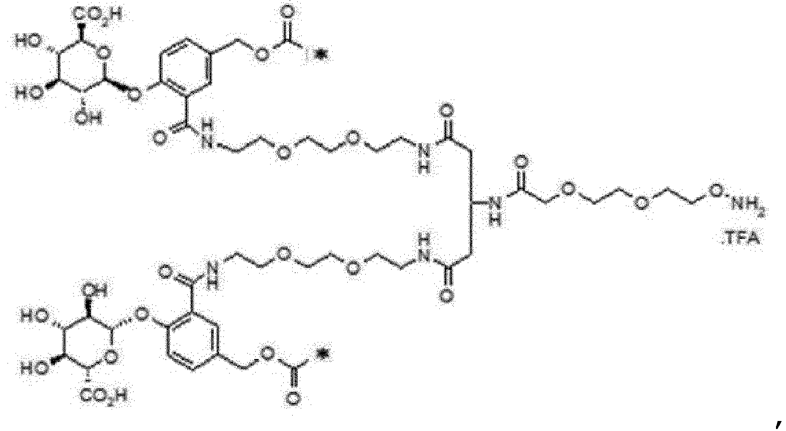 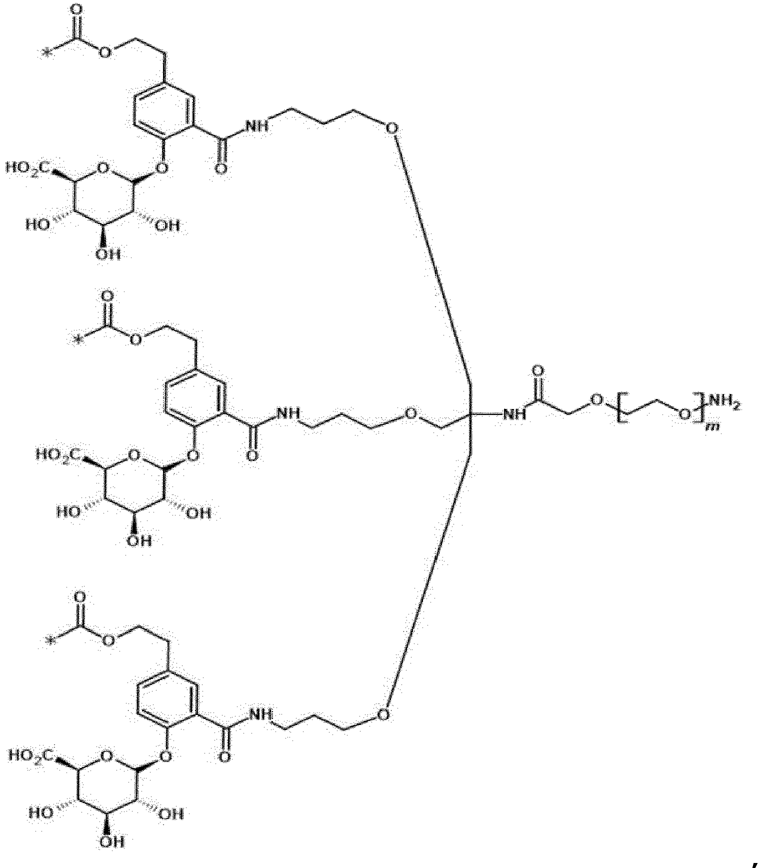 and

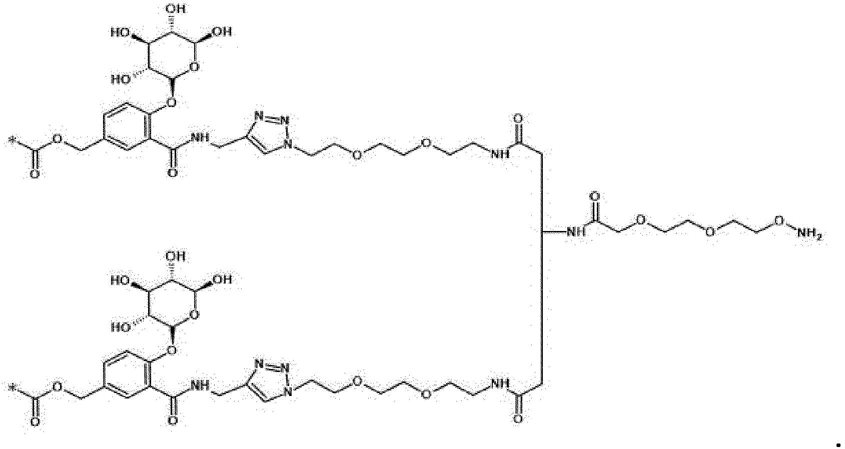

57. The ligand-drug conjugate according to claim 14, wherein the linker binds to a C-terminus of an antibody.

58. A pharmaceutical composition for prevention or treatment of a hyperproliferative, cancer or angiogenic disease, comprising the ligand-drug conjugate according to any one of claims 14 to 57 or a pharmaceutically acceptable salt or solvate of the ligand-drug conjugate as an active ingredient.

59. The pharmaceutical composition according to claim 58, further comprising a chemotherapeutic agent in a therapeutically effective amount.

60. The pharmaceutical composition according to claim 58, wherein the cancer is selected from the group consisting of lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.

61. A method of treating a hyperproliferative, cancer or angiogenic disease in a subject by administering the pharmaceutical composition according to claim 58 or 59 to the subject.

62. The method according to claim 61, wherein the subject is a mammal.

63. The method according to claim 62, wherein the subject is selected from the group consisting of rodents, lagomorphs, felines, canines, porcines, ovines, bovines, equines, and primates.
